# EUROPEAN PATENT APPLICATION

(11) **EP 3 995 510 A1**
(43) Date of publication of application: **11.05.2022**
(21) Application number: 20834925.8
(22) Date of filing: 01.07.2020
(51) Int. Cl.: C07K 16/12, C12N 15/13, C12N 15/63, C12P 21/08, A61K 39/40, A61P 31/04, G01N 33/569

(54) **PERTUSSIS TOXIN BINDING PROTEIN**

(30) Priority: 01.07.2019 CN 201910584155
(71) Applicant: Suzhou Alphamab Co., Ltd, Jiangsu 215125 (CN)
(72) Inventor: XU, Ting, Suzhou, Jiangsu 215125 (CN); WANG, Xiaoxiao, Suzhou, Jiangsu 215125 (CN); WANG, Ling, Suzhou, Jiangsu 215125 (CN); ZHU, Danming, Suzhou, Jiangsu 215125 (CN); GAO, Li, Suzhou, Jiangsu 215125 (CN)
(74) Representative: Michalski Hüttermann & Partner Patentanwälte mbB
(86) International application number: PCT/CN2020/099691
(87) International publication number: WO 2021/000886

(57) **Abstract**

The invention relates to the field of biomedicine, and an anti-pertussis toxin (PT) single domain antibody and derivative protein thereof are disclosed. Specifically, a pertussis toxin-binding protein and use thereof are disclosed.

Sequence listing

<110>  Suzhou Alphamab Co., Ltd.

<120>  Pertussis toxin-binding protein

<130>  P2020TC1158

<150>  201910584155.7
<151>  2019-07-01

<160>  109

<170>  PatentIn version 3.5

<210>  1
<211>  10
<212>  PRT
<213>  Artificial Sequence

<220>
<223>  iPT3 CDR1

<400>  1

Gly Tyr Thr Val Ser Ser Ile Cys Met Gly
1               5                   10


<210>  2
<211>  16
<212>  PRT
<213>  Artificial Sequence

<220>
<223>  iPT3 CDR2

<400>  2

Ala Val Ala Ser Asp Gly Val Thr Ser Tyr Thr Asp Ser Val Lys Gly
1               5                   10                  15


<210>  3
<211>  15
<212>  PRT
<213>  Artificial Sequence

<220>
<223>  iPT3 CDR3

<400>  3

Arg Lys Arg Gly Tyr Cys Ser Leu Asp Arg Thr Phe Phe Thr Trp
1               5                   10                  15


<210>  4
<211>  10
<212>  PRT
<213>  Artificial Sequence

<220>

<223>  iPT7 CDR1

<400>  4

Gly Tyr Thr Tyr Ser Ser Tyr Cys Met Gly
1               5                   10


<210>  5
<211>  16
<212>  PRT
<213>  Artificial Sequence

<220>
<223>  iPT7 CDR2

<400>  5

Ala Ile Asp Ser Asp Gly Arg Thr Ser Tyr Ala Asp Phe Val Lys Gly
1               5                   10                  15


<210>  6
<211>  19
<212>  PRT
<213>  Artificial Sequence

<220>
<223>  iPT7 CDR3

<400>  6

Glu Gln Arg Ile Gly Gly Ser Trp Tyr Asp Leu Cys Gly Gly Ala Asp
1               5                   10                  15


Phe Gly Tyr




<210>  7
<211>  10
<212>  PRT
<213>  Artificial Sequence

<220>
<223>  iPT12 CDR1

<400>  7

Gly Tyr Thr Tyr Ser Ser Tyr Cys Met Gly
1               5                   10


<210>  8
<211>  16
<212>  PRT
<213>  Artificial Sequence

<220>
<223>  iPT12 CDR2

<400>  8

Gly Ile Glu Ser Asp Gly Ser Thr Ser Tyr Ala Asp Ser Val Lys Gly
1               5                   10                  15

<210>  9
<211>  20
<212>  PRT
<213>  Artificial Sequence

<220>
<223>  iPT12 CDR3

<400>  9

Gln Ser Pro Leu Pro Gly Gly Ser Trp Tyr Ser Leu Cys Gly Gly Ala
1               5                   10                  15

Asp Phe Gly Tyr
            20

<210>  10
<211>  7
<212>  PRT
<213>  Artificial Sequence

<220>
<223>  iPT13 CDR1

<400>  10

Gly Tyr Arg Ser Cys Met Gly
1               5

<210>  11
<211>  16
<212>  PRT
<213>  Artificial Sequence

<220>
<223>  iPT13 CDR2

<400>  11

Ala Ile Trp Pro Asp Gly Ile Thr Tyr Tyr Ala Asp Ser Val Lys Gly
1               5                   10                  15

<210>  12
<211>  17
<212>  PRT
<213>  Artificial Sequence

<220>
<223>  iPT13 CDR3

<400>  12

Ala Asp Leu Ile Cys Pro Thr Asp Trp Ala Gln Arg Leu Gln Tyr Asn
1               5                   10                  15

Tyr

<210> 13
<211> 10
<212> PRT
<213> Artificial Sequence

<220>
<223> iPT15 CDR1

<400> 13

Gly Tyr Thr Tyr Gly Ser Tyr Cys Met Ala
1               5                   10

<210> 14
<211> 16
<212> PRT
<213> Artificial Sequence

<220>
<223> iPT15 CDR2

<400> 14

Gly Val Asp Ser Asp Gly Thr Thr Arg Tyr Ala Asp Ser Val Lys Gly
1               5                   10                  15

<210> 15
<211> 23
<212> PRT
<213> Artificial Sequence

<220>
<223> iPT15 CDR3

<400> 15

Gly Val Cys Gly Thr Gly Gly Ser Trp Tyr Glu Gly Asp Ala Gly Leu
1               5                   10                  15

Phe Met Tyr Glu Phe Ala Tyr
            20

<210> 16
<211> 7
<212> PRT
<213> Artificial Sequence

<220>
<223> iPT20 CDR1

<400> 16

Glu Tyr Arg Tyr Cys Met Ala

1               5


<210>  17
<211>  16
<212>  PRT
<213>  Artificial Sequence

<220>
<223>  iPT20 CDR2

<400>  17

Gly Ile Tyr Ile Gly Asp Arg Thr Val Tyr Ser Asp Ser Val Lys Gly
1               5                   10                  15


<210>  18
<211>  17
<212>  PRT
<213>  Artificial Sequence

<220>
<223>  iPT20 CDR3

<400>  18

Val Asp Leu Ile Cys Pro Ser Asp Trp Ala Gln Arg Leu Gln Tyr Gln
1               5                   10                  15


Tyr




<210>  19
<211>  10
<212>  PRT
<213>  Artificial Sequence

<220>
<223>  iPT21 CDR1

<400>  19

Gly Tyr Pro Tyr Ser Arg Asn Cys Met Ala
1               5                   10


<210>  20
<211>  17
<212>  PRT
<213>  Artificial Sequence

<220>
<223>  iPT21 CDR2

<400>  20

Thr Ile Tyr Asn Gly Asp Gly Thr Pro Tyr Tyr Ala Asp Ser Val Lys
1               5                   10                  15

Gly

<210>  21
<211>  17
<212>  PRT
<213>  Artificial Sequence

<220>
<223>  iPT21 CDR3

<400>  21

Ala Arg Gly Cys Ser Leu Arg Thr Trp Ser Asp Gln Asn Leu Tyr Ala
1               5                   10                  15

Tyr

<210>  22
<211>  9
<212>  PRT
<213>  Artificial Sequence

<220>
<223>  iPT22 CDR1

<400>  22

Asp Ile Thr Tyr Ser Arg Cys Met Ala
1               5

<210>  23
<211>  17
<212>  PRT
<213>  Artificial Sequence

<220>
<223>  iPT22 CDR2

<400>  23

Ala Leu Ser Thr Asn Gly Gly Ser Thr Trp Tyr Ala Asp Ser Val Lys
1               5                   10                  15

Gly

<210>  24
<211>  22
<212>  PRT
<213>  Artificial Sequence

<220>
<223>  iPT22 CDR3

<400>  24

Ser Ser Phe Phe Ser Cys Arg Asp Cys Ser Gly Gly Asp Cys Pro Arg
1               5                   10                  15

Ser Arg Asp Tyr Lys Tyr
            20

<210>  25
<211>  10
<212>  PRT
<213>  Artificial Sequence

<220>
<223>  iPT26 CDR1

<400>  25

Gly Phe Thr Thr Ser Ser Arg Cys Met Ala
1               5                   10

<210>  26
<211>  17
<212>  PRT
<213>  Artificial Sequence

<220>
<223>  iPT26 CDR2

<400>  26

Leu Ile Val Thr Gly Thr Gly Ala Thr Arg Tyr Ala Asp Ser Met Lys
1               5                   10                  15

Gly

<210>  27
<211>  17
<212>  PRT
<213>  Artificial Sequence

<220>
<223>  iPT26 CDR3

<400>  27

Ile Asp Ile Thr Trp Arg Gly Cys Ser Phe Glu Lys Arg Asp Val Asn
1               5                   10                  15

Tyr

<210>  28
<211>  10
<212>  PRT
<213>  Artificial Sequence

<220>
<223>  iPT28 CDR1

<400>  28

Gly Tyr Thr Tyr Thr Ser Tyr Cys Met Gly
1               5                   10


<210>  29
<211>  16
<212>  PRT
<213>  Artificial Sequence

<220>
<223>  iPT28 CDR2

<400>  29

Gly Ile Asp Arg Asp Gly Ile Ala Val Tyr Ala Asp Ser Val Lys Gly
1               5                   10                  15


<210>  30
<211>  12
<212>  PRT
<213>  Artificial Sequence

<220>
<223>  iPT28 CDR3

<400>  30

Asp Tyr Arg Arg Trp Cys Arg Asp Glu Tyr Glu Tyr
1               5                   10


<210>  31
<211>  10
<212>  PRT
<213>  Artificial Sequence

<220>
<223>  iPT35 CDR1

<400>  31

Thr Tyr Thr Thr Thr Ser Arg Cys Met Ala
1               5                   10


<210>  32
<211>  17
<212>  PRT
<213>  Artificial Sequence

<220>
<223>  iPT35 CDR2

<400>  32

Leu Ile Val Thr Gly Thr Gly Ala Thr Arg Tyr Ala Asp Ser Met Lys

1               5                   10                  15

Gly

<210>  33
<211>  17
<212>  PRT
<213>  Artificial Sequence

<220>
<223>  iPT35 CDR3

<400>  33

Ile Asp Ile Thr Trp Arg Gly Cys Ser Phe Glu Thr Arg Asp Val Asn
1               5                   10                  15

Tyr

<210>  34
<211>  7
<212>  PRT
<213>  Artificial Sequence

<220>
<223>  iPT36 CDR1

<400>  34

Glu Tyr Arg Ser Cys Met Gly
1               5

<210>  35
<211>  16
<212>  PRT
<213>  Artificial Sequence

<220>
<223>  iPT36 CDR2

<400>  35

Ala Ile Trp Pro Asp Gly Ile Thr Tyr Tyr Ala Asp Ser Val Lys Gly
1               5                   10                  15

<210>  36
<211>  17
<212>  PRT
<213>  Artificial Sequence

<220>
<223>  iPT36 CDR3

<400>  36

Ala Glu Leu Ile Cys Pro Thr Asp Trp Ala Gln Arg Gln Gln Tyr Asn
1               5                   10                  15

Tyr

<210>  37
<211>  10
<212>  PRT
<213>  Artificial Sequence

<220>
<223>  iPT42 CDR1

<400>  37

Gly Tyr Thr Tyr Ser Asp Val Cys Met Gly
1               5                   10

<210>  38
<211>  16
<212>  PRT
<213>  Artificial Sequence

<220>
<223>  iPT42 CDR2

<400>  38

Ala Ile Asp Ser Val Gly Asn Thr Asn Tyr Ala Asp Ser Val Lys Gly
1               5                   10                  15

<210>  39
<211>  16
<212>  PRT
<213>  Artificial Sequence

<220>
<223>  iPT42 CDR3

<400>  39

Gln Arg Cys Leu Gly Trp Val Pro Leu Val Pro His Glu Tyr Gly Phe
1               5                   10                  15

<210>  40
<211>  123
<212>  PRT
<213>  Artificial Sequence

<220>
<223>  iPT3

<400>  40

Gln Val Gln Leu Gln Glu Ser Gly Gly Gly Ser Val Gln Ala Gly Glu
1               5                   10                  15

Ser Leu Arg Leu Ser Cys Val Val Ser Gly Tyr Thr Val Ser Ser Ile
            20                  25                  30

Cys Met Gly Trp Phe Arg Gln Ala Pro Gly Lys Glu Arg Glu Gly Val
        35                  40                  45

Ala Ala Val Ala Ser Asp Gly Val Thr Ser Tyr Thr Asp Ser Val Lys
    50                  55                  60

Gly Arg Phe Thr Ile Ser Lys Asp Asn Ala Lys Asn Thr Leu Tyr Leu
65                  70                  75                  80

Gln Met Asn Ser Leu Thr Pro Glu Asp Thr Ser Met Tyr Tyr Cys Ala
                85                  90                  95

Ala Arg Lys Arg Gly Tyr Cys Ser Leu Asp Arg Thr Phe Phe Thr Trp
            100                 105                 110

Trp Gly Gln Gly Thr Gln Val Thr Val Ser Ser
        115                 120

<210>  41
<211>  127
<212>  PRT
<213>  Artificial Sequence

<220>
<223>  iPT7

<400>  41

Gln Val Gln Leu Gln Glu Ser Gly Gly Gly Ser Val Gln Pro Gly Gly
1               5                   10                  15

Ser Leu Arg Leu Ser Cys Ala Ala Ser Gly Tyr Thr Tyr Ser Ser Tyr
            20                  25                  30

Cys Met Gly Trp Phe Arg Gln Ala Pro Gly Lys Glu Arg Glu Gly Val
        35                  40                  45

Ala Ala Ile Asp Ser Asp Gly Arg Thr Ser Tyr Ala Asp Phe Val Lys
    50                  55                  60

Gly Arg Phe Thr Ile Ser Lys Asp Asn Ala Lys Asn Thr Leu Tyr Leu
65                  70                  75                  80

Gln Met Asn Ser Leu Lys Pro Glu Asp Thr Ala Lys Tyr Tyr Cys Ala
                85                  90                  95

Thr Glu Gln Arg Ile Gly Gly Ser Trp Tyr Asp Leu Cys Gly Gly Ala
            100                 105                 110

Asp Phe Gly Tyr Trp Gly Gln Gly Thr Gln Val Thr Val Ser Ser
        115                 120                 125

<210>  42
<211>  128
<212>  PRT
<213>  Artificial Sequence

<220>
<223>  iPT12

<400>  42

Gln Val Gln Leu Gln Glu Ser Gly Gly Gly Ser Val Gln Ala Gly Gly
1               5                   10                  15

Ser Leu Arg Leu Ser Cys Ala Ala Ser Gly Tyr Thr Tyr Ser Ser Tyr
            20                  25                  30

Cys Met Gly Trp Phe Arg Gln Ala Pro Gly Lys Glu Arg Glu Gly Val
        35                  40                  45

Ala Gly Ile Glu Ser Asp Gly Ser Thr Ser Tyr Ala Asp Ser Val Lys
    50                  55                  60

Gly Arg Phe Ser Ile Ser Lys Asp Asn Val Lys Asn Thr Leu Tyr Leu
65                  70                  75                  80

Gln Met Asn Thr Leu Lys Pro Glu Asp Thr Gly Ile Tyr Tyr Cys Ala
                85                  90                  95

Val Gln Ser Pro Leu Pro Gly Gly Ser Trp Tyr Ser Leu Cys Gly Gly
            100                 105                 110

Ala Asp Phe Gly Tyr Trp Gly Gln Gly Thr Gln Val Thr Val Ser Ser
        115                 120                 125

<210>  43
<211>  122
<212>  PRT
<213>  Artificial Sequence

<220>
<223>  iPT13

<400>  43

Gln Val Gln Leu Gln Glu Ser Gly Gly Gly Ser Val Gln Ala Gly Gly
1               5                   10                  15

Ser Leu Arg Leu Thr Cys Ala Ala Ser Gly Tyr Arg Ser Cys Met Gly
            20                  25                  30

Trp Phe Arg Gln Ala Pro Gly Lys Glu Arg Glu Gly Val Ala Ala Ile
        35                  40                  45

Trp Pro Asp Gly Ile Thr Tyr Tyr Ala Asp Ser Val Lys Gly Arg Phe
    50                  55                  60

Thr Ile Ser Arg Asp Lys Ala Val Asn Thr Met Tyr Leu Gln Met Asn
65                  70                  75                  80

Ser Leu Lys Pro Glu Asp Thr Ala Met Tyr Tyr Cys Ala Thr Ala Asp
                85                  90                  95

Leu Ile Cys Pro Thr Asp Trp Ala Gln Arg Leu Gln Tyr Asn Tyr Trp
            100                 105                 110

Gly Gln Gly Thr Gln Val Thr Val Ser Ser
        115                 120

<210>  44
<211>  131
<212>  PRT
<213>  Artificial Sequence

<220>
<223>  iPT15

<400>  44

Gln Val Gln Leu Gln Glu Ser Gly Gly Gly Ser Val Gln Ala Gly Gly
1               5                   10                  15

Ser Leu Arg Leu Ser Cys Ala Ala Ser Gly Tyr Thr Tyr Gly Ser Tyr
            20                  25                  30

Cys Met Ala Trp Phe Arg Gln Ala Pro Gly Lys Glu Arg Glu Gly Val
        35                  40                  45

Ala Gly Val Asp Ser Asp Gly Thr Thr Arg Tyr Ala Asp Ser Val Lys
    50                  55                  60

Gly Arg Phe Thr Ile Ser Lys Asp Asn Ala Glu Asn Thr Leu Tyr Leu
65                  70                  75                  80

Gln Met Asn Ser Leu Lys Pro Glu Asp Thr Ser Met Tyr Tyr Cys Ala
                85                  90                  95

Ala Gly Val Cys Gly Thr Gly Gly Ser Trp Tyr Glu Gly Asp Ala Gly
            100                 105                 110

Leu Phe Met Tyr Glu Phe Ala Tyr Trp Gly Gln Gly Thr Gln Val Thr
        115                 120                 125

Val Ser Ser
    130

<210>  45
<211>  122
<212>  PRT
<213>  Artificial Sequence

<220>
<223>  iPT20

<400>  45

Gln Val Gln Leu Gln Glu Ser Gly Gly Gly Ser Val Gln Ala Gly Gly
1               5                   10                  15

Ser Leu Arg Leu Ser Cys Ala Ala Ser Glu Tyr Arg Tyr Cys Met Ala
            20                  25                  30

Trp Phe Arg Gln Ala Pro Gly Lys Glu Arg Glu Gly Val Ala Gly Ile
        35                  40                  45

Tyr Ile Gly Asp Arg Thr Val Tyr Ser Asp Ser Val Lys Gly Arg Phe
    50                  55                  60

Thr Ile Ser Gln Asp Asn Ala Asp Asn Thr Val Tyr Leu Gln Met Asn
65                  70                  75                  80

Ser Leu Lys Pro Glu Asp Thr Ala Met Tyr Tyr Cys Ala Thr Val Asp
                85                  90                  95

Leu Ile Cys Pro Ser Asp Trp Ala Gln Arg Leu Gln Tyr Gln Tyr Trp
            100                 105                 110

Gly Gln Gly Thr Gln Val Thr Val Ser Ser
        115                 120

<210>  46
<211>  126
<212>  PRT
<213>  Artificial Sequence

<220>
<223>  iPT21

<400>  46

Gln Val Gln Leu Gln Glu Ser Gly Gly Gly Ser Val Gln Ala Gly Gly
1               5                   10                  15

Ser Leu Arg Leu Ser Cys Ala Ala Ser Gly Tyr Pro Tyr Ser Arg Asn
            20                  25                  30

Cys Met Ala Trp Phe Arg Gln Ala Pro Glu Lys Gln Arg Glu Gly Val
        35                  40                  45

Ala Thr Ile Tyr Asn Gly Asp Gly Thr Pro Tyr Tyr Ala Asp Ser Val
    50                  55                  60

Lys Gly Arg Phe Thr Ile Ser Gln Asp Asn Ala Lys Asn Thr Val Tyr
65                  70                  75                  80

Leu Gln Met Asn Ser Leu Lys Pro Asp Asp Thr Ala Ile Tyr Tyr Cys
                85                  90                  95

Ala Ala Ala Arg Gly Cys Ser Leu Arg Thr Trp Ser Asp Gln Asn Leu
            100                 105                 110

Tyr Ala Tyr Trp Gly Gln Gly Thr Gln Val Thr Val Ser Ser
        115                 120                 125

<210>  47
<211>  130
<212>  PRT
<213>  Artificial Sequence

<220>
<223>  iPT22

<400>  47

Gln Val Gln Leu Gln Glu Ser Gly Gly Gly Ser Val Gln Ala Gly Gly
1               5                   10                  15

Ser Leu Gln Leu Ser Cys Ala Ala Thr Asp Ile Thr Tyr Ser Arg Cys
            20                  25                  30

Met Ala Trp Phe Arg Gln Ala Pro Gly Lys Ser Arg Glu Gly Val Ala
        35                  40                  45

Ala Leu Ser Thr Asn Gly Gly Ser Thr Trp Tyr Ala Asp Ser Val Lys
    50                  55                  60

Gly Arg Phe Thr Ile Ser Arg Asp Gly Ala Lys Asn Thr Ile Tyr Leu
65                  70                  75                  80

Gln Met Arg Ser Leu Lys Pro Glu Asp Thr Ala Met Tyr Tyr Cys Ala
                85                  90                  95

Asp Ser Ser Phe Phe Ser Cys Arg Asp Cys Ser Gly Gly Asp Cys Pro
            100                 105                 110

Arg Ser Arg Asp Tyr Lys Tyr Trp Gly Gln Gly Thr Gln Val Thr Val
        115                 120                 125

Ser Ser
    130

<210>  48
<211>  126
<212>  PRT
<213>  Artificial Sequence

<220>
<223>  iPT26

<400>  48

Gln Val Gln Leu Gln Glu Ser Gly Gly Gly Ser Val Gln Ala Gly Gly
1               5                   10                  15

Ser Leu Arg Leu Ser Cys Glu Ala Ser Gly Phe Thr Thr Ser Ser Arg
            20                  25                  30

Cys Met Ala Trp Phe Arg Gln Ala Pro Gly Lys Glu Arg Glu Gly Val
        35                  40                  45

Gly Leu Ile Val Thr Gly Thr Gly Ala Thr Arg Tyr Ala Asp Ser Met
    50                  55                  60

Lys Gly Arg Phe Thr Ile Ser Gln Asp Asn Ala Lys Asn Thr Met Trp
65                  70                  75                  80

Leu Gln Ile Asn Ser Leu Lys Pro Glu Asp Thr Ala Met Tyr Phe Cys
                85                  90                  95

Ala Ala Ile Asp Ile Thr Trp Arg Gly Cys Ser Phe Glu Lys Arg Asp
            100                 105                 110

Val Asn Tyr Trp Gly Gln Gly Thr Gln Val Thr Val Ser Ser
        115                 120                 125

<210>  49
<211>  120
<212>  PRT
<213>  Artificial Sequence

<220>
<223>  iPT28

<400>  49

Gln Val Gln Leu Gln Glu Ser Gly Gly Gly Ser Val Gln Ala Gly Gly
1               5                   10                  15

Ser Leu Arg Leu Ser Cys Ala Ala Ser Gly Tyr Thr Tyr Thr Ser Tyr
            20                  25                  30

Cys Met Gly Trp Phe Arg Gln Ala Pro Gly Lys Glu Arg Glu Gly Val
        35                  40                  45

Ala Gly Ile Asp Arg Asp Gly Ile Ala Val Tyr Ala Asp Ser Val Lys
    50                  55                  60

Gly Arg Phe Thr Val Ser Lys Asp Asn Val Glu Asp Thr Leu Ser Leu
65                  70                  75                  80

Gln Met Asn Ser Leu Lys Pro Glu Asp Thr Ala Met Tyr Tyr Cys Ala
                85                  90                  95

Ala Asp Tyr Arg Arg Trp Cys Arg Asp Glu Tyr Glu Tyr Trp Gly Gln
            100                 105                 110

Gly Thr Gln Val Thr Val Ser Ser
        115                 120


<210>  50
<211>  126
<212>  PRT
<213>  Artificial Sequence

<220>
<223>  iPT35

<400>  50

Gln Val Gln Leu Gln Glu Ser Gly Gly Gly Ser Val Gln Ala Gly Gly
1               5                   10                  15

Ser Leu Arg Leu Ser Cys Ala Ala Ser Thr Tyr Thr Thr Thr Ser Arg
            20                  25                  30

Cys Met Ala Trp Phe Arg Gln Ala Pro Gly Lys Glu Arg Glu Gly Val
        35                  40                  45

Gly Leu Ile Val Thr Gly Thr Gly Ala Thr Arg Tyr Ala Asp Ser Met
    50                  55                  60

Lys Gly Arg Phe Thr Ile Ser Gln Asp Thr Ala Lys Asn Thr Met Trp
65                  70                  75                  80

Leu Gln Met Asn Ser Leu Lys Pro Glu Asp Thr Gly Met Tyr Phe Cys
                85                  90                  95

Ala Ala Ile Asp Ile Thr Trp Arg Gly Cys Ser Phe Glu Thr Arg Asp
            100                 105                 110

Val Asn Tyr Trp Gly Gln Gly Thr Gln Val Thr Val Ser Ser
        115                 120                 125

<210>  51
<211>  122
<212>  PRT
<213>  Artificial Sequence

<220>
<223>  iPT36

<400>  51

Gln Val Gln Leu Gln Glu Ser Gly Gly Gly Ser Val Gln Val Gly Gly
1               5                   10                  15

Ser Leu Arg Leu Thr Cys Ala Ala Ser Glu Tyr Arg Ser Cys Met Gly
            20                  25                  30

Trp Phe Arg Gln Ala Pro Gly Lys Glu Arg Glu Gly Val Ala Ala Ile
        35                  40                  45

Trp Pro Asp Gly Ile Thr Tyr Tyr Ala Asp Ser Val Lys Gly Arg Phe
    50                  55                  60

Thr Ile Ser Arg Asp Lys Asp Val Asn Thr Met Tyr Leu Ala Met Asn
65                  70                  75                  80

Ser Leu Lys Pro Glu Asp Thr Ala Met Tyr Tyr Cys Ala Thr Ala Glu
                85                  90                  95

Leu Ile Cys Pro Thr Asp Trp Ala Gln Arg Gln Gln Tyr Asn Tyr Trp
            100                 105                 110

Gly Gln Gly Thr Gln Val Thr Val Ser Ser
        115                 120

<210>  52
<211>  124
<212>  PRT
<213>  Artificial Sequence

<220>
<223>  iPT42

<400>  52

Gln Val Gln Leu Gln Glu Ser Gly Gly Gly Ser Val Gln Ala Gly Gly
1               5                   10                  15

Ser Leu Arg Leu Ser Cys Ala Ala Ser Gly Tyr Thr Tyr Ser Asp Val
            20                  25                  30

Cys Met Gly Trp Phe Arg Gln Ala Pro Gly Lys Glu Arg Glu Gly Val
        35                  40                  45

Ala Ala Ile Asp Ser Val Gly Asn Thr Asn Tyr Ala Asp Ser Val Lys
    50                  55                  60

Gly Arg Phe Thr Ile Ser Lys Asp Asn Ala Lys Asn Thr Leu Tyr Leu
65                  70                  75                  80

Gln Met Asn Ser Leu Lys Pro Glu Asp Thr Ala Met Tyr Tyr Cys Ala
                85                  90                  95

Ala Gln Arg Cys Leu Gly Trp Val Pro Leu Val Pro His Glu Tyr Gly
            100                 105                 110

Phe Trp Gly Gln Gly Thr Gln Val Thr Val Ser Ser
        115                 120


<210>  53
<211>  127
<212>  PRT
<213>  Artificial Sequence

<220>
<223>  huPT7V1

<400>  53

Gln Val Gln Leu Val Glu Ser Gly Gly Gly Ser Val Gln Ala Gly Gly
1               5                   10                  15

Ser Leu Arg Leu Ser Cys Ala Ala Ser Gly Tyr Thr Tyr Ser Ser Tyr
            20                  25                  30

Cys Met Gly Trp Phe Arg Gln Ala Pro Gly Lys Glu Arg Glu Gly Val
        35                  40                  45

Ala Ala Ile Asp Ser Asp Gly Arg Thr Ser Tyr Ala Asp Phe Val Lys
    50                  55                  60

Gly Arg Phe Thr Ile Ser Lys Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Thr Glu Gln Arg Ile Gly Gly Ser Trp Tyr Asp Leu Cys Gly Gly Ala
            100                 105                 110

Asp Phe Gly Tyr Trp Gly Gln Gly Thr Leu Val Thr Val Ser Ser
        115                 120                 125

<210>  54
<211>  127
<212>  PRT
<213>  Artificial Sequence

<220>
<223>  huPT7V2

<400>  54

Gln Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Tyr Ser Ser Tyr
            20                  25                  30

Cys Met Gly Trp Phe Arg Gln Ala Pro Gly Lys Glu Arg Glu Gly Val
        35                  40                  45

Ala Ala Ile Asp Ser Asp Gly Arg Thr Ser Tyr Ala Asp Phe Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Thr Glu Gln Arg Ile Gly Gly Ser Trp Tyr Asp Leu Cys Gly Gly Ala
            100                 105                 110

Asp Phe Gly Tyr Trp Gly Gln Gly Thr Leu Val Thr Val Ser Ser
        115                 120                 125

<210>  55
<211>  127
<212>  PRT
<213>  Artificial Sequence

<220>
<223>  huPT7V3

<400>  55

Gln Val Gln Leu Val Glu Ser Gly Gly Gly Ser Val Gln Ala Gly Gly
1               5                   10                  15

Ser Leu Arg Leu Ser Cys Ala Ala Ser Gly Tyr Thr Tyr Ser Ser Tyr
            20                  25                  30

Cys Met Gly Trp Phe Arg Gln Ala Pro Gly Lys Gly Leu Glu Gly Val
        35                  40                  45

Ala Ala Ile Asp Ser Asp Gly Arg Thr Ser Tyr Ala Asp Phe Val Lys
    50                  55                  60

Gly Arg Phe Thr Ile Ser Lys Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Thr Glu Gln Arg Ile Gly Gly Ser Trp Tyr Asp Leu Cys Gly Gly Ala
            100                 105                 110

Asp Phe Gly Tyr Trp Gly Gln Gly Thr Leu Val Thr Val Ser Ser
        115                 120                 125

<210>  56
<211>  127
<212>  PRT
<213>  Artificial Sequence

<220>
<223>  huPT7V4

<400>  56

Gln Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Tyr Ser Ser Tyr
            20                  25                  30

Cys Met Gly Trp Phe Arg Gln Ala Pro Gly Lys Gly Leu Glu Gly Val
        35                  40                  45

Ala Ala Ile Asp Ser Asp Gly Arg Thr Ser Tyr Ala Asp Phe Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Thr Glu Gln Arg Ile Gly Gly Ser Trp Tyr Asp Leu Cys Gly Gly Ala
            100                 105                 110

Asp Phe Gly Tyr Trp Gly Gln Gly Thr Leu Val Thr Val Ser Ser
        115                 120                 125

<210>  57
<211>  127
<212>  PRT
<213>  Artificial Sequence

<220>
<223>  huPT7V5

<400>  57

Gln Val Gln Leu Val Glu Ser Gly Gly Gly Ser Val Gln Ala Gly Gly
1               5                   10                  15

Ser Leu Arg Leu Ser Cys Ala Ala Ser Gly Tyr Thr Tyr Ser Ser Tyr
            20                  25                  30

Cys Met Gly Trp Phe Arg Gln Ala Pro Gly Lys Gly Leu Glu Gly Val
        35                  40                  45

Ala Ala Ile Asp Ser Asp Gly Arg Thr Ser Tyr Ala Asp Phe Val Lys
    50                  55                  60

Gly Arg Phe Thr Ile Ser Lys Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Thr Glu Gln Arg Ile Gly Gly Ser Trp Tyr Asp Leu Cys Gly Gly Ala
            100                 105                 110

Asp Phe Gly Tyr Trp Gly Gln Gly Thr Leu Val Thr Val Ser Ser
        115                 120                 125

<210>  58
<211>  127
<212>  PRT
<213>  Artificial Sequence

<220>
<223>  huPT7V6

<400>  58

Gln Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Tyr Ser Ser Tyr
            20                  25                  30

Cys Met Gly Trp Phe Arg Gln Ala Pro Gly Lys Gly Leu Glu Gly Val
        35                  40                  45

Ala Ala Ile Asp Ser Asp Gly Arg Thr Ser Tyr Ala Asp Phe Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Thr Glu Gln Arg Ile Gly Gly Ser Trp Tyr Asp Leu Cys Gly Gly Ala
            100                 105                 110

Asp Phe Gly Tyr Trp Gly Gln Gly Thr Leu Val Thr Val Ser Ser
        115                 120                 125

<210>  59
<211>  127
<212>  PRT
<213>  Artificial Sequence

<220>
<223>  huPT7V7

<400>  59

Gln Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Tyr Ser Ser Tyr
            20                  25                  30

Cys Met Gly Trp Phe Arg Gln Ala Pro Gly Lys Glu Arg Glu Gly Val
        35                  40                  45

Ala Ala Ile Asp Ser Asp Gly Arg Thr Ser Tyr Ala Asp Phe Val Lys
    50                  55                  60

Gly Arg Phe Thr Ile Ser Lys Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Thr Glu Gln Arg Ile Gly Gly Ser Trp Tyr Asp Leu Cys Gly Gly Ala
            100                 105                 110

Asp Phe Gly Tyr Trp Gly Gln Gly Thr Leu Val Thr Val Ser Ser
        115                 120                 125

<210>  60
<211>  127
<212>  PRT
<213>  Artificial Sequence

<220>
<223>  huPT7V8

<400>  60

Gln Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Tyr Ser Ser Tyr
            20                  25                  30

Cys Met Gly Trp Phe Arg Gln Ala Pro Gly Lys Glu Arg Glu Gly Val
        35                  40                  45

Ala Ala Ile Asp Ser Asp Gly Arg Thr Ser Tyr Ala Asp Phe Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Thr Glu Gln Arg Ile Gly Gly Ser Trp Tyr Asp Leu Cys Gly Gly Ala
            100                 105                 110

Asp Phe Gly Tyr Trp Gly Gln Gly Thr Leu Val Thr Val Ser Ser
        115                 120                 125

<210>  61
<211>  127
<212>  PRT
<213>  Artificial Sequence

<220>
<223>  huPT7V9

<400>  61

Gln Val Gln Leu Val Glu Ser Gly Gly Gly Ser Val Gln Ala Gly Gly
1               5                   10                  15

Ser Leu Arg Leu Ser Cys Ala Ala Ser Gly Tyr Thr Tyr Ser Ser Tyr
            20                  25                  30

Cys Met Gly Trp Phe Arg Gln Ala Pro Gly Lys Gly Leu Glu Gly Val
        35                  40                  45

Ala Ala Ile Asp Ser Asp Gly Arg Thr Ser Tyr Ala Asp Phe Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Thr Glu Gln Arg Ile Gly Gly Ser Trp Tyr Asp Leu Cys Gly Gly Ala
            100                 105                 110

Asp Phe Gly Tyr Trp Gly Gln Gly Thr Leu Val Thr Val Ser Ser
        115                 120                 125

<210>  62
<211>  127
<212>  PRT
<213>  Artificial Sequence

<220>
<223>  huPT7V10

<400>  62

Gln Val Gln Leu Val Glu Ser Gly Gly Gly Ser Val Gln Ala Gly Gly
1               5                   10                  15

Ser Leu Arg Leu Ser Cys Ala Ala Ser Gly Tyr Thr Tyr Ser Ser Tyr
            20                  25                  30

Cys Met Gly Trp Phe Arg Gln Ala Pro Gly Lys Glu Arg Glu Gly Val
        35                  40                  45

Ala Ala Ile Asp Ser Asp Gly Arg Thr Ser Tyr Ala Asp Phe Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Thr Glu Gln Arg Ile Gly Gly Ser Trp Tyr Asp Leu Cys Gly Gly Ala
            100                 105                 110

Asp Phe Gly Tyr Trp Gly Gln Gly Thr Leu Val Thr Val Ser Ser
        115                 120                 125

<210>  63
<211>  127
<212>  PRT
<213>  Artificial Sequence

<220>
<223>  huPT7v11

<400>  63

Gln Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Tyr Ser Ser Tyr
            20                  25                  30

Cys Met Gly Trp Phe Arg Gln Ala Pro Gly Lys Gly Leu Glu Gly Val
        35                  40                  45

Ala Ala Ile Asp Ser Asp Gly Arg Thr Ser Tyr Ala Asp Phe Val Lys
    50                  55                  60

Gly Arg Phe Thr Ile Ser Lys Asp Asn Ser Lys Asn Thr Leu Tyr Leu
65                  70                  75                  80

Gln Met Asn Ser Leu Lys Pro Glu Asp Thr Ala Val Tyr Tyr Cys Ala
                85                  90                  95

Thr Glu Gln Arg Ile Gly Gly Ser Trp Tyr Asp Leu Cys Gly Gly Ala
            100                 105                 110

Asp Phe Gly Tyr Trp Gly Gln Gly Thr Leu Val Thr Val Ser Ser
        115                 120                 125

<210>  64
<211>  131
<212>  PRT
<213>  Artificial Sequence

<220>
<223>  huPT15V1

<400>  64

Gln Val Gln Leu Val Glu Ser Gly Gly Gly Ser Val Gln Ala Gly Gly
1               5                   10                  15

Ser Leu Arg Leu Ser Cys Ala Ala Ser Gly Tyr Thr Tyr Gly Ser Tyr
            20                  25                  30

Cys Met Ala Trp Phe Arg Gln Ala Pro Gly Lys Glu Arg Glu Gly Val
        35                  40                  45

Ala Gly Val Asp Ser Asp Gly Thr Thr Arg Tyr Ala Asp Ser Val Lys
    50                  55                  60

Gly Arg Phe Thr Ile Ser Lys Asp Asn Ser Glu Asn Thr Leu Tyr Leu
65                  70                  75                  80

Gln Met Asn Ser Leu Arg Ala Glu Asp Thr Ala Val Tyr Tyr Cys Ala
                85                  90                  95

Ala Gly Val Cys Gly Thr Gly Gly Ser Trp Tyr Glu Gly Asp Ala Gly
            100                 105                 110

Leu Phe Met Tyr Glu Phe Ala Tyr Trp Gly Gln Gly Thr Leu Val Thr
        115                 120                 125

Val Ser Ser
    130

<210>  65
<211>  131
<212>  PRT
<213>  Artificial Sequence

<220>
<223>  huPT15V2

<400>  65

Gln Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Tyr Gly Ser Tyr
            20                  25                  30

Cys Met Ala Trp Phe Arg Gln Ala Pro Gly Lys Glu Arg Glu Gly Val
        35                  40                  45

Ala Gly Val Asp Ser Asp Gly Thr Thr Arg Tyr Ala Asp Ser Val Lys
    50                  55                  60

Gly Arg Phe Thr Ile Ser Arg Asp Asn Ser Glu Asn Thr Leu Tyr Leu
65                  70                  75                  80

Gln Met Asn Ser Leu Arg Ala Glu Asp Thr Ala Val Tyr Tyr Cys Ala
                85                  90                  95

Ala Gly Val Cys Gly Thr Gly Gly Ser Trp Tyr Glu Gly Asp Ala Gly
            100                 105                 110

Leu Phe Met Tyr Glu Phe Ala Tyr Trp Gly Gln Gly Thr Leu Val Thr
        115                 120                 125

Val Ser Ser
    130

<210>  66
<211>  131
<212>  PRT
<213>  Artificial Sequence

<220>
<223>  huPT15V3

<400>  66

Gln Val Gln Leu Val Glu Ser Gly Gly Gly Ser Val Gln Ala Gly Gly
1               5                   10                  15

Ser Leu Arg Leu Ser Cys Ala Ala Ser Gly Tyr Thr Tyr Gly Ser Tyr
            20                  25                  30

Cys Met Ala Trp Phe Arg Gln Ala Pro Gly Lys Gly Leu Glu Gly Val
        35                  40                  45

Ala Gly Val Asp Ser Asp Gly Thr Thr Arg Tyr Ala Asp Ser Val Lys
    50                  55                  60

Gly Arg Phe Thr Ile Ser Lys Asp Asn Ser Glu Asn Thr Leu Tyr Leu
65                  70                  75                  80

Gln Met Asn Ser Leu Arg Ala Glu Asp Thr Ala Val Tyr Tyr Cys Ala
                85                  90                  95

Ala Gly Val Cys Gly Thr Gly Gly Ser Trp Tyr Glu Gly Asp Ala Gly
            100                 105                 110

Leu Phe Met Tyr Glu Phe Ala Tyr Trp Gly Gln Gly Thr Leu Val Thr

115                 120                 125

Val Ser Ser
    130

<210>  67
<211>  131
<212>  PRT
<213>  Artificial Sequence

<220>
<223>  huPT15V4

<400>  67

Gln Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Tyr Gly Ser Tyr
            20                  25                  30

Cys Met Ala Trp Phe Arg Gln Ala Pro Gly Lys Gly Leu Glu Gly Val
        35                  40                  45

Ala Gly Val Asp Ser Asp Gly Thr Thr Arg Tyr Ala Asp Ser Val Lys
    50                  55                  60

Gly Arg Phe Thr Ile Ser Arg Asp Asn Ser Glu Asn Thr Leu Tyr Leu
65                  70                  75                  80

Gln Met Asn Ser Leu Arg Ala Glu Asp Thr Ala Val Tyr Tyr Cys Ala
                85                  90                  95

Ala Gly Val Cys Gly Thr Gly Gly Ser Trp Tyr Glu Gly Asp Ala Gly
            100                 105                 110

Leu Phe Met Tyr Glu Phe Ala Tyr Trp Gly Gln Gly Thr Leu Val Thr
        115                 120                 125

Val Ser Ser
    130

<210>  68
<211>  131
<212>  PRT
<213>  Artificial Sequence

<220>
<223>  huPT15V5

<400>  68

Gln Val Gln Leu Val Glu Ser Gly Gly Gly Ser Val Gln Ala Gly Gly
1               5                   10                  15

Ser Leu Arg Leu Ser Cys Ala Ala Ser Gly Tyr Thr Tyr Gly Ser Tyr
            20                  25                  30

Cys Met Ala Trp Phe Arg Gln Ala Pro Gly Lys Gly Leu Glu Gly Val
        35                  40                  45

Ala Gly Val Asp Ser Asp Gly Thr Thr Arg Tyr Ala Asp Ser Val Lys
    50                  55                  60

Gly Arg Phe Thr Ile Ser Lys Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Ala Gly Val Cys Gly Thr Gly Gly Ser Trp Tyr Glu Gly Asp Ala Gly
            100                 105                 110

Leu Phe Met Tyr Glu Phe Ala Tyr Trp Gly Gln Gly Thr Leu Val Thr
        115                 120                 125

Val Ser Ser
    130

<210>  69
<211>  131
<212>  PRT
<213>  Artificial Sequence

<220>
<223>  huPT15V6

<400>  69

Gln Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Tyr Gly Ser Tyr
            20                  25                  30

Cys Met Ala Trp Phe Arg Gln Ala Pro Gly Lys Gly Leu Glu Gly Val
        35                  40                  45

Ala Gly Val Asp Ser Asp Gly Thr Thr Arg Tyr Ala Asp Ser Val Lys
    50                  55                  60

Gly Arg Phe Thr Ile Ser Arg Asp Asn Ser Lys Asn Thr Leu Tyr Leu

65                  70                  75                  80

Gln Met Asn Ser Leu Arg Ala Glu Asp Thr Ala Val Tyr Tyr Cys Ala
                85                  90                  95

Ala Gly Val Cys Gly Thr Gly Gly Ser Trp Tyr Glu Gly Asp Ala Gly
            100                 105                 110

Leu Phe Met Tyr Glu Phe Ala Tyr Trp Gly Gln Gly Thr Leu Val Thr
        115                 120                 125

Val Ser Ser
    130

<210>  70
<211>  131
<212>  PRT
<213>  Artificial Sequence

<220>
<223>  huPT15V7

<400>  70

Gln Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Tyr Gly Ser Tyr
            20                  25                  30

Cys Met Ala Trp Phe Arg Gln Ala Pro Gly Lys Glu Arg Glu Gly Val
        35                  40                  45

Ala Gly Val Asp Ser Asp Gly Thr Thr Arg Tyr Ala Asp Ser Val Lys
    50                  55                  60

Gly Arg Phe Thr Ile Ser Lys Asp Asn Ser Glu Asn Thr Leu Tyr Leu
65                  70                  75                  80

Gln Met Asn Ser Leu Arg Ala Glu Asp Thr Ala Val Tyr Tyr Cys Ala
                85                  90                  95

Ala Gly Val Cys Gly Thr Gly Gly Ser Trp Tyr Glu Gly Asp Ala Gly
            100                 105                 110

Leu Phe Met Tyr Glu Phe Ala Tyr Trp Gly Gln Gly Thr Leu Val Thr
        115                 120                 125

Val Ser Ser
    130

<210>  71
<211>  131
<212>  PRT
<213>  Artificial Sequence

<220>
<223>  huPT15V8

<400>  71

Gln Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Tyr Gly Ser Tyr
            20                  25                  30

Cys Met Ala Trp Phe Arg Gln Ala Pro Gly Lys Glu Arg Glu Gly Val
        35                  40                  45

Ala Gly Val Asp Ser Asp Gly Thr Thr Arg Tyr Ala Asp Ser Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Ala Gly Val Cys Gly Thr Gly Gly Ser Trp Tyr Glu Gly Asp Ala Gly
            100                 105                 110

Leu Phe Met Tyr Glu Phe Ala Tyr Trp Gly Gln Gly Thr Leu Val Thr
        115                 120                 125

Val Ser Ser
    130

<210>  72
<211>  131
<212>  PRT
<213>  Artificial Sequence

<220>
<223>  huPT15V9

<400>  72

Gln Val Gln Leu Val Glu Ser Gly Gly Gly Ser Val Gln Ala Gly Gly
1               5                   10                  15

Ser Leu Arg Leu Ser Cys Ala Ala Ser Gly Tyr Thr Tyr Gly Ser Tyr

20                  25                  30

Cys Met Ala Trp Phe Arg Gln Ala Pro Gly Lys Gly Leu Glu Gly Val
        35                  40                  45

Ala Gly Val Asp Ser Asp Gly Thr Thr Arg Tyr Ala Asp Ser Val Lys
    50                  55                  60

Gly Arg Phe Thr Ile Ser Arg Asp Asn Ser Glu Asn Thr Leu Tyr Leu
65                  70                  75                  80

Gln Met Asn Ser Leu Arg Ala Glu Asp Thr Ala Val Tyr Tyr Cys Ala
                85                  90                  95

Ala Gly Val Cys Gly Thr Gly Gly Ser Trp Tyr Glu Gly Asp Ala Gly
            100                 105                 110

Leu Phe Met Tyr Glu Phe Ala Tyr Trp Gly Gln Gly Thr Leu Val Thr
        115                 120                 125

Val Ser Ser
    130

<210>  73
<211>  131
<212>  PRT
<213>  Artificial Sequence

<220>
<223>  huPT15V10

<400>  73

Gln Val Gln Leu Val Glu Ser Gly Gly Gly Ser Val Gln Ala Gly Gly
1               5                   10                  15

Ser Leu Arg Leu Ser Cys Ala Ala Ser Gly Tyr Thr Tyr Gly Ser Tyr
            20                  25                  30

Cys Met Ala Trp Phe Arg Gln Ala Pro Gly Lys Glu Arg Glu Gly Val
        35                  40                  45

Ala Gly Val Asp Ser Asp Gly Thr Thr Arg Tyr Ala Asp Ser Val Lys
    50                  55                  60

Gly Arg Phe Thr Ile Ser Arg Asp Asn Ser Glu Asn Thr Leu Tyr Leu
65                  70                  75                  80

Gln Met Asn Ser Leu Arg Ala Glu Asp Thr Ala Val Tyr Tyr Cys Ala
                85                  90                  95

Ala Gly Val Cys Gly Thr Gly Gly Ser Trp Tyr Glu Gly Asp Ala Gly
            100                 105                 110

Leu Phe Met Tyr Glu Phe Ala Tyr Trp Gly Gln Gly Thr Leu Val Thr
        115                 120                 125

Val Ser Ser
    130

<210>  74
<211>  131
<212>  PRT
<213>  Artificial Sequence

<220>
<223>  huPT15V11

<400>  74

Gln Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Tyr Gly Ser Tyr
            20                  25                  30

Cys Met Ala Trp Phe Arg Gln Ala Pro Gly Lys Glu Arg Glu Gly Val
        35                  40                  45

Ala Gly Val Asp Ser Asp Gly Thr Thr Arg Tyr Ala Asp Ser Val Lys
    50                  55                  60

Gly Arg Phe Thr Ile Ser Arg Asp Asn Ser Glu Asn Thr Leu Tyr Leu
65                  70                  75                  80

Gln Met Asn Ser Leu Lys Pro Glu Asp Thr Ala Val Tyr Tyr Cys Ala
                85                  90                  95

Ala Gly Val Cys Gly Thr Gly Gly Ser Trp Tyr Glu Gly Asp Ala Gly
            100                 105                 110

Leu Phe Met Tyr Glu Phe Ala Tyr Trp Gly Gln Gly Thr Leu Val Thr
        115                 120                 125

Val Ser Ser
    130

<210>  75
<211>  124
<212>  PRT

<213>  Artificial Sequence

<220>
<223>  huPT42V1

<400>  75

Gln Val Gln Leu Val Glu Ser Gly Gly Gly Ser Val Gln Ala Gly Gly
1               5                   10                  15

Ser Leu Arg Leu Ser Cys Ala Ala Ser Gly Tyr Thr Tyr Ser Asp Val
            20                  25                  30

Cys Met Gly Trp Phe Arg Gln Ala Pro Gly Lys Glu Arg Glu Gly Val
        35                  40                  45

Ala Ala Ile Asp Ser Val Gly Asn Thr Asn Tyr Ala Asp Ser Val Lys
    50                  55                  60

Gly Arg Phe Thr Ile Ser Lys Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Ala Gln Arg Cys Leu Gly Trp Val Pro Leu Val Pro His Glu Tyr Gly
            100                 105                 110

Tyr Trp Gly Gln Gly Thr Leu Val Thr Val Ser Ser
        115                 120


<210>  76
<211>  124
<212>  PRT
<213>  Artificial Sequence

<220>
<223>  huPT42V2

<400>  76

Gln Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Tyr Ser Asp Val
            20                  25                  30

Cys Met Gly Trp Phe Arg Gln Ala Pro Gly Lys Glu Arg Glu Gly Val
        35                  40                  45

Ala Ala Ile Asp Ser Val Gly Asn Thr Asn Tyr Ala Asp Ser Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Ala Gln Arg Cys Leu Gly Trp Val Pro Leu Val Pro His Glu Tyr Gly
            100                 105                 110

Tyr Trp Gly Gln Gly Thr Leu Val Thr Val Ser Ser
        115                 120

<210>  77
<211>  124
<212>  PRT
<213>  Artificial Sequence

<220>
<223>  huPT42V3

<400>  77

Gln Val Gln Leu Val Glu Ser Gly Gly Gly Ser Val Gln Ala Gly Gly
1               5                   10                  15

Ser Leu Arg Leu Ser Cys Ala Ala Ser Gly Tyr Thr Tyr Ser Asp Val
            20                  25                  30

Cys Met Gly Trp Phe Arg Gln Ala Pro Gly Lys Gly Leu Glu Gly Val
        35                  40                  45

Ala Ala Ile Asp Ser Val Gly Asn Thr Asn Tyr Ala Asp Ser Val Lys
    50                  55                  60

Gly Arg Phe Thr Ile Ser Lys Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Ala Gln Arg Cys Leu Gly Trp Val Pro Leu Val Pro His Glu Tyr Gly
            100                 105                 110

Tyr Trp Gly Gln Gly Thr Leu Val Thr Val Ser Ser
        115                 120

<210>  78
<211>  124
<212>  PRT

<213>  Artificial Sequence

<220>
<223>  huPT42V4

<400>  78

Gln Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Tyr Ser Asp Val
            20                  25                  30

Cys Met Gly Trp Phe Arg Gln Ala Pro Gly Lys Gly Leu Glu Gly Val
        35                  40                  45

Ala Ala Ile Asp Ser Val Gly Asn Thr Asn Tyr Ala Asp Ser Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Ala Gln Arg Cys Leu Gly Trp Val Pro Leu Val Pro His Glu Tyr Gly
            100                 105                 110

Tyr Trp Gly Gln Gly Thr Leu Val Thr Val Ser Ser
        115                 120


<210>  79
<211>  124
<212>  PRT
<213>  Artificial Sequence

<220>
<223>  huPT42V5

<400>  79

Gln Val Gln Leu Val Glu Ser Gly Gly Gly Ser Val Gln Ala Gly Gly
1               5                   10                  15

Ser Leu Arg Leu Ser Cys Ala Ala Ser Gly Tyr Thr Tyr Ser Asp Val
            20                  25                  30

Cys Met Gly Trp Phe Arg Gln Ala Pro Gly Lys Gly Leu Glu Gly Val
        35                  40                  45

Ala Ala Ile Asp Ser Val Gly Asn Thr Asn Tyr Ala Asp Ser Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Ala Gln Arg Cys Leu Gly Trp Val Pro Leu Val Pro His Glu Tyr Gly
            100                 105                 110

Tyr Trp Gly Gln Gly Thr Leu Val Thr Val Ser Ser
        115                 120

<210>  80
<211>  124
<212>  PRT
<213>  Artificial Sequence

<220>
<223>  huPT42V6

<400>  80

Gln Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Tyr Ser Asp Val
            20                  25                  30

Cys Met Gly Trp Phe Arg Gln Ala Pro Gly Lys Gly Leu Glu Gly Val
        35                  40                  45

Ala Ala Ile Asp Ser Val Gly Asn Thr Asn Tyr Ala Asp Ser Val Lys
    50                  55                  60

Gly Arg Phe Thr Ile Ser Lys Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Ala Gln Arg Cys Leu Gly Trp Val Pro Leu Val Pro His Glu Tyr Gly
            100                 105                 110

Tyr Trp Gly Gln Gly Thr Leu Val Thr Val Ser Ser
        115                 120

<210>  81
<211>  124
<212>  PRT

<213>  Artificial Sequence

<220>
<223>  huPT42V7

<400>  81

Gln Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Tyr Ser Asp Val
            20                  25                  30

Cys Met Gly Trp Phe Arg Gln Ala Pro Gly Lys Glu Arg Glu Gly Val
        35                  40                  45

Ala Ala Ile Asp Ser Val Gly Asn Thr Asn Tyr Ala Asp Ser Val Lys
    50                  55                  60

Gly Arg Phe Thr Ile Ser Lys Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Ala Gln Arg Cys Leu Gly Trp Val Pro Leu Val Pro His Glu Tyr Gly
            100                 105                 110

Tyr Trp Gly Gln Gly Thr Leu Val Thr Val Ser Ser
        115                 120


<210>  82
<211>  124
<212>  PRT
<213>  Artificial Sequence

<220>
<223>  huPT42V8

<400>  82

Gln Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Tyr Ser Asp Val
            20                  25                  30

Cys Met Gly Trp Phe Arg Gln Ala Pro Gly Lys Glu Arg Glu Gly Val
        35                  40                  45

Ala Ala Ile Asp Ser Val Gly Asn Thr Asn Tyr Ala Asp Ser Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Ala Gln Arg Cys Leu Gly Trp Val Pro Leu Val Pro His Glu Tyr Gly
            100                 105                 110

Tyr Trp Gly Gln Gly Thr Leu Val Thr Val Ser Ser
        115                 120

<210>  83
<211>  124
<212>  PRT
<213>  Artificial Sequence

<220>
<223>  huPT42V9

<400>  83

Gln Val Gln Leu Val Glu Ser Gly Gly Gly Ser Val Gln Ala Gly Gly
1               5                   10                  15

Ser Leu Arg Leu Ser Cys Ala Ala Ser Gly Tyr Thr Tyr Ser Asp Val
            20                  25                  30

Cys Met Gly Trp Phe Arg Gln Ala Pro Gly Lys Gly Leu Glu Gly Val
        35                  40                  45

Ala Ala Ile Asp Ser Val Gly Asn Thr Asn Tyr Ala Asp Ser Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Ala Gln Arg Cys Leu Gly Trp Val Pro Leu Val Pro His Glu Tyr Gly
            100                 105                 110

Tyr Trp Gly Gln Gly Thr Leu Val Thr Val Ser Ser
        115                 120

<210>  84
<211>  124
<212>  PRT

<213>  Artificial Sequence

<220>
<223>  huPT42V10

<400>  84

Gln Val Gln Leu Val Glu Ser Gly Gly Gly Ser Val Gln Ala Gly Gly
1               5                   10                  15

Ser Leu Arg Leu Ser Cys Ala Ala Ser Gly Tyr Thr Tyr Ser Asp Val
            20                  25                  30

Cys Met Gly Trp Phe Arg Gln Ala Pro Gly Lys Glu Arg Glu Gly Val
        35                  40                  45

Ala Ala Ile Asp Ser Val Gly Asn Thr Asn Tyr Ala Asp Ser Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Ala Gln Arg Cys Leu Gly Trp Val Pro Leu Val Pro His Glu Tyr Gly
            100                 105                 110

Tyr Trp Gly Gln Gly Thr Leu Val Thr Val Ser Ser
        115                 120


<210>  85
<211>  124
<212>  PRT
<213>  Artificial Sequence

<220>
<223>  huPT42V11

<400>  85

Gln Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Tyr Ser Asp Val
            20                  25                  30

Cys Met Gly Trp Phe Arg Gln Ala Pro Gly Lys Glu Arg Glu Gly Val
        35                  40                  45

Ala Ala Ile Asp Ser Val Gly Asn Thr Asn Tyr Ala Asp Ser Val Lys
    50                  55                  60

Gly Arg Phe Thr Ile Ser Lys Asp Asn Ser Lys Asn Thr Leu Tyr Leu
65                  70                  75                  80

Gln Met Asn Ser Leu Lys Pro Glu Asp Thr Ala Val Tyr Tyr Cys Ala
                85                  90                  95

Ala Gln Arg Cys Leu Gly Trp Val Pro Leu Val Pro His Glu Tyr Gly
            100                 105                 110

Tyr Trp Gly Gln Gly Thr Leu Val Thr Val Ser Ser
        115                 120

<210>  86
<211>  232
<212>  PRT
<213>  Artificial Sequence

<220>
<223>  IgG1-FC

<400>  86

Glu Pro Lys Ser Cys Asp Lys Thr His Thr Cys Pro Pro Cys Pro Ala
1               5                   10                  15

Pro Glu Leu Leu Gly Gly Pro Ser Val Phe Leu Phe Pro Pro Lys Pro
            20                  25                  30

Lys Asp Thr Leu Met Ile Ser Arg Thr Pro Glu Val Thr Cys Val Val
        35                  40                  45

Val Asp Val Ser His Glu Asp Pro Glu Val Lys Phe Asn Trp Tyr Val
    50                  55                  60

Asp Gly Val Glu Val His Asn Ala Lys Thr Lys Pro Arg Glu Glu Gln
65                  70                  75                  80

Tyr Asn Ser Thr Tyr Arg Val Val Ser Val Leu Thr Val Leu His Gln
                85                  90                  95

Asp Trp Leu Asn Gly Lys Glu Tyr Lys Cys Lys Val Ser Asn Lys Ala
            100                 105                 110

Leu Pro Ala Pro Ile Glu Lys Thr Ile Ser Lys Ala Lys Gly Gln Pro
        115                 120                 125

Arg Glu Pro Gln Val Tyr Thr Leu Pro Pro Ser Arg Asp Glu Leu Thr
    130                 135                 140

Lys Asn Gln Val Ser Leu Thr Cys Leu Val Lys Gly Phe Tyr Pro Ser
145                 150                 155                 160

Asp Ile Ala Val Glu Trp Glu Ser Asn Gly Gln Pro Glu Asn Asn Tyr
                165                 170                 175

Lys Thr Thr Pro Pro Val Leu Asp Ser Asp Gly Ser Phe Phe Leu Tyr
            180                 185                 190

Ser Lys Leu Thr Val Asp Lys Ser Arg Trp Gln Gln Gly Asn Val Phe
        195                 200                 205

Ser Cys Ser Val Met His Glu Ala Leu His Asn His Tyr Thr Gln Lys
    210                 215                 220

Ser Leu Ser Leu Ser Pro Gly Lys
225                 230

<210>  87
<211>  258
<212>  PRT
<213>  Artificial Sequence

<220>
<223>  iPT7n15

<400>  87

Gln Val Gln Leu Gln Glu Ser Gly Gly Gly Ser Val Gln Pro Gly Gly
1               5                   10                  15

Ser Leu Arg Leu Ser Cys Ala Ala Ser Gly Tyr Thr Tyr Ser Ser Tyr
            20                  25                  30

Cys Met Gly Trp Phe Arg Gln Ala Pro Gly Lys Glu Arg Glu Gly Val
        35                  40                  45

Ala Ala Ile Asp Ser Asp Gly Arg Thr Ser Tyr Ala Asp Phe Val Lys
    50                  55                  60

Gly Arg Phe Thr Ile Ser Lys Asp Asn Ala Lys Asn Thr Leu Tyr Leu
65                  70                  75                  80

Gln Met Asn Ser Leu Lys Pro Glu Asp Thr Ala Lys Tyr Tyr Cys Ala
                85                  90                  95

Thr Glu Gln Arg Ile Gly Gly Ser Trp Tyr Asp Leu Cys Gly Gly Ala
            100                 105                 110

Asp Phe Gly Tyr Trp Gly Gln Gly Thr Gln Val Thr Val Ser Ser Gln
        115                 120                 125

Val Gln Leu Gln Glu Ser Gly Gly Gly Ser Val Gln Ala Gly Gly Ser
    130                 135                 140

Leu Arg Leu Ser Cys Ala Ala Ser Gly Tyr Thr Tyr Gly Ser Tyr Cys
145                 150                 155                 160

Met Ala Trp Phe Arg Gln Ala Pro Gly Lys Glu Arg Glu Gly Val Ala
                165                 170                 175

Gly Val Asp Ser Asp Gly Thr Thr Arg Tyr Ala Asp Ser Val Lys Gly
            180                 185                 190

Arg Phe Thr Ile Ser Lys Asp Asn Ala Glu Asn Thr Leu Tyr Leu Gln
        195                 200                 205

Met Asn Ser Leu Lys Pro Glu Asp Thr Ser Met Tyr Tyr Cys Ala Ala
    210                 215                 220

Gly Val Cys Gly Thr Gly Gly Ser Trp Tyr Glu Gly Asp Ala Gly Leu
225                 230                 235                 240

Phe Met Tyr Glu Phe Ala Tyr Trp Gly Gln Gly Thr Gln Val Thr Val
                245                 250                 255

Ser Ser

<210>  88
<211>  258
<212>  PRT
<213>  Artificial Sequence

<220>
<223>  iPT15n7

<400>  88

Gln Val Gln Leu Gln Glu Ser Gly Gly Gly Ser Val Gln Ala Gly Gly
1               5                   10                  15

Ser Leu Arg Leu Ser Cys Ala Ala Ser Gly Tyr Thr Tyr Gly Ser Tyr
            20                  25                  30

Cys Met Ala Trp Phe Arg Gln Ala Pro Gly Lys Glu Arg Glu Gly Val
        35                  40                  45

Ala Gly Val Asp Ser Asp Gly Thr Thr Arg Tyr Ala Asp Ser Val Lys

50                  55                  60

Gly Arg Phe Thr Ile Ser Lys Asp Asn Ala Glu Asn Thr Leu Tyr Leu
65                  70                  75                  80

Gln Met Asn Ser Leu Lys Pro Glu Asp Thr Ser Met Tyr Tyr Cys Ala
                85                  90                  95

Ala Gly Val Cys Gly Thr Gly Gly Ser Trp Tyr Glu Gly Asp Ala Gly
            100                 105                 110

Leu Phe Met Tyr Glu Phe Ala Tyr Trp Gly Gln Gly Thr Gln Val Thr
        115                 120                 125

Val Ser Ser Gln Val Gln Leu Gln Glu Ser Gly Gly Gly Ser Val Gln
    130                 135                 140

Pro Gly Gly Ser Leu Arg Leu Ser Cys Ala Ala Ser Gly Tyr Thr Tyr
145                 150                 155                 160

Ser Ser Tyr Cys Met Gly Trp Phe Arg Gln Ala Pro Gly Lys Glu Arg
                165                 170                 175

Glu Gly Val Ala Ala Ile Asp Ser Asp Gly Arg Thr Ser Tyr Ala Asp
            180                 185                 190

Phe Val Lys Gly Arg Phe Thr Ile Ser Lys Asp Asn Ala Lys Asn Thr
        195                 200                 205

Leu Tyr Leu Gln Met Asn Ser Leu Lys Pro Glu Asp Thr Ala Lys Tyr
    210                 215                 220

Tyr Cys Ala Thr Glu Gln Arg Ile Gly Gly Ser Trp Tyr Asp Leu Cys
225                 230                 235                 240

Gly Gly Ala Asp Phe Gly Tyr Trp Gly Gln Gly Thr Gln Val Thr Val
                245                 250                 255

Ser Ser

<210>  89
<211>  257
<212>  PRT
<213>  Artificial Sequence

<220>
<223>  iPT7n22

<400>  89

Gln Val Gln Leu Gln Glu Ser Gly Gly Gly Ser Val Gln Pro Gly Gly
1               5                   10                  15

Ser Leu Arg Leu Ser Cys Ala Ala Ser Gly Tyr Thr Tyr Ser Ser Tyr
            20                  25                  30

Cys Met Gly Trp Phe Arg Gln Ala Pro Gly Lys Glu Arg Glu Gly Val
        35                  40                  45

Ala Ala Ile Asp Ser Asp Gly Arg Thr Ser Tyr Ala Asp Phe Val Lys
    50                  55                  60

Gly Arg Phe Thr Ile Ser Lys Asp Asn Ala Lys Asn Thr Leu Tyr Leu
65                  70                  75                  80

Gln Met Asn Ser Leu Lys Pro Glu Asp Thr Ala Lys Tyr Tyr Cys Ala
                85                  90                  95

Thr Glu Gln Arg Ile Gly Gly Ser Trp Tyr Asp Leu Cys Gly Gly Ala
            100                 105                 110

Asp Phe Gly Tyr Trp Gly Gln Gly Thr Gln Val Thr Val Ser Ser Gln
        115                 120                 125

Val Gln Leu Gln Glu Ser Gly Gly Gly Ser Val Gln Ala Gly Gly Ser
    130                 135                 140

Leu Gln Leu Ser Cys Ala Ala Thr Asp Ile Thr Tyr Ser Arg Cys Met
145                 150                 155                 160

Ala Trp Phe Arg Gln Ala Pro Gly Lys Ser Arg Glu Gly Val Ala Ala
                165                 170                 175

Leu Ser Thr Asn Gly Gly Ser Thr Trp Tyr Ala Asp Ser Val Lys Gly
            180                 185                 190

Arg Phe Thr Ile Ser Arg Asp Gly Ala Lys Asn Thr Ile Tyr Leu Gln
        195                 200                 205

Met Arg Ser Leu Lys Pro Glu Asp Thr Ala Met Tyr Tyr Cys Ala Asp
    210                 215                 220

Ser Ser Phe Phe Ser Cys Arg Asp Cys Ser Gly Gly Asp Cys Pro Arg
225                 230                 235                 240

Ser Arg Asp Tyr Lys Tyr Trp Gly Gln Gly Thr Gln Val Thr Val Ser

245                 250                 255

Ser

<210>  90
<211>  257
<212>  PRT
<213>  Artificial Sequence

<220>
<223>  iPT22n7

<400>  90

Gln Val Gln Leu Gln Glu Ser Gly Gly Gly Ser Val Gln Ala Gly Gly
1               5                   10                  15

Ser Leu Gln Leu Ser Cys Ala Ala Thr Asp Ile Thr Tyr Ser Arg Cys
            20                  25                  30

Met Ala Trp Phe Arg Gln Ala Pro Gly Lys Ser Arg Glu Gly Val Ala
        35                  40                  45

Ala Leu Ser Thr Asn Gly Gly Ser Thr Trp Tyr Ala Asp Ser Val Lys
    50                  55                  60

Gly Arg Phe Thr Ile Ser Arg Asp Gly Ala Lys Asn Thr Ile Tyr Leu
65                  70                  75                  80

Gln Met Arg Ser Leu Lys Pro Glu Asp Thr Ala Met Tyr Tyr Cys Ala
                85                  90                  95

Asp Ser Ser Phe Phe Ser Cys Arg Asp Cys Ser Gly Gly Asp Cys Pro
            100                 105                 110

Arg Ser Arg Asp Tyr Lys Tyr Trp Gly Gln Gly Thr Gln Val Thr Val
        115                 120                 125

Ser Ser Gln Val Gln Leu Gln Glu Ser Gly Gly Gly Ser Val Gln Pro
    130                 135                 140

Gly Gly Ser Leu Arg Leu Ser Cys Ala Ala Ser Gly Tyr Thr Tyr Ser
145                 150                 155                 160

Ser Tyr Cys Met Gly Trp Phe Arg Gln Ala Pro Gly Lys Glu Arg Glu
                165                 170                 175

Gly Val Ala Ala Ile Asp Ser Asp Gly Arg Thr Ser Tyr Ala Asp Phe
            180                 185                 190

Val Lys Gly Arg Phe Thr Ile Ser Lys Asp Asn Ala Lys Asn Thr Leu
        195                 200                 205

Tyr Leu Gln Met Asn Ser Leu Lys Pro Glu Asp Thr Ala Lys Tyr Tyr
    210                 215                 220

Cys Ala Thr Glu Gln Arg Ile Gly Gly Ser Trp Tyr Asp Leu Cys Gly
225                 230                 235                 240

Gly Ala Asp Phe Gly Tyr Trp Gly Gln Gly Thr Gln Val Thr Val Ser
                245                 250                 255

Ser

<210>  91
<211>  251
<212>  PRT
<213>  Artificial Sequence

<220>
<223>  iPT7n42

<400>  91

Gln Val Gln Leu Gln Glu Ser Gly Gly Gly Ser Val Gln Pro Gly Gly
1               5                   10                  15

Ser Leu Arg Leu Ser Cys Ala Ala Ser Gly Tyr Thr Tyr Ser Ser Tyr
            20                  25                  30

Cys Met Gly Trp Phe Arg Gln Ala Pro Gly Lys Glu Arg Glu Gly Val
        35                  40                  45

Ala Ala Ile Asp Ser Asp Gly Arg Thr Ser Tyr Ala Asp Phe Val Lys
    50                  55                  60

Gly Arg Phe Thr Ile Ser Lys Asp Asn Ala Lys Asn Thr Leu Tyr Leu
65                  70                  75                  80

Gln Met Asn Ser Leu Lys Pro Glu Asp Thr Ala Lys Tyr Tyr Cys Ala
                85                  90                  95

Thr Glu Gln Arg Ile Gly Gly Ser Trp Tyr Asp Leu Cys Gly Gly Ala
            100                 105                 110

Asp Phe Gly Tyr Trp Gly Gln Gly Thr Gln Val Thr Val Ser Ser Gln
        115                 120                 125

Val Gln Leu Gln Glu Ser Gly Gly Gly Ser Val Gln Ala Gly Gly Ser
    130                 135                 140

Leu Arg Leu Ser Cys Ala Ala Ser Gly Tyr Thr Tyr Ser Asp Val Cys
145                 150                 155                 160

Met Gly Trp Phe Arg Gln Ala Pro Gly Lys Glu Arg Glu Gly Val Ala
                165                 170                 175

Ala Ile Asp Ser Val Gly Asn Thr Asn Tyr Ala Asp Ser Val Lys Gly
            180                 185                 190

Arg Phe Thr Ile Ser Lys Asp Asn Ala Lys Asn Thr Leu Tyr Leu Gln
        195                 200                 205

Met Asn Ser Leu Lys Pro Glu Asp Thr Ala Met Tyr Tyr Cys Ala Ala
    210                 215                 220

Gln Arg Cys Leu Gly Trp Val Pro Leu Val Pro His Glu Tyr Gly Phe
225                 230                 235                 240

Trp Gly Gln Gly Thr Gln Val Thr Val Ser Ser
                245                 250

<210>  92
<211>  251
<212>  PRT
<213>  Artificial Sequence

<220>
<223>  iPT42n7

<400>  92

Gln Val Gln Leu Gln Glu Ser Gly Gly Gly Ser Val Gln Ala Gly Gly
1               5                   10                  15

Ser Leu Arg Leu Ser Cys Ala Ala Ser Gly Tyr Thr Tyr Ser Asp Val
            20                  25                  30

Cys Met Gly Trp Phe Arg Gln Ala Pro Gly Lys Glu Arg Glu Gly Val
        35                  40                  45

Ala Ala Ile Asp Ser Val Gly Asn Thr Asn Tyr Ala Asp Ser Val Lys
    50                  55                  60

Gly Arg Phe Thr Ile Ser Lys Asp Asn Ala Lys Asn Thr Leu Tyr Leu
65                  70                  75                  80

Gln Met Asn Ser Leu Lys Pro Glu Asp Thr Ala Met Tyr Tyr Cys Ala
                85                  90                  95

Ala Gln Arg Cys Leu Gly Trp Val Pro Leu Val Pro His Glu Tyr Gly
            100                 105                 110

Phe Trp Gly Gln Gly Thr Gln Val Thr Val Ser Ser Gln Val Gln Leu
        115                 120                 125

Gln Glu Ser Gly Gly Gly Ser Val Gln Pro Gly Gly Ser Leu Arg Leu
    130                 135                 140

Ser Cys Ala Ala Ser Gly Tyr Thr Tyr Ser Ser Tyr Cys Met Gly Trp
145                 150                 155                 160

Phe Arg Gln Ala Pro Gly Lys Glu Arg Glu Gly Val Ala Ala Ile Asp
                165                 170                 175

Ser Asp Gly Arg Thr Ser Tyr Ala Asp Phe Val Lys Gly Arg Phe Thr
            180                 185                 190

Ile Ser Lys Asp Asn Ala Lys Asn Thr Leu Tyr Leu Gln Met Asn Ser
        195                 200                 205

Leu Lys Pro Glu Asp Thr Ala Lys Tyr Tyr Cys Ala Thr Glu Gln Arg
    210                 215                 220

Ile Gly Gly Ser Trp Tyr Asp Leu Cys Gly Gly Ala Asp Phe Gly Tyr
225                 230                 235                 240

Trp Gly Gln Gly Thr Gln Val Thr Val Ser Ser
                245                 250

<210>  93
<211>  254
<212>  PRT
<213>  Artificial Sequence

<220>
<223>  iPT12n35

<400>  93

Gln Val Gln Leu Gln Glu Ser Gly Gly Gly Ser Val Gln Ala Gly Gly
1               5                   10                  15

Ser Leu Arg Leu Ser Cys Ala Ala Ser Gly Tyr Thr Tyr Ser Ser Tyr
            20                  25                  30

Cys Met Gly Trp Phe Arg Gln Ala Pro Gly Lys Glu Arg Glu Gly Val

35                  40                  45

Ala Gly Ile Glu Ser Asp Gly Ser Thr Ser Tyr Ala Asp Ser Val Lys
    50                  55                  60

Gly Arg Phe Ser Ile Ser Lys Asp Asn Val Lys Asn Thr Leu Tyr Leu
65                  70                  75                  80

Gln Met Asn Thr Leu Lys Pro Glu Asp Thr Gly Ile Tyr Tyr Cys Ala
                85                  90                  95

Val Gln Ser Pro Leu Pro Gly Gly Ser Trp Tyr Ser Leu Cys Gly Gly
            100                 105                 110

Ala Asp Phe Gly Tyr Trp Gly Gln Gly Thr Gln Val Thr Val Ser Ser
        115                 120                 125

Gln Val Gln Leu Gln Glu Ser Gly Gly Gly Ser Val Gln Ala Gly Gly
    130                 135                 140

Ser Leu Arg Leu Ser Cys Ala Ala Ser Thr Tyr Thr Thr Thr Ser Arg
145                 150                 155                 160

Cys Met Ala Trp Phe Arg Gln Ala Pro Gly Lys Glu Arg Glu Gly Val
                165                 170                 175

Gly Leu Ile Val Thr Gly Thr Gly Ala Thr Arg Tyr Ala Asp Ser Met
            180                 185                 190

Lys Gly Arg Phe Thr Ile Ser Gln Asp Thr Ala Lys Asn Thr Met Trp
        195                 200                 205

Leu Gln Met Asn Ser Leu Lys Pro Glu Asp Thr Gly Met Tyr Phe Cys
    210                 215                 220

Ala Ala Ile Asp Ile Thr Trp Arg Gly Cys Ser Phe Glu Thr Arg Asp
225                 230                 235                 240

Val Asn Tyr Trp Gly Gln Gly Thr Gln Val Thr Val Ser Ser
                245                 250

<210>  94
<211>  254
<212>  PRT
<213>  Artificial Sequence

<220>
<223>  iPT35n12

<400> 94

Gln Val Gln Leu Gln Glu Ser Gly Gly Gly Ser Val Gln Ala Gly Gly
1               5                   10                  15

Ser Leu Arg Leu Ser Cys Ala Ala Ser Thr Tyr Thr Thr Thr Ser Arg
            20                  25                  30

Cys Met Ala Trp Phe Arg Gln Ala Pro Gly Lys Glu Arg Glu Gly Val
        35                  40                  45

Gly Leu Ile Val Thr Gly Thr Gly Ala Thr Arg Tyr Ala Asp Ser Met
    50                  55                  60

Lys Gly Arg Phe Thr Ile Ser Gln Asp Thr Ala Lys Asn Thr Met Trp
65                  70                  75                  80

Leu Gln Met Asn Ser Leu Lys Pro Glu Asp Thr Gly Met Tyr Phe Cys
                85                  90                  95

Ala Ala Ile Asp Ile Thr Trp Arg Gly Cys Ser Phe Glu Thr Arg Asp
            100                 105                 110

Val Asn Tyr Trp Gly Gln Gly Thr Gln Val Thr Val Ser Ser Gln Val
        115                 120                 125

Gln Leu Gln Glu Ser Gly Gly Gly Ser Val Gln Ala Gly Gly Ser Leu
    130                 135                 140

Arg Leu Ser Cys Ala Ala Ser Gly Tyr Thr Tyr Ser Ser Tyr Cys Met
145                 150                 155                 160

Gly Trp Phe Arg Gln Ala Pro Gly Lys Glu Arg Glu Gly Val Ala Gly
                165                 170                 175

Ile Glu Ser Asp Gly Ser Thr Ser Tyr Ala Asp Ser Val Lys Gly Arg
            180                 185                 190

Phe Ser Ile Ser Lys Asp Asn Val Lys Asn Thr Leu Tyr Leu Gln Met
        195                 200                 205

Asn Thr Leu Lys Pro Glu Asp Thr Gly Ile Tyr Tyr Cys Ala Val Gln
    210                 215                 220

Ser Pro Leu Pro Gly Gly Ser Trp Tyr Ser Leu Cys Gly Gly Ala Asp
225                 230                 235                 240

Phe Gly Tyr Trp Gly Gln Gly Thr Gln Val Thr Val Ser Ser

245                 250

<210>  95
<211>  257
<212>  PRT
<213>  Artificial Sequence

<220>
<223>  iPT15n35

<400>  95

Gln Val Gln Leu Gln Glu Ser Gly Gly Gly Ser Val Gln Ala Gly Gly
1               5                   10                  15

Ser Leu Arg Leu Ser Cys Ala Ala Ser Gly Tyr Thr Tyr Gly Ser Tyr
            20                  25                  30

Cys Met Ala Trp Phe Arg Gln Ala Pro Gly Lys Glu Arg Glu Gly Val
        35                  40                  45

Ala Gly Val Asp Ser Asp Gly Thr Thr Arg Tyr Ala Asp Ser Val Lys
    50                  55                  60

Gly Arg Phe Thr Ile Ser Lys Asp Asn Ala Glu Asn Thr Leu Tyr Leu
65                  70                  75                  80

Gln Met Asn Ser Leu Lys Pro Glu Asp Thr Ser Met Tyr Tyr Cys Ala
                85                  90                  95

Ala Gly Val Cys Gly Thr Gly Gly Ser Trp Tyr Glu Gly Asp Ala Gly
            100                 105                 110

Leu Phe Met Tyr Glu Phe Ala Tyr Trp Gly Gln Gly Thr Gln Val Thr
        115                 120                 125

Val Ser Ser Gln Val Gln Leu Gln Glu Ser Gly Gly Gly Ser Val Gln
    130                 135                 140

Ala Gly Gly Ser Leu Arg Leu Ser Cys Ala Ala Ser Thr Tyr Thr Thr
145                 150                 155                 160

Thr Ser Arg Cys Met Ala Trp Phe Arg Gln Ala Pro Gly Lys Glu Arg
                165                 170                 175

Glu Gly Val Gly Leu Ile Val Thr Gly Thr Gly Ala Thr Arg Tyr Ala
            180                 185                 190

Asp Ser Met Lys Gly Arg Phe Thr Ile Ser Gln Asp Thr Ala Lys Asn
        195                 200                 205

Thr Met Trp Leu Gln Met Asn Ser Leu Lys Pro Glu Asp Thr Gly Met
    210                 215                 220

Tyr Phe Cys Ala Ala Ile Asp Ile Thr Trp Arg Gly Cys Ser Phe Glu
225                 230                 235                 240

Thr Arg Asp Val Asn Tyr Trp Gly Gln Gly Thr Gln Val Thr Val Ser
                245                 250                 255

Ser

<210>  96
<211>  255
<212>  PRT
<213>  Artificial Sequence

<220>
<223>  iPT15n42

<400>  96

Gln Val Gln Leu Gln Glu Ser Gly Gly Gly Ser Val Gln Ala Gly Gly
1               5                   10                  15

Ser Leu Arg Leu Ser Cys Ala Ala Ser Gly Tyr Thr Tyr Gly Ser Tyr
            20                  25                  30

Cys Met Ala Trp Phe Arg Gln Ala Pro Gly Lys Glu Arg Glu Gly Val
        35                  40                  45

Ala Gly Val Asp Ser Asp Gly Thr Thr Arg Tyr Ala Asp Ser Val Lys
    50                  55                  60

Gly Arg Phe Thr Ile Ser Lys Asp Asn Ala Glu Asn Thr Leu Tyr Leu
65                  70                  75                  80

Gln Met Asn Ser Leu Lys Pro Glu Asp Thr Ser Met Tyr Tyr Cys Ala
                85                  90                  95

Ala Gly Val Cys Gly Thr Gly Gly Ser Trp Tyr Glu Gly Asp Ala Gly
            100                 105                 110

Leu Phe Met Tyr Glu Phe Ala Tyr Trp Gly Gln Gly Thr Gln Val Thr
        115                 120                 125

Val Ser Ser Gln Val Gln Leu Gln Glu Ser Gly Gly Gly Ser Val Gln
    130                 135                 140

Ala Gly Gly Ser Leu Arg Leu Ser Cys Ala Ala Ser Gly Tyr Thr Tyr
145                 150                 155                 160

Ser Asp Val Cys Met Gly Trp Phe Arg Gln Ala Pro Gly Lys Glu Arg
                165                 170                 175

Glu Gly Val Ala Ala Ile Asp Ser Val Gly Asn Thr Asn Tyr Ala Asp
            180                 185                 190

Ser Val Lys Gly Arg Phe Thr Ile Ser Lys Asp Asn Ala Lys Asn Thr
        195                 200                 205

Leu Tyr Leu Gln Met Asn Ser Leu Lys Pro Glu Asp Thr Ala Met Tyr
    210                 215                 220

Tyr Cys Ala Ala Gln Arg Cys Leu Gly Trp Val Pro Leu Val Pro His
225                 230                 235                 240

Glu Tyr Gly Phe Trp Gly Gln Gly Thr Gln Val Thr Val Ser Ser
                245                 250                 255

<210>  97
<211>  250
<212>  PRT
<213>  Artificial Sequence

<220>
<223>  iPT35n42

<400>  97

Gln Val Gln Leu Gln Glu Ser Gly Gly Gly Ser Val Gln Ala Gly Gly
1               5                   10                  15

Ser Leu Arg Leu Ser Cys Ala Ala Ser Thr Tyr Thr Thr Thr Ser Arg
            20                  25                  30

Cys Met Ala Trp Phe Arg Gln Ala Pro Gly Lys Glu Arg Glu Gly Val
        35                  40                  45

Gly Leu Ile Val Thr Gly Thr Gly Ala Thr Arg Tyr Ala Asp Ser Met
    50                  55                  60

Lys Gly Arg Phe Thr Ile Ser Gln Asp Thr Ala Lys Asn Thr Met Trp
65                  70                  75                  80

Leu Gln Met Asn Ser Leu Lys Pro Glu Asp Thr Gly Met Tyr Phe Cys
                85                  90                  95

Ala Ala Ile Asp Ile Thr Trp Arg Gly Cys Ser Phe Glu Thr Arg Asp
            100                 105                 110

Val Asn Tyr Trp Gly Gln Gly Thr Gln Val Thr Val Ser Ser Gln Val
        115                 120                 125

Gln Leu Gln Glu Ser Gly Gly Gly Ser Val Gln Ala Gly Gly Ser Leu
    130                 135                 140

Arg Leu Ser Cys Ala Ala Ser Gly Tyr Thr Tyr Ser Asp Val Cys Met
145                 150                 155                 160

Gly Trp Phe Arg Gln Ala Pro Gly Lys Glu Arg Glu Gly Val Ala Ala
                165                 170                 175

Ile Asp Ser Val Gly Asn Thr Asn Tyr Ala Asp Ser Val Lys Gly Arg
            180                 185                 190

Phe Thr Ile Ser Lys Asp Asn Ala Lys Asn Thr Leu Tyr Leu Gln Met
        195                 200                 205

Asn Ser Leu Lys Pro Glu Asp Thr Ala Met Tyr Tyr Cys Ala Ala Gln
    210                 215                 220

Arg Cys Leu Gly Trp Val Pro Leu Val Pro His Glu Tyr Gly Phe Trp
225                 230                 235                 240

Gly Gln Gly Thr Gln Val Thr Val Ser Ser
                245                 250

<210>  98
<211>  259
<212>  PRT
<213>  Artificial Sequence

<220>
<223>  iPT12n15

<400>  98

Gln Val Gln Leu Gln Glu Ser Gly Gly Gly Ser Val Gln Ala Gly Gly
1               5                   10                  15

Ser Leu Arg Leu Ser Cys Ala Ala Ser Gly Tyr Thr Tyr Ser Ser Tyr
            20                  25                  30

Cys Met Gly Trp Phe Arg Gln Ala Pro Gly Lys Glu Arg Glu Gly Val
        35                  40                  45

Ala Gly Ile Glu Ser Asp Gly Ser Thr Ser Tyr Ala Asp Ser Val Lys

50                  55                  60

Gly Arg Phe Ser Ile Ser Lys Asp Asn Val Lys Asn Thr Leu Tyr Leu
65                  70                  75                  80

Gln Met Asn Thr Leu Lys Pro Glu Asp Thr Gly Ile Tyr Tyr Cys Ala
                85                  90                  95

Val Gln Ser Pro Leu Pro Gly Gly Ser Trp Tyr Ser Leu Cys Gly Gly
            100                 105                 110

Ala Asp Phe Gly Tyr Trp Gly Gln Gly Thr Gln Val Thr Val Ser Ser
        115                 120                 125

Gln Val Gln Leu Gln Glu Ser Gly Gly Gly Ser Val Gln Ala Gly Gly
    130                 135                 140

Ser Leu Arg Leu Ser Cys Ala Ala Ser Gly Tyr Thr Tyr Gly Ser Tyr
145                 150                 155                 160

Cys Met Ala Trp Phe Arg Gln Ala Pro Gly Lys Glu Arg Glu Gly Val
                165                 170                 175

Ala Gly Val Asp Ser Asp Gly Thr Thr Arg Tyr Ala Asp Ser Val Lys
            180                 185                 190

Gly Arg Phe Thr Ile Ser Lys Asp Asn Ala Glu Asn Thr Leu Tyr Leu
        195                 200                 205

Gln Met Asn Ser Leu Lys Pro Glu Asp Thr Ser Met Tyr Tyr Cys Ala
    210                 215                 220

Ala Gly Val Cys Gly Thr Gly Gly Ser Trp Tyr Glu Gly Asp Ala Gly
225                 230                 235                 240

Leu Phe Met Tyr Glu Phe Ala Tyr Trp Gly Gln Gly Thr Gln Val Thr
                245                 250                 255

Val Ser Ser

<210>  99
<211>  259
<212>  PRT
<213>  Artificial Sequence

<220>
<223>  iPT15n12

<400>  99

Gln Val Gln Leu Gln Glu Ser Gly Gly Gly Ser Val Gln Ala Gly Gly
1               5                   10                  15

Ser Leu Arg Leu Ser Cys Ala Ala Ser Gly Tyr Thr Tyr Gly Ser Tyr
            20                  25                  30

Cys Met Ala Trp Phe Arg Gln Ala Pro Gly Lys Glu Arg Glu Gly Val
        35                  40                  45

Ala Gly Val Asp Ser Asp Gly Thr Thr Arg Tyr Ala Asp Ser Val Lys
    50                  55                  60

Gly Arg Phe Thr Ile Ser Lys Asp Asn Ala Glu Asn Thr Leu Tyr Leu
65                  70                  75                  80

Gln Met Asn Ser Leu Lys Pro Glu Asp Thr Ser Met Tyr Tyr Cys Ala
                85                  90                  95

Ala Gly Val Cys Gly Thr Gly Gly Ser Trp Tyr Glu Gly Asp Ala Gly
            100                 105                 110

Leu Phe Met Tyr Glu Phe Ala Tyr Trp Gly Gln Gly Thr Gln Val Thr
        115                 120                 125

Val Ser Ser Gln Val Gln Leu Gln Glu Ser Gly Gly Gly Ser Val Gln
    130                 135                 140

Ala Gly Gly Ser Leu Arg Leu Ser Cys Ala Ala Ser Gly Tyr Thr Tyr
145                 150                 155                 160

Ser Ser Tyr Cys Met Gly Trp Phe Arg Gln Ala Pro Gly Lys Glu Arg
                165                 170                 175

Glu Gly Val Ala Gly Ile Glu Ser Asp Gly Ser Thr Ser Tyr Ala Asp
            180                 185                 190

Ser Val Lys Gly Arg Phe Ser Ile Ser Lys Asp Asn Val Lys Asn Thr
        195                 200                 205

Leu Tyr Leu Gln Met Asn Thr Leu Lys Pro Glu Asp Thr Gly Ile Tyr
    210                 215                 220

Tyr Cys Ala Val Gln Ser Pro Leu Pro Gly Gly Ser Trp Tyr Ser Leu
225                 230                 235                 240

Cys Gly Gly Ala Asp Phe Gly Tyr Trp Gly Gln Gly Thr Gln Val Thr

245                 250                 255

Val Ser Ser

<210>  100
<211>  252
<212>  PRT
<213>  Artificial Sequence

<220>
<223>  iPT12n42

<400>  100

Gln Val Gln Leu Gln Glu Ser Gly Gly Gly Ser Val Gln Ala Gly Gly
1               5                   10                  15

Ser Leu Arg Leu Ser Cys Ala Ala Ser Gly Tyr Thr Tyr Ser Ser Tyr
            20                  25                  30

Cys Met Gly Trp Phe Arg Gln Ala Pro Gly Lys Glu Arg Glu Gly Val
        35                  40                  45

Ala Gly Ile Glu Ser Asp Gly Ser Thr Ser Tyr Ala Asp Ser Val Lys
    50                  55                  60

Gly Arg Phe Ser Ile Ser Lys Asp Asn Val Lys Asn Thr Leu Tyr Leu
65                  70                  75                  80

Gln Met Asn Thr Leu Lys Pro Glu Asp Thr Gly Ile Tyr Tyr Cys Ala
                85                  90                  95

Val Gln Ser Pro Leu Pro Gly Gly Ser Trp Tyr Ser Leu Cys Gly Gly
            100                 105                 110

Ala Asp Phe Gly Tyr Trp Gly Gln Gly Thr Gln Val Thr Val Ser Ser
        115                 120                 125

Gln Val Gln Leu Gln Glu Ser Gly Gly Gly Ser Val Gln Ala Gly Gly
    130                 135                 140

Ser Leu Arg Leu Ser Cys Ala Ala Ser Gly Tyr Thr Tyr Ser Asp Val
145                 150                 155                 160

Cys Met Gly Trp Phe Arg Gln Ala Pro Gly Lys Glu Arg Glu Gly Val
                165                 170                 175

Ala Ala Ile Asp Ser Val Gly Asn Thr Asn Tyr Ala Asp Ser Val Lys
            180                 185                 190

Gly Arg Phe Thr Ile Ser Lys Asp Asn Ala Lys Asn Thr Leu Tyr Leu
        195                 200                 205

Gln Met Asn Ser Leu Lys Pro Glu Asp Thr Ala Met Tyr Tyr Cys Ala
    210                 215                 220

Ala Gln Arg Cys Leu Gly Trp Val Pro Leu Val Pro His Glu Tyr Gly
225                 230                 235                 240

Phe Trp Gly Gln Gly Thr Gln Val Thr Val Ser Ser
                245                 250

<210>  101
<211>  252
<212>  PRT
<213>  Artificial Sequence

<220>
<223>  iPT42n12

<400>  101

Gln Val Gln Leu Gln Glu Ser Gly Gly Gly Ser Val Gln Ala Gly Gly
1               5                   10                  15

Ser Leu Arg Leu Ser Cys Ala Ala Ser Gly Tyr Thr Tyr Ser Asp Val
            20                  25                  30

Cys Met Gly Trp Phe Arg Gln Ala Pro Gly Lys Glu Arg Glu Gly Val
        35                  40                  45

Ala Ala Ile Asp Ser Val Gly Asn Thr Asn Tyr Ala Asp Ser Val Lys
    50                  55                  60

Gly Arg Phe Thr Ile Ser Lys Asp Asn Ala Lys Asn Thr Leu Tyr Leu
65                  70                  75                  80

Gln Met Asn Ser Leu Lys Pro Glu Asp Thr Ala Met Tyr Tyr Cys Ala
                85                  90                  95

Ala Gln Arg Cys Leu Gly Trp Val Pro Leu Val Pro His Glu Tyr Gly
            100                 105                 110

Phe Trp Gly Gln Gly Thr Gln Val Thr Val Ser Ser Gln Val Gln Leu
        115                 120                 125

Gln Glu Ser Gly Gly Gly Ser Val Gln Ala Gly Gly Ser Leu Arg Leu
    130                 135                 140

Ser Cys Ala Ala Ser Gly Tyr Thr Tyr Ser Ser Tyr Cys Met Gly Trp
145                 150                 155                 160

Phe Arg Gln Ala Pro Gly Lys Glu Arg Glu Gly Val Ala Gly Ile Glu
                165                 170                 175

Ser Asp Gly Ser Thr Ser Tyr Ala Asp Ser Val Lys Gly Arg Phe Ser
            180                 185                 190

Ile Ser Lys Asp Asn Val Lys Asn Thr Leu Tyr Leu Gln Met Asn Thr
        195                 200                 205

Leu Lys Pro Glu Asp Thr Gly Ile Tyr Tyr Cys Ala Val Gln Ser Pro
    210                 215                 220

Leu Pro Gly Gly Ser Trp Tyr Ser Leu Cys Gly Gly Ala Asp Phe Gly
225                 230                 235                 240

Tyr Trp Gly Gln Gly Thr Gln Val Thr Val Ser Ser
                245                 250

<210>  102
<211>  258
<212>  PRT
<213>  Artificial Sequence

<220>
<223>  hu7v11n15v7

<400>  102

Gln Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Tyr Ser Ser Tyr
            20                  25                  30

Cys Met Gly Trp Phe Arg Gln Ala Pro Gly Lys Gly Leu Glu Gly Val
        35                  40                  45

Ala Ala Ile Asp Ser Asp Gly Arg Thr Ser Tyr Ala Asp Phe Val Lys
    50                  55                  60

Gly Arg Phe Thr Ile Ser Lys Asp Asn Ser Lys Asn Thr Leu Tyr Leu
65                  70                  75                  80

Gln Met Asn Ser Leu Lys Pro Glu Asp Thr Ala Val Tyr Tyr Cys Ala
                85                  90                  95

Thr Glu Gln Arg Ile Gly Gly Ser Trp Tyr Asp Leu Cys Gly Gly Ala
                100                 105                 110

Asp Phe Gly Tyr Trp Gly Gln Gly Thr Leu Val Thr Val Ser Ser Gln
        115                 120                 125

Val Gln Leu Val Glu Ser Gly Gly Gly Leu Val Gln Pro Gly Gly Ser
    130                 135                 140

Leu Arg Leu Ser Cys Ala Ala Ser Gly Tyr Thr Tyr Gly Ser Tyr Cys
145                 150                 155                 160

Met Ala Trp Phe Arg Gln Ala Pro Gly Lys Glu Arg Glu Gly Val Ala
                165                 170                 175

Gly Val Asp Ser Asp Gly Thr Thr Arg Tyr Ala Asp Ser Val Lys Gly
                180                 185                 190

Arg Phe Thr Ile Ser Lys Asp Asn Ser Glu Asn Thr Leu Tyr Leu Gln
        195                 200                 205

Met Asn Ser Leu Arg Ala Glu Asp Thr Ala Val Tyr Tyr Cys Ala Ala
    210                 215                 220

Gly Val Cys Gly Thr Gly Gly Ser Trp Tyr Glu Gly Asp Ala Gly Leu
225                 230                 235                 240

Phe Met Tyr Glu Phe Ala Tyr Trp Gly Gln Gly Thr Leu Val Thr Val
                245                 250                 255

Ser Ser

<210>  103
<211>  258
<212>  PRT
<213>  Artificial Sequence

<220>
<223>  hu7v11n15v11

<400>  103

Gln Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Tyr Ser Ser Tyr
            20                  25                  30

Cys Met Gly Trp Phe Arg Gln Ala Pro Gly Lys Gly Leu Glu Gly Val

35                  40                  45

Ala Ala Ile Asp Ser Asp Gly Arg Thr Ser Tyr Ala Asp Phe Val Lys
    50                  55                  60

Gly Arg Phe Thr Ile Ser Lys Asp Asn Ser Lys Asn Thr Leu Tyr Leu
65                  70                  75                  80

Gln Met Asn Ser Leu Lys Pro Glu Asp Thr Ala Val Tyr Tyr Cys Ala
                85                  90                  95

Thr Glu Gln Arg Ile Gly Gly Ser Trp Tyr Asp Leu Cys Gly Gly Ala
            100                 105                 110

Asp Phe Gly Tyr Trp Gly Gln Gly Thr Leu Val Thr Val Ser Ser Gln
        115                 120                 125

Val Gln Leu Val Glu Ser Gly Gly Gly Leu Val Gln Pro Gly Gly Ser
    130                 135                 140

Leu Arg Leu Ser Cys Ala Ala Ser Gly Tyr Thr Tyr Gly Ser Tyr Cys
145                 150                 155                 160

Met Ala Trp Phe Arg Gln Ala Pro Gly Lys Glu Arg Glu Gly Val Ala
                165                 170                 175

Gly Val Asp Ser Asp Gly Thr Thr Arg Tyr Ala Asp Ser Val Lys Gly
            180                 185                 190

Arg Phe Thr Ile Ser Arg Asp Asn Ser Glu Asn Thr Leu Tyr Leu Gln
        195                 200                 205

Met Asn Ser Leu Lys Pro Glu Asp Thr Ala Val Tyr Tyr Cys Ala Ala
    210                 215                 220

Gly Val Cys Gly Thr Gly Gly Ser Trp Tyr Glu Gly Asp Ala Gly Leu
225                 230                 235                 240

Phe Met Tyr Glu Phe Ala Tyr Trp Gly Gln Gly Thr Leu Val Thr Val
                245                 250                 255

Ser Ser

<210>  104
<211>  255
<212>  PRT
<213>  Artificial Sequence

<220>
<223>  hu42v6n15v11-Fc

<400>  104

Gln Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Tyr Ser Asp Val
            20                  25                  30

Cys Met Gly Trp Phe Arg Gln Ala Pro Gly Lys Gly Leu Glu Gly Val
        35                  40                  45

Ala Ala Ile Asp Ser Val Gly Asn Thr Asn Tyr Ala Asp Ser Val Lys
    50                  55                  60

Gly Arg Phe Thr Ile Ser Lys Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Ala Gln Arg Cys Leu Gly Trp Val Pro Leu Val Pro His Glu Tyr Gly
            100                 105                 110

Tyr Trp Gly Gln Gly Thr Leu Val Thr Val Ser Ser Gln Val Gln Leu
        115                 120                 125

Val Glu Ser Gly Gly Gly Leu Val Gln Pro Gly Gly Ser Leu Arg Leu
    130                 135                 140

Ser Cys Ala Ala Ser Gly Tyr Thr Tyr Gly Ser Tyr Cys Met Ala Trp
145                 150                 155                 160

Phe Arg Gln Ala Pro Gly Lys Glu Arg Glu Gly Val Ala Gly Val Asp
                165                 170                 175

Ser Asp Gly Thr Thr Arg Tyr Ala Asp Ser Val Lys Gly Arg Phe Thr
            180                 185                 190

Ile Ser Arg Asp Asn Ser Glu Asn Thr Leu Tyr Leu Gln Met Asn Ser
        195                 200                 205

Leu Lys Pro Glu Asp Thr Ala Val Tyr Tyr Cys Ala Ala Gly Val Cys
    210                 215                 220

Gly Thr Gly Gly Ser Trp Tyr Glu Gly Asp Ala Gly Leu Phe Met Tyr

225                 230                 235                 240

Glu Phe Ala Tyr Trp Gly Gln Gly Thr Leu Val Thr Val Ser Ser
                245                 250                 255

<210>  105
<211>  251
<212>  PRT
<213>  Artificial Sequence

<220>
<223>  hu42v6n7v11

<400>  105

Gln Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Tyr Ser Asp Val
            20                  25                  30

Cys Met Gly Trp Phe Arg Gln Ala Pro Gly Lys Gly Leu Glu Gly Val
        35                  40                  45

Ala Ala Ile Asp Ser Val Gly Asn Thr Asn Tyr Ala Asp Ser Val Lys
    50                  55                  60

Gly Arg Phe Thr Ile Ser Lys Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Ala Gln Arg Cys Leu Gly Trp Val Pro Leu Val Pro His Glu Tyr Gly
            100                 105                 110

Tyr Trp Gly Gln Gly Thr Leu Val Thr Val Ser Ser Gln Val Gln Leu
        115                 120                 125

Val Glu Ser Gly Gly Gly Leu Val Gln Pro Gly Gly Ser Leu Arg Leu
    130                 135                 140

Ser Cys Ala Ala Ser Gly Tyr Thr Tyr Ser Ser Tyr Cys Met Gly Trp
145                 150                 155                 160

Phe Arg Gln Ala Pro Gly Lys Gly Leu Glu Gly Val Ala Ala Ile Asp
                165                 170                 175

Ser Asp Gly Arg Thr Ser Tyr Ala Asp Phe Val Lys Gly Arg Phe Thr
            180                 185                 190

Ile Ser Lys Asp Asn Ser Lys Asn Thr Leu Tyr Leu Gln Met Asn Ser
        195                 200                 205

Leu Lys Pro Glu Asp Thr Ala Val Tyr Tyr Cys Ala Thr Glu Gln Arg
    210                 215                 220

Ile Gly Gly Ser Trp Tyr Asp Leu Cys Gly Gly Ala Asp Phe Gly Tyr
225                 230                 235                 240

Trp Gly Gln Gly Thr Leu Val Thr Val Ser Ser
                245                 250

<210>  106
<211>  490
<212>  PRT
<213>  Artificial Sequence

<220>
<223>  hu7v11n15v7-Fc

<400>  106

Gln Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Tyr Ser Ser Tyr
            20                  25                  30

Cys Met Gly Trp Phe Arg Gln Ala Pro Gly Lys Gly Leu Glu Gly Val
        35                  40                  45

Ala Ala Ile Asp Ser Asp Gly Arg Thr Ser Tyr Ala Asp Phe Val Lys
    50                  55                  60

Gly Arg Phe Thr Ile Ser Lys Asp Asn Ser Lys Asn Thr Leu Tyr Leu
65                  70                  75                  80

Gln Met Asn Ser Leu Lys Pro Glu Asp Thr Ala Val Tyr Tyr Cys Ala
                85                  90                  95

Thr Glu Gln Arg Ile Gly Gly Ser Trp Tyr Asp Leu Cys Gly Gly Ala
            100                 105                 110

Asp Phe Gly Tyr Trp Gly Gln Gly Thr Leu Val Thr Val Ser Ser Gln
        115                 120                 125

Val Gln Leu Val Glu Ser Gly Gly Gly Leu Val Gln Pro Gly Gly Ser
    130                 135                 140

Leu Arg Leu Ser Cys Ala Ala Ser Gly Tyr Thr Tyr Gly Ser Tyr Cys
145                 150                 155                 160

Met Ala Trp Phe Arg Gln Ala Pro Gly Lys Glu Arg Glu Gly Val Ala
                165                 170                 175

Gly Val Asp Ser Asp Gly Thr Thr Arg Tyr Ala Asp Ser Val Lys Gly
            180                 185                 190

Arg Phe Thr Ile Ser Lys Asp Asn Ser Glu Asn Thr Leu Tyr Leu Gln
        195                 200                 205

Met Asn Ser Leu Arg Ala Glu Asp Thr Ala Val Tyr Tyr Cys Ala Ala
    210                 215                 220

Gly Val Cys Gly Thr Gly Gly Ser Trp Tyr Glu Gly Asp Ala Gly Leu
225                 230                 235                 240

Phe Met Tyr Glu Phe Ala Tyr Trp Gly Gln Gly Thr Leu Val Thr Val
                245                 250                 255

Ser Ser Glu Pro Lys Ser Cys Asp Lys Thr His Thr Cys Pro Pro Cys
            260                 265                 270

Pro Ala Pro Glu Leu Leu Gly Gly Pro Ser Val Phe Leu Phe Pro Pro
        275                 280                 285

Lys Pro Lys Asp Thr Leu Met Ile Ser Arg Thr Pro Glu Val Thr Cys
    290                 295                 300

Val Val Val Asp Val Ser His Glu Asp Pro Glu Val Lys Phe Asn Trp
305                 310                 315                 320

Tyr Val Asp Gly Val Glu Val His Asn Ala Lys Thr Lys Pro Arg Glu
                325                 330                 335

Glu Gln Tyr Asn Ser Thr Tyr Arg Val Val Ser Val Leu Thr Val Leu
            340                 345                 350

His Gln Asp Trp Leu Asn Gly Lys Glu Tyr Lys Cys Lys Val Ser Asn
        355                 360                 365

Lys Ala Leu Pro Ala Pro Ile Glu Lys Thr Ile Ser Lys Ala Lys Gly
    370                 375                 380

Gln Pro Arg Glu Pro Gln Val Tyr Thr Leu Pro Pro Ser Arg Asp Glu
385                 390                 395                 400

Leu Thr Lys Asn Gln Val Ser Leu Thr Cys Leu Val Lys Gly Phe Tyr
                405                 410                 415

Pro Ser Asp Ile Ala Val Glu Trp Glu Ser Asn Gly Gln Pro Glu Asn
            420                 425                 430

Asn Tyr Lys Thr Thr Pro Pro Val Leu Asp Ser Asp Gly Ser Phe Phe
        435                 440                 445

Leu Tyr Ser Lys Leu Thr Val Asp Lys Ser Arg Trp Gln Gln Gly Asn
    450                 455                 460

Val Phe Ser Cys Ser Val Met His Glu Ala Leu His Asn His Tyr Thr
465                 470                 475                 480

Gln Lys Ser Leu Ser Leu Ser Pro Gly Lys
                485                 490

<210>  107
<211>  490
<212>  PRT
<213>  Artificial Sequence

<220>
<223>  hu7v11n15v11-Fc

<400>  107

Gln Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Tyr Ser Ser Tyr
            20                  25                  30

Cys Met Gly Trp Phe Arg Gln Ala Pro Gly Lys Gly Leu Glu Gly Val
        35                  40                  45

Ala Ala Ile Asp Ser Asp Gly Arg Thr Ser Tyr Ala Asp Phe Val Lys
    50                  55                  60

Gly Arg Phe Thr Ile Ser Lys Asp Asn Ser Lys Asn Thr Leu Tyr Leu
65                  70                  75                  80

Gln Met Asn Ser Leu Lys Pro Glu Asp Thr Ala Val Tyr Tyr Cys Ala
                85                  90                  95

Thr Glu Gln Arg Ile Gly Gly Ser Trp Tyr Asp Leu Cys Gly Gly Ala
            100                 105                 110

Asp Phe Gly Tyr Trp Gly Gln Gly Thr Leu Val Thr Val Ser Ser Gln
        115                 120                 125

Val Gln Leu Val Glu Ser Gly Gly Gly Leu Val Gln Pro Gly Gly Ser
    130                 135                 140

Leu Arg Leu Ser Cys Ala Ala Ser Gly Tyr Thr Tyr Gly Ser Tyr Cys
145                 150                 155                 160

Met Ala Trp Phe Arg Gln Ala Pro Gly Lys Glu Arg Glu Gly Val Ala
                165                 170                 175

Gly Val Asp Ser Asp Gly Thr Thr Arg Tyr Ala Asp Ser Val Lys Gly
            180                 185                 190

Arg Phe Thr Ile Ser Arg Asp Asn Ser Glu Asn Thr Leu Tyr Leu Gln
        195                 200                 205

Met Asn Ser Leu Lys Pro Glu Asp Thr Ala Val Tyr Tyr Cys Ala Ala
    210                 215                 220

Gly Val Cys Gly Thr Gly Gly Ser Trp Tyr Glu Gly Asp Ala Gly Leu
225                 230                 235                 240

Phe Met Tyr Glu Phe Ala Tyr Trp Gly Gln Gly Thr Leu Val Thr Val
                245                 250                 255

Ser Ser Glu Pro Lys Ser Cys Asp Lys Thr His Thr Cys Pro Pro Cys
            260                 265                 270

Pro Ala Pro Glu Leu Leu Gly Gly Pro Ser Val Phe Leu Phe Pro Pro
        275                 280                 285

Lys Pro Lys Asp Thr Leu Met Ile Ser Arg Thr Pro Glu Val Thr Cys
    290                 295                 300

Val Val Val Asp Val Ser His Glu Asp Pro Glu Val Lys Phe Asn Trp
305                 310                 315                 320

Tyr Val Asp Gly Val Glu Val His Asn Ala Lys Thr Lys Pro Arg Glu
                325                 330                 335

Glu Gln Tyr Asn Ser Thr Tyr Arg Val Val Ser Val Leu Thr Val Leu
            340                 345                 350

His Gln Asp Trp Leu Asn Gly Lys Glu Tyr Lys Cys Lys Val Ser Asn
        355                 360                 365

Lys Ala Leu Pro Ala Pro Ile Glu Lys Thr Ile Ser Lys Ala Lys Gly
    370                 375                 380

Gln Pro Arg Glu Pro Gln Val Tyr Thr Leu Pro Pro Ser Arg Asp Glu
385                 390                 395                 400

Leu Thr Lys Asn Gln Val Ser Leu Thr Cys Leu Val Lys Gly Phe Tyr
                405                 410                 415

Pro Ser Asp Ile Ala Val Glu Trp Glu Ser Asn Gly Gln Pro Glu Asn
            420                 425                 430

Asn Tyr Lys Thr Thr Pro Pro Val Leu Asp Ser Asp Gly Ser Phe Phe
        435                 440                 445

Leu Tyr Ser Lys Leu Thr Val Asp Lys Ser Arg Trp Gln Gln Gly Asn
    450                 455                 460

Val Phe Ser Cys Ser Val Met His Glu Ala Leu His Asn His Tyr Thr
465                 470                 475                 480

Gln Lys Ser Leu Ser Leu Ser Pro Gly Lys
                485                 490

<210>  108
<211>  487
<212>  PRT
<213>  Artificial Sequence

<220>
<223>  hu42v6n15v11-Fc

<400>  108

Gln Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Tyr Ser Asp Val
            20                  25                  30

Cys Met Gly Trp Phe Arg Gln Ala Pro Gly Lys Gly Leu Glu Gly Val
        35                  40                  45

Ala Ala Ile Asp Ser Val Gly Asn Thr Asn Tyr Ala Asp Ser Val Lys
    50                  55                  60

Gly Arg Phe Thr Ile Ser Lys Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Ala Gln Arg Cys Leu Gly Trp Val Pro Leu Val Pro His Glu Tyr Gly
            100                 105                 110

Tyr Trp Gly Gln Gly Thr Leu Val Thr Val Ser Ser Gln Val Gln Leu
        115                 120                 125

Val Glu Ser Gly Gly Gly Leu Val Gln Pro Gly Gly Ser Leu Arg Leu
    130                 135                 140

Ser Cys Ala Ala Ser Gly Tyr Thr Tyr Gly Ser Tyr Cys Met Ala Trp
145                 150                 155                 160

Phe Arg Gln Ala Pro Gly Lys Glu Arg Glu Gly Val Ala Gly Val Asp
                165                 170                 175

Ser Asp Gly Thr Thr Arg Tyr Ala Asp Ser Val Lys Gly Arg Phe Thr
            180                 185                 190

Ile Ser Arg Asp Asn Ser Glu Asn Thr Leu Tyr Leu Gln Met Asn Ser
        195                 200                 205

Leu Lys Pro Glu Asp Thr Ala Val Tyr Tyr Cys Ala Ala Gly Val Cys
    210                 215                 220

Gly Thr Gly Gly Ser Trp Tyr Glu Gly Asp Ala Gly Leu Phe Met Tyr
225                 230                 235                 240

Glu Phe Ala Tyr Trp Gly Gln Gly Thr Leu Val Thr Val Ser Ser Glu
                245                 250                 255

Pro Lys Ser Cys Asp Lys Thr His Thr Cys Pro Pro Cys Pro Ala Pro
            260                 265                 270

Glu Leu Leu Gly Gly Pro Ser Val Phe Leu Phe Pro Pro Lys Pro Lys
        275                 280                 285

Asp Thr Leu Met Ile Ser Arg Thr Pro Glu Val Thr Cys Val Val Val
    290                 295                 300

Asp Val Ser His Glu Asp Pro Glu Val Lys Phe Asn Trp Tyr Val Asp
305                 310                 315                 320

Gly Val Glu Val His Asn Ala Lys Thr Lys Pro Arg Glu Glu Gln Tyr
                325                 330                 335

Asn Ser Thr Tyr Arg Val Val Ser Val Leu Thr Val Leu His Gln Asp
            340                 345                 350

Trp Leu Asn Gly Lys Glu Tyr Lys Cys Lys Val Ser Asn Lys Ala Leu
        355                 360                 365

Pro Ala Pro Ile Glu Lys Thr Ile Ser Lys Ala Lys Gly Gln Pro Arg
    370                 375                 380

Glu Pro Gln Val Tyr Thr Leu Pro Pro Ser Arg Asp Glu Leu Thr Lys
385                 390                 395                 400

Asn Gln Val Ser Leu Thr Cys Leu Val Lys Gly Phe Tyr Pro Ser Asp
                405                 410                 415

Ile Ala Val Glu Trp Glu Ser Asn Gly Gln Pro Glu Asn Asn Tyr Lys
            420                 425                 430

Thr Thr Pro Pro Val Leu Asp Ser Asp Gly Ser Phe Phe Leu Tyr Ser
        435                 440                 445

Lys Leu Thr Val Asp Lys Ser Arg Trp Gln Gln Gly Asn Val Phe Ser
    450                 455                 460

Cys Ser Val Met His Glu Ala Leu His Asn His Tyr Thr Gln Lys Ser
465                 470                 475                 480

Leu Ser Leu Ser Pro Gly Lys
                485

<210> 109
<211> 483
<212> PRT
<213> Artificial Sequence

<220>
<223> hu42v6n7v11-Fc

<400> 109

Gln Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Tyr Ser Asp Val
            20                  25                  30

Cys Met Gly Trp Phe Arg Gln Ala Pro Gly Lys Gly Leu Glu Gly Val
        35                  40                  45

Ala Ala Ile Asp Ser Val Gly Asn Thr Asn Tyr Ala Asp Ser Val Lys
    50                  55                  60

Gly Arg Phe Thr Ile Ser Lys Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Ala Gln Arg Cys Leu Gly Trp Val Pro Leu Val Pro His Glu Tyr Gly
            100                 105                 110

Tyr Trp Gly Gln Gly Thr Leu Val Thr Val Ser Ser Gln Val Gln Leu
        115                 120                 125

Val Glu Ser Gly Gly Gly Leu Val Gln Pro Gly Gly Ser Leu Arg Leu
    130                 135                 140

Ser Cys Ala Ala Ser Gly Tyr Thr Tyr Ser Ser Tyr Cys Met Gly Trp
145                 150                 155                 160

Phe Arg Gln Ala Pro Gly Lys Gly Leu Glu Gly Val Ala Ala Ile Asp
                165                 170                 175

Ser Asp Gly Arg Thr Ser Tyr Ala Asp Phe Val Lys Gly Arg Phe Thr
            180                 185                 190

Ile Ser Lys Asp Asn Ser Lys Asn Thr Leu Tyr Leu Gln Met Asn Ser
        195                 200                 205

Leu Lys Pro Glu Asp Thr Ala Val Tyr Tyr Cys Ala Thr Glu Gln Arg
    210                 215                 220

Ile Gly Gly Ser Trp Tyr Asp Leu Cys Gly Gly Ala Asp Phe Gly Tyr
225                 230                 235                 240

Trp Gly Gln Gly Thr Leu Val Thr Val Ser Ser Glu Pro Lys Ser Cys
                245                 250                 255

Asp Lys Thr His Thr Cys Pro Pro Cys Pro Ala Pro Glu Leu Leu Gly
            260                 265                 270

Gly Pro Ser Val Phe Leu Phe Pro Pro Lys Pro Lys Asp Thr Leu Met
        275                 280                 285

Ile Ser Arg Thr Pro Glu Val Thr Cys Val Val Val Asp Val Ser His
    290                 295                 300

Glu Asp Pro Glu Val Lys Phe Asn Trp Tyr Val Asp Gly Val Glu Val
305                 310                 315                 320

His Asn Ala Lys Thr Lys Pro Arg Glu Glu Gln Tyr Asn Ser Thr Tyr
                325                 330                 335

Arg Val Val Ser Val Leu Thr Val Leu His Gln Asp Trp Leu Asn Gly
            340                 345                 350

Lys Glu Tyr Lys Cys Lys Val Ser Asn Lys Ala Leu Pro Ala Pro Ile
        355                 360                 365

Glu Lys Thr Ile Ser Lys Ala Lys Gly Gln Pro Arg Glu Pro Gln Val
    370                 375                 380

Tyr Thr Leu Pro Pro Ser Arg Asp Glu Leu Thr Lys Asn Gln Val Ser
385                 390                 395                 400

Leu Thr Cys Leu Val Lys Gly Phe Tyr Pro Ser Asp Ile Ala Val Glu
                405                 410                 415

Trp Glu Ser Asn Gly Gln Pro Glu Asn Asn Tyr Lys Thr Thr Pro Pro
            420                 425                 430

Val Leu Asp Ser Asp Gly Ser Phe Phe Leu Tyr Ser Lys Leu Thr Val
        435                 440                 445

Asp Lys Ser Arg Trp Gln Gln Gly Asn Val Phe Ser Cys Ser Val Met
    450                 455                 460

His Glu Ala Leu His Asn His Tyr Thr Gln Lys Ser Leu Ser Leu Ser
465                 470                 475                 480

Pro Gly Lys

## Description

### Technical Field

The invention relates to the field of biomedicine, and an anti-pertussis toxin (PT) single domain antibody and derivative protein thereof are disclosed. Specifically, a pertussis toxin-binding protein and use thereof are disclosed.

### Background

*Bordetella pertussis* (*B. pertussis*) is a Gram-negative bacterium that infects the upper respiratory tract to cause uncontrollable violent coughing. According to the World Health Organization, *Bordetella pertussis* infection causes an estimated 300,000 deaths worldwide every year, mainly among young unvaccinated infants. Babies with pertussis often need to be hospitalized in the pediatric intensive care unit, and their treatment usually involves mechanical ventilation. Pertussis in adults usually leads to chronic cough. The incidence of pertussis increased due to the exposure of unvaccinated and under-vaccinated individuals (including infants who have not been fully vaccinated), individuals whose immunity has weakened over time and asymptomatic carriers.

The previous pertussis vaccines were reported not to provide long-term protection. There is no approved therapeutic vaccine for pertussis. Antibiotics do not play a primary role in the course of pertussis due to not neutralizing pertussis toxin proteins, although *Bordetella pertussis* bacteria can be eliminated from respiratory tract by treatment. Furthermore, in the developing world, it is inconsistent and very difficult to obtain the existing pertussis vaccine (although defective). Therefore, a more effective treatment for pertussis is still needed.

### Summary of the Invention

In one aspect, the present invention provides a pertussis toxin-binding protein capable of specifically binding to pertussis toxin and comprising at least one immunoglobulin single variable domain comprising CDR1, CDR2 and CDR3 selected from the group consisting of:
(1) CDR1 shown in SEQ ID NO: 1, CDR2 shown in SEQ ID NO: 2, and CDR3 shown in SEQ ID NO: 3;
(2) CDR1 shown in SEQ ID NO: 4, CDR2 shown in SEQ ID NO: 5, and CDR3 shown in SEQ ID NO: 6;
(3) CDR1 shown in SEQ ID NO: 7, CDR2 shown in SEQ ID NO: 8, and CDR3 shown in SEQ ID NO: 9;
(4) CDR1 shown in SEQ ID NO: 10, CDR2 shown in SEQ ID NO: 11, and CDR3 shown in SEQ ID NO: 12;
(5) CDR1 shown in SEQ ID NO: 13, CDR2 shown in SEQ ID NO: 14, and CDR3 shown in SEQ ID NO: 15;
(6) CDR1 shown in SEQ ID NO: 16, CDR2 shown in SEQ ID NO: 17, and CDR3 shown in SEQ ID NO: 18;
(7) CDR1 shown in SEQ ID NO: 19, CDR2 shown in SEQ ID NO: 20, and CDR3 shown in SEQ ID NO: 21;
(8) CDR1 shown in SEQ ID NO: 22, CDR2 shown in SEQ ID NO: 23, and CDR3 shown in SEQ ID NO: 24;
(9) CDR1 shown in SEQ ID NO: 25, CDR2 shown in SEQ ID NO: 26, and CDR3 shown in SEQ ID NO: 27;
(10) CDR1 shown in SEQ ID NO: 28, CDR2 shown in SEQ ID NO: 29, and CDR3 shown in SEQ ID NO: 30;
(11) CDR1 shown in SEQ ID NO: 31, CDR2 shown in SEQ ID NO: 32, and CDR3 shown in SEQ ID NO: 33;
(12) CDR1 shown in SEQ ID NO: 34, CDR2 shown in SEQ ID NO: 35, and CDR3 shown in SEQ ID NO: 36;
(13) CDR1 shown in SEQ ID NO: 37, CDR2 shown in SEQ ID NO: 38, and CDR3 shown in SEQ ID NO: 39.

In one aspect, the present invention provides a nucleic acid molecule encoding the pertussis toxin binding protein of the present invention.

In one aspect, the present invention provides an expression vector comprising the nucleic acid molecule of the present invention operably linked to an expression regulatory element.

In one aspect, the present invention provides a recombinant cell comprising the nucleic acid molecule of the present invention or transformed with the expression vector of the present invention, and capable of expressing the pertussis toxin binding protein.

In one aspect, the present invention provides a method for producing the pertussis toxin binding protein of the present invention, comprising:
a) culturing the recombinant cell of the present invention under conditions allowing the expression of the pertussis toxin binding protein;
b) recovering the pertussis toxin binding protein expressed by the recombinant cells from the culture obtained from step a); and
c) optionally further purifying and/or modifying the pertussis toxin binding protein obtained from step b).

In one aspect, the present invention provides a pharmaceutical composition comprising the pertussis toxin binding protein of the present invention and a pharmaceutically acceptable carrier.

In one aspect, the present invention provides a method for treating *Bordetella pertussis* infection in a subject, the method comprising administering to the subject an effective amount of pertussis toxin binding protein of the present invention or pharmaceutical composition of the present invention.

In one aspect, the present invention provides a method for preventing *Bordetella pertussis* infection in a subject, the method comprising administering to the subject an effective amount of pertussis toxin binding protein of the present invention or pharmaceutical composition of the present invention.

In one aspect, the present invention provides a method for detecting the presence and/or amount of pertussis toxin in a biological sample, the method comprising:
(a) contacting the biological sample and the control sample with the pertussis toxin binding protein of the present invention respectively under conditions allowing the formation of complex between the pertussis toxin binding protein and pertussis toxin;
(b) detecting the formation of the complex,
wherein the difference in the complex formation between the biological sample and the control sample indicates the presence and/or amount of pertussis toxin in the sample.

In one aspect, the present invention provides a kit comprising the pertussis toxin binding protein of the present invention.

### Brief Description of the Drawings

Fig. 1 is a survival curve of protection against PT toxin with single-dose administration of 20 µg/dose.
Fig. 2 is a survival curve of protection against PT toxin with single-dose administration of 40 µg/dose.
Fig. 3 is a survival curve of protection against PT toxin with multiple-dose administration of 20 µg/dose.
Fig. 4 is a survival curve of protection against PT toxin with multiple-dose administration of 40 µg/dose.
Fig. 5 is a survival curve of protection against PT toxin with multiple-dose administration of 20 µg/dose or 40 µg/dose.
Fig. 6 shows the amino acid sequence alignment of iPT7 humanized variants.
Fig. 7 shows the amino acid sequence alignment of iPT15 humanized variants.
Fig. 8 shows the amino acid sequence alignment of iPT42 humanized variants.

### Detailed Description of the Invention

Unless otherwise indicated or defined, all used terms have the common meaning in the field, which will be understood by those skilled in the field. See, for example, the standard manual, such as Sambrook et al., "Molecular cloning: a laboratory manual" (2nd edition), volumes 1-3, Cold Spring Harbor Laboratory Press (1989); Lewin,"Genes IV", Oxford University Press, New York (1990); and Roitt et al., "Immunology" (2nd edition), Gover Medical Publishing, London, New York (1989), and the general prior art cited herein; in addition, unless otherwise stated, all the methods, steps, techniques and operations that are not specifically detailed may and have been performed in a manner known per se, which will be understood by those skilled in the art. Also see, for example, the standard manual, the above general prior art, and other references cited therein.

Unless otherwise specified, the terms "antibody" and "immunoglobulin" used interchangeably, whether referring to a heavy chain antibody or a conventional 4-chain antibody herein, are used as general terms to include a full-length antibody, its single chain and all parts, domains or fragments thereof (including but not limited to antigen-binding domains or fragments, such as VHH domains or VHNL domains, respectively). In addition, the term "sequence" as used herein (for example, in the terms "immunoglobulin sequence", "antibody sequence", "single variable domain sequence", "VHH sequence" or "protein sequence", etc.) should generally be understood to include not only the relevant amino acid sequence, but also the nucleic acid sequence or nucleotide sequence encoding the sequence, unless a more limited explanation is required herein.

As used herein, the term "domain" (of a polypeptide or protein) refers to a folded protein structure, which can maintain its tertiary structure independent of the rest of the protein. Generally speaking, the domain is responsible for the individual functional properties of the protein, and in many cases, it can be added, removed or transferred to other proteins without losing the functions of the rest of the protein and/or the domain.

As used herein, the term "immunoglobulin domain" refers to a spherical region of an antibody chain (for example, a chain of a conventional 4-chain antibody or a heavy chain antibody), or a polypeptide basically composed of such spherical regions. The immunoglobulin domain is characterized by maintaining the immunoglobulin folding characteristics of antibody molecules.

As used herein, the term "immunoglobulin variable domain" refers to an immunoglobulin domain which is basically composed of four "framework regions" called "framework region 1" or "FR1", "framework region 2" or "FR2", "framework region 3" or "FR3", and "framework region 4" or "FR4" in the field and hereinafter, respectively, wherein the framework regions are spaced apart by three "complementarity determining regions" called "complementarity determining region 1" or "CDR1", "complementarity determining region 2" or "CDR2" and "complementarity determining region 3" or "CDR3", respectively. Therefore, the general structure or sequence of immunoglobulin variable domain may be expressed as follows: FR1-CDR1-FR2-CDR2-FR3-CDR3-FR4. The immunoglobulin variable domain endows the antibody with specificity for antigen because of its antigen-binding site.

As used herein, the term "immunoglobulin single variable domain" refers to an immunoglobulin variable domain that can specifically bind to an epitope without pairing with other immunoglobulin variable domains. An example of immunoglobulin single variable domain within the meaning of the present invention is a "domain antibody", such as immunoglobulin single variable domains VH and VL (a VH domain and a VL domain). Another example of the immunoglobulin single variable domain is the "VHH domain" (or simply "VHH") of Camelidae as defined below.

The "VHH domain", also known as the heavy chain single domain antibody, VHH domain, VHH antibody fragment and VHH antibody, is the variable domain of antigen-binding immunoglobulin called "heavy chain antibody (i.e., an antibody devoid of light chains)" (Hamers-Casterman C, Atarhouch T, Muyldermans S, Robinson G, Hamers C, Songa EB, Bendahman N, Hamers R.: "Naturally occurring antibodies devoid of light chains"; Nature 363,446-448(1993))_{∘} The term "VHH domain" is used to distinguish the variable domain from the heavy chain variable domain (which is referred to herein as "VH domain") and the light chain variable domain (which is referred to herein as "VL domain") both existing in the conventional 4-chain antibody. The VHH domain specifically binds to the epitope without other antigen binding domains (this is contrary to the VH or VL domain in the conventional 4-chain antibody, in which case the epitope is recognized by the VL domain together with the VH domain). The VHH domain is a small, stable and efficient antigen recognition unit formed by a single immunoglobulin domain.

In the context of the present invention, the terms "heavy chain single domain antibody", "VHH domain", "VHH", "VHH domain", "VHH antibody fragment" and "VHH antibody" can be used interchangeably.

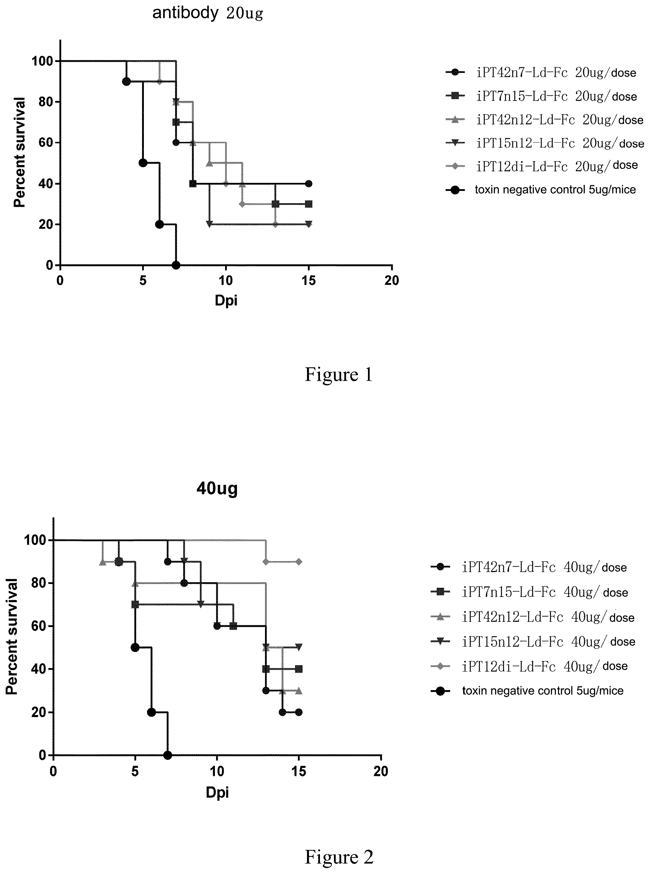

For example, as shown in Figure 2 in Riechmann and Muyldermans, J. Immunol. Methods 231,25-38 (1999), the amino acid residues used in the VHH domain of Camelidae can be numbered according to the general numbering method for VH domain given by Kabat et al. (Kabat et al. Sequences of Proteins of Immunological Interest, 5th Ed. Public Health Service, National Institutes of Health, Bethesda, Md. (1991)).

Alternative methods of numbering amino acid residues of the VH domain are also known in the art. For example, Chothia CDR refers to the position of the structural loop (Chothia and Lesk, J. mol. Biol. 196:901-917 (1987)). AbM CDR represents the compromise between Kabat hypervariable region and Chothia structural loop, and is used in the antibody modeling software Oxford Molecular's AbM. The "Contact" CDR is based on the analysis of the crystal structure of the available complex. The residues of CDR from each method are described as follows:

| **Loop** | **Kabat** | **AbM** | **Chothia** | **Contact** |
|---|---|---|---|---|
| LCDR1 | L24-L34 | L24-L34 | L26-L32 | L30-L36 |
| LCDR2 | L50-L56 | L50-L56 | L50-L52 | L46-L55 |
| LCDR3 | L89-L97 | L89-L97 | L91-L96 | L89-L96 |
| HCDR1 (Kabat numbering) | H31-H35B | H26-H35B | H26-H32 | H30-H35B |
| HCDR1 (Chothia numbering) | H31-H35 | H26-H35 | H26-H32 | H30-H35 |
| HCDR2 | H50-H65 | H50-H58 | H53-H55 | H47-H58 |
| HCDR3 | H95-H102 | H95-H102 | H96-H101 | H93-H101 |

However, it should be noted that, as well known for the VH domain and VHH domain in the art, the total number of amino acid residues in each CDR may be different and may not correspond to the total number of amino acid residues indicated by the Kabat numbering (that is, one or more positions according to the Kabat numbering system may not be occupied in the actual sequence, or the actual sequence may comprise more amino acid residues than allowed by the Kabat numbering). This means that, in general, the numbering according to Kabat may or may not correspond to the actual numbering of amino acid residues in the actual sequence.

For example, CDR can include an extended CDR, such as 24-36 or 24-34 (LCDR1), 46-56 or 50-56 (LCDR2) and 89-97 or 89-96 (LCDR3) in the VL; 26-35 (HCDR1), 50-65 or 49-65 (HCDR2) and 93-102, 94-102 or 95-102 (HCDR3) in the VH.

The total number of amino acid residues in the VHH domain will usually be in the range of 110 to 120, often between 112 and 115. However, it should be noted that smaller and longer sequences may also be suitable for the purpose described herein.

Other structural and functional properties of the VHH domain and a polypeptide containing the same can be summarized as follows:
The VHH domain, which has been naturally "designed" to functionally bind to the antigen devoid of the light chain variable domain and without interaction with the light chain variable domain, can be used as a single and relatively small functional antigen-binding structural unit, domain or polypeptide. This differentiates the VHH domain from the VH and VL domains of a conventional 4-chain antibody which are usually not suitable for practical application per se as a single antigen-binding protein or an immunoglobulin single variable domain, but need to be combined in some form or another form to provide functional antigen-binding units (such as in the form of conventional antibody fragments such as Fab fragments; or in the form of scFv composed of VH domains covalently linked to VL domains).

Because of these unique properties, the use of VHH domain-alone or as a part of a larger polypeptide-provides many significant advantages superior to that using conventional VH and VL domains, scFv or conventional antibody fragments (such as Fab- or F(ab')2- fragments): only a single domain is needed to bind to antigen with high affinity and selectivity, so that there is neither need to exist two separate domains nor need to ensure that these two domains exist in an appropriate spatial conformation and configuration (for example, scFv generally needs specially designed linkers); the VHH domain can be expressed from a single gene without post-translational folding or modification; the VHH domain can be easily transformed into multivalent and multispecific formats (formatting); the VHH domain is highly soluble and has no aggregation tendency; the VHH domain is highly stable to heat, pH, protease and other denaturants or conditions, and therefore can be prepared, stored or transported without using freezing equipment, thereby saving cost, time and environment; the VHH domain is easy to prepare and relatively cheap, even on the scale required for production; compared with a conventional 4-chain antibody and its antigen-binding fragments, the VHH domain is relatively small (about 15 kDa or 1/10 of the size of conventional IgG), which shows higher tissue permeability and can be administered at a higher dose, as still compared with the conventional 4-chain antibody and its antigen-binding fragments; the VHH domain can show the so-called cavity binding properties (especially due to its extended CDR3 loop compared with the conventional VH domain), thus reaching the targets and epitopes that the conventional 4-chain antibody and its antigen binding fragments cannot reach.

Methods for obtaining VHH binding to specific antigens or epitopes have been previously disclosed in the following documents: R. van der Linden et al., Journal of Immunological Methods, 240 (2000) 185 -195; Li et al., J Biol Chem., 287 (2012) 13713-13721; Deffar et al., African Journal of Biotechnology Vol. 8 (12), pp. 2645-2652, 17 June, 2009 and WO94/04678.

The VHH domain derived from Camelidae can be humanized by replacing one or more amino acid residues in the amino acid sequence of the original VHH sequence with one or more amino acid residues existing at the corresponding positions in the VH domain of a human conventional 4-chain antibody (also referred to as "sequence optimization" herein, besides humanization, "sequence optimization" can also encompass other modifications of the sequence by one or more mutations providing VHH improvement properties, such as deleting potential post-translational modification sites). A humanized VHH domain can contain one or more complete human framework sequences. The humanization may be achieved by humanization of amino acids on the protein surface (resurfacing) and/or CDR grafting to a universal framework, such as examples illustrated herein.

As used herein, the term "epitope" or "antigenic determinant" used interchangeably refers to any antigenic determinant on the antigen to which the complementary site of an antibody binds. Generally, antigenic determinants comprise chemically active surface groups of molecules, such as amino acids or sugar side chains, and usually have specific three-dimensional structure characteristics and specific charge characteristics. For example, the epitope usually includes at least 3, 4, 5, 6, 7, 8, 9, 10, 11, 12, 13, 14 or 15 continuous or discontinuous amino acids in a unique spatial conformation, which can be a "linear" epitope or "conformational" epitope. See, for example, Epitope mapping protocols in methods in molecular biology, Vol. 66, G.E. Morris, Ed. (1996). In the linear epitope, all interaction points between proteins and interacting molecules (such as antibodies) exist linearly along the primary amino acid sequence of proteins. In the conformational epitope, the interaction points exist across protein amino acid residues separately from one another.

Many epitope mapping techniques well known in the art can be used to identify the epitope of a given antigen. See, for example, Epitope Mapping Protocols in Methods in Molecular Biology, Vol. 66, G.E. Morris, Ed. (1996). For example, linear epitopes can be determined by, for example, the method as follows: synthesizing a large number of peptides on a solid support at the same time, wherein these peptides correspond to various parts of protein molecules, and reacting these peptides while still being linked to the support with antibodies. These techniques are known in the art and described in, for example, U.S. Pat. No.4,708,871; Geysen et al. (1984) Proc. Natl. Acad. Sci. USA 81:3998-4002; Geysen et al. (1986) Molec. Immunol. 23:709-715. Similarly, a conformational epitope can be identified by determining the spatial configuration of amino acids by, for example, X-ray crystallography and 2-dimensional nuclear magnetic resonance. See, for example, Epitope Mapping Protocols (supra).

Antibodies can be screened for competitively binding to the same epitope using conventional techniques known to those skilled in the art. For example, competitive and cross-competitive studies may be conducted to obtain antibodies that compete or cross-compete with each other for binding to antigens. A high-throughput method for obtaining antibodies that bind to the same epitope based on their cross-competition is described in the international patent application WO03/48731. Therefore, the conventional techniques known to those skilled in the art may be used to obtain antibodies and antigen-binding fragments thereof which compete with the antibody molecules of the present invention for binding to the same epitope on pertussis toxin.

Generally speaking, the term "specificity" refers to the number of different types of antigens or epitopes that a specific antigen-binding molecule or antigen-binding protein (such as the immunoglobulin single variable domain of the present invention) can bind to. The specificity of antigen binding proteins can be determined based on their affinity and/or affinity. The affinity expressed by the dissociation equilibrium constant (KD) between an antigen and an antigen-binding protein is a measure of the binding strength between an epitope and an antigen-binding site on the antigen-binding protein: the smaller KD value, the stronger the binding strength between the epitope and the antigen-binding protein (or, the affinity can also be expressed as the association constant (KA), which is 1/KD). As will be understood by those skilled in the art, the affinity can be determined in a known manner, depending on the specific antigen of interest. The affinity is a measure of the binding strength between an antigen-binding protein (such as immunoglobulin, antibody, immunoglobulin single variable domain or polypeptide comprising the same) and the associated antigen. The affinity is related to both the affinity between the antigen-binding sites on the antigen-binding protein and the number of associated binding sites present on the antigen-binding protein.

As used herein, the term "pertussis toxin binding protein" means any protein capable of specifically binding to pertussis toxin. The pertussis toxin binding protein may include an antibody against pertussis toxin as defined herein. The pertussis toxin binding protein also encompasses immunoglobulin superfamily antibodies (IgSF) or CDR grafted molecules.

"Pertussis toxin" is a multi-subunit protein toxin derived from Bordetella pertussis (B. pertussis), which may act specifically on G proteins to inhibit the function of signaling pathway of G proteins. The pertussis toxin comprises subunit S1, subunit S2, subunit S3, subunit S4 and subunit S5, the amino acid sequences of which were logged in the UniProtKB database, with the corresponding accession numbers being: P04977 ,P04978, P04979, P0A3R5 and P04981, respectively.

The "pertussis toxin binding protein" of the present invention may comprise at least one immunoglobulin single variable domain (e.g., VHH) that binds to pertussis toxin. In some embodiments, the "pertussis toxin binding molecule" of the present invention may comprise 2, 3, 4 or more immunoglobulin single variable domains (e.g., VHH) that bind to pertussis toxin. In addition to the immunoglobulin single variable domain binding to pertussis toxin, the pertussis toxin binding protein of the present invention may still comprise a linker and/or a moiety with effector function, such as a half-life extending moiety (for example, the immunoglobulin single variable domain binding to serum albumin), and/or a fusion partner (e.g., serum albumin) and/or a conjugated polymer (e.g., PEG) and/or a Fc region. In some embodiments, the "pertussis toxin binding protein" of the present invention also encompasses bispecific antibodies, which contain immunoglobulin single variable domains that bind to different antigens.

Generally, the pertussis toxin binding protein of the present invention may bind to the antigen of interest (i.e. pertussis toxin) with a dissociation constant(KD) of preferably 10⁻⁷-10⁻¹⁰ mol/L (M), more preferably 10⁻⁸-10⁻¹⁰ mol/L, even more preferably 10⁻⁹-10⁻¹⁰ mol/L or even more less and/or with an association constant (KA) of at least 10⁷ M⁻¹, preferably at least 10⁸ M⁻¹, more preferably at least 10⁹ M⁻¹, even more preferably at least 10¹⁰ M⁻¹, as measured by Biacore or KinExA or Fortibio assay. Any KD value greater than 10⁻⁴ m is generally regarded as an indicator of nonspecific binding. Specific binding of antigen-binding proteins to antigens or epitopes can be determined in any known suitable way, including, for example, surface plasmon resonance (SPR) assay, Scatchard assay and/or competitive binding assay (such as radioimmunoassay (RIA), enzyme immunoassay (EIA) and sandwich and competitive assay) described herein.

The amino acid residues will be represented according to the standard three-letter or single-letter amino acid code as well known and agreed in the art. When comparing two amino acid sequences, the term "amino acid difference" refers to an addition, deletion or substitution of a specified number of amino acid residues at a certain position in the reference sequence compared with another sequence. In the case of substitution, the substitution will preferably be a conservative amino acid substitution, which means that the amino acid residue is replaced by another amino acid residue with similar chemical structure, which has little or no influence on the functions, activity or other biological properties of the polypeptide. The conservative amino acid substitution is well known in the art, for example, the conservative amino acid substitution is preferably that one amino acid in the following groups (i)-(v) is replaced by another amino acid residue in the same group: (i) smaller aliphatic nonpolar or weakly polar residues: Ala, Ser, Thr, Pro and Gly; (ii) polar negatively charged residues and their (uncharged) amides: Asp, Asn, Glu and Gln; (iii) polar positively charged residues: His, Arg and Lys; (iv) larger aliphatic nonpolar residues: Met, Leu, Ile, Val and Cys; and (v) aromatic residues: Phe, Tyr and Trp. Particularly preferred conservative amino acid substitutions are as follows: Ala-to-Gly/Ser substitution; Arg-to-Lys substitution; Asn-to-Gln/His substitution; Asp-to-Glu substitution; Cys-to-Ser substitution ; Gln-to-Asn substitution; Glu-to-Asp substitution; Gly-Ala/Pro substitution; His-to-Asn/Gln substitution; Ile-to-Leu/Val substitution; Leu-to-Ile/Val substitution; Lys-to-Arg/Gln/Glu substitution; Met-to-Leu/Tyr/Ile substitution; Phe-to-Met/Leu/Tyr substitution; Ser-to-Thr substitution; Thr-to-Ser substitution; Trp-to-Tyr substitution; Tyr-to-Trp/Phe substitution; Val-to-Ile/Leu substitution.

"Sequence identity" between two polypeptide sequences indicates the percentage of identical amino acids between the two sequences. "Sequence similarity" indicates the percentage of amino acids that are the same or represent conservative amino acid substitutions. A method for evaluating the degree of sequence identity between amino acids or nucleotides is known to those skilled in the art. For example, the amino acid sequence identity is usually measured using a sequence analysis software. For example, a BLAST program of NCBI's database may be applied to determine the identity. For determination of sequence identity, see, for example, Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987 and Sequence Analysis Primer, Gribskov, M. and Devereux, J., eds., M Stockton Press, New York, 1991.

Compared with the natural biological source of a polypeptide or nucleic acid molecule and/or the reaction medium or culture medium used for obtaining the polypeptide or nucleic acid molecule, when the polypeptide or nucleic acid molecule has been separated from at least one other usually associated component (such as another protein/polypeptide, another nucleic acid, another biological component or macromolecule or at least one pollutant, impurity or trace component) in the source or medium (culture medium), the polypeptide or nucleic acid molecule is regarded as "isolated". In particular, a polypeptide or nucleic acid molecule is considered as "isolated" when it has been purified at least 2 times, especially at least 10 times, more particularly at least 100 times and up to 1000 times or more. The "isolated" polypeptide or nucleic acid molecules are preferably substantially homogeneous as determined by suitable techniques (for example, suitable chromatographic techniques, such as polyacrylamide gel electrophoresis).

An "effective amount" means that the amount of pertussis toxin binding protein or pharmaceutical composition of the present invention may result in a decrease in the severity of disorder symptoms, an increase in the frequency and duration of the asymptomatic period of the disorder, or the prevention of injury or disability as a result of suffering from the disorder.

As used herein, the term "subject" refers to a mammal, especially a primate mammal, in particular a human.

### The pertussis toxin binding protein of the present invention

The invention provides a pertussis toxin binding protein, which comprises at least one immunoglobulin single variable domain capable of specifically binding to pertussis toxin.

In some embodiments, the at least one immunoglobulin single variable domain comprises CDR1, CDR2 and CDR3 in VHH shown in any one of SEQ ID NOs: 40-52. The CDRs can be Kabat CDRs, AbM CDRs, Chothia CDRs or Contact CDRs. In some embodiments, the CDRs are Kabat CDR.

In some embodiments, the at least one immunoglobulin single variable domain comprises CDR1, CDR2 and CDR3 selected from the group consisting of:
(1) CDR1 shown in SEQ ID NO: 1, CDR2 shown in SEQ ID NO: 2, and CDR3 shown in SEQ ID NO: 3 (corresponding to CDRs of iPT 3);
(2) CDR1 shown in SEQ ID NO: 4, CDR2 shown in SEQ ID NO: 5, and CDR3 shown in SEQ ID NO: 6 (corresponding to CDRs of iPT 7);
(3) CDR1 shown in SEQ ID NO: 7, CDR2 shown in SEQ ID NO: 8, and CDR3 shown in SEQ ID NO: 9 (corresponding to CDRs of iPT 12);
(4) CDR1 shown in SEQ ID NO: 10, CDR2 shown in SEQ ID NO: 11, and CDR3 shown in SEQ ID NO: 12 (corresponding to CDRs of iPT 13);
(5) CDR1 shown in SEQ ID NO: 13, CDR2 shown in SEQ ID NO: 14, and CDR3 shown in SEQ ID NO: 15 (corresponding to CDRs of iPT 15);
(6) CDR1 shown in SEQ ID NO: 16, CDR2 shown in SEQ ID NO: 17, and CDR3 shown in SEQ ID NO: 18 (corresponding to CDRs of iPT 20);
(7) CDR1 shown in SEQ ID NO: 19, CDR2 shown in SEQ ID NO: 20, and CDR3 shown in SEQ ID NO: 21 (corresponding to CDRs of iPT 21);
(8) CDR1 shown in SEQ ID NO: 22, CDR2 shown in SEQ ID NO: 23, and CDR3 shown in SEQ ID NO: 24 (corresponding to CDRs of iPT 22);
(9) CDR1 shown in SEQ ID NO: 25, CDR2 shown in SEQ ID NO: 26, and CDR3 shown in SEQ ID NO: 27 (corresponding to CDRs of iPT 26);
(10) CDR1 shown in SEQ ID NO: 28, CDR2 shown in SEQ ID NO: 29, and CDR3 shown in SEQ ID NO: 30 (corresponding to CDRs of iPT 28);
(11) CDR1 shown in SEQ ID NO: 31, CDR2 shown in SEQ ID NO: 32, and CDR3 shown in SEQ ID NO: 33 (corresponding to CDRs of iPT 35);
(12) CDR1 shown in SEQ ID NO: 34, CDR2 shown in SEQ ID NO: 35, and CDR3 shown in SEQ ID NO: 36 (corresponding to CDRs of iPT 36);
(13) CDR1 shown in SEQ ID NO: 37, CDR2 shown in SEQ ID NO: 38, and CDR3 shown in SEQ ID NO: 39 (corresponding to CDRs of iPT 42).

In some embodiments, the at least one immunoglobulin single variable domain in the pertussis toxin binding protein of the present invention is a VHH. In some embodiments, the VHH comprises any one of the amino acid sequences shown in SEQ ID NOs: 40 -52.

In some embodiments, the at least one immunoglobulin single variable domain in the pertussis toxin binding protein of the present invention is a humanized VHH.

In some embodiments, the at least one immunoglobulin single variable domain in the pertussis toxin binding protein of the present invention is a humanized VHH, which comprises an amino acid sequence having at least 80%, preferably at least 90%, more preferably at least 95%, even more preferably at least 99% sequence identity to any one of the sequences shown in SEQ ID NOs: 40-52. In some embodiments, the humanized VHH comprises one or more amino acid substitutions as compared to any one of the amino acid sequences shown in SEQ ID NOs: 40 -52. For example, 1, 2, 3, 4, 5, 6, 7, 8, 9 or 10 conservative amino acid substitutions are comprised.

In some embodiments, at least one immunoglobulin single variable domain in the pertussis toxin binding protein of the present invention is a humanized VHH, wherein the humanized VHH comprises any one amino acid sequence shown in SEQ ID NOs: 53-85.

In some embodiments, the pertussis toxin binding protein comprises one immunoglobulin single variable domain that specifically binds to pertussis toxin.

In some embodiments, the pertussis toxin binding protein comprises at least two, such as 2, 3, 4 or more immunoglobulin single variable domains that specifically bind to pertussis toxin.

In some embodiments, the at least two immunoglobulin single variable domains bind to the same epitope or compete or partially compete for binding to the same epitope, for example, the at least two immunoglobulin single variable domains are the same.

In some embodiments, the at least two immunoglobulin single variable domains bind to different epitopes or do not compete for binding to the same epitope.

Whether two antibody or immunoglobulin single variable domains bind to or compete for binding to the same epitope can be determined by epitope binning using Bio-Layer Interferometry (BLI), as exemplified in the examples of the present invention.

In some embodiments, the at least two immunoglobulin single variable domains that specifically bind to pertussis toxin are directly connected to each other.

In some embodiments, the at least two immunoglobulin single variable domains that specifically bind to pertussis toxin are interconnected by a linker. The linker may be a nonfunctional amino acid sequence having 1-20 or more amino acids in length, without secondary and above structure. For example, the linker is a flexible linker, such as GGGGS, GS, GAP, (GGGGS) x 3, and so on.

In some embodiments, the pertussis toxin binding protein comprises a first immunoglobulin single variable domain and a second immunoglobulin single variable domain, wherein the first immunoglobulin single variable domain is located at the N-terminal of the second immunoglobulin single variable domain, and
wherein the first immunoglobulin single variable domain comprises CDR1 shown in SEQ ID NO: 4, CDR2 shown in SEQ ID NO: 5 and CDR3 shown in SEQ ID NO: 6, and the second immunoglobulin single variable domain comprises CDR1 shown in SEQ ID NO: 13, CDR2 shown in SEQ ID NO: 14 and CDR3 shown in SEQ ID NO: 15; or
wherein the first immunoglobulin single variable domain comprises CDR1 shown in SEQ ID NO: 13, CDR2 shown in SEQ ID NO: 14 and CDR3 shown in SEQ ID NO: 15, and the second immunoglobulin single variable domain comprises CDR1 shown in SEQ ID NO: 4, CDR2 shown in SEQ ID NO: 5 and CDR3 shown in SEQ ID NO: 6; or
wherein the first immunoglobulin single variable domain comprises CDR1 shown in SEQ ID NO: 4, CDR2 shown in SEQ ID NO: 5 and CDR3 shown in SEQ ID NO: 6, and the second immunoglobulin single variable domain comprises CDR1 shown in SEQ ID NO: 22, CDR2 shown in SEQ ID NO: 23 and CDR3 shown in SEQ ID NO: 24; or
wherein the first immunoglobulin single variable domain comprises CDR1 shown in SEQ ID NO: 22, CDR2 shown in SEQ ID NO: 23 and CDR3 shown in SEQ ID NO: 24, and the second immunoglobulin single variable domain comprises CDR1 shown in SEQ ID NO: 4, CDR2 shown in SEQ ID NO: 5 and CDR3 shown in SEQ ID NO: 6; or
wherein the first immunoglobulin single variable domain comprises CDR1 shown in SEQ ID NO: 4, CDR2 shown in SEQ ID NO: 5 and CDR3 shown in SEQ ID NO: 6, and the second immunoglobulin single variable domain comprises CDR1 shown in SEQ ID NO: 37, CDR2 shown in SEQ ID NO: 38 and CDR3 shown in SEQ ID NO: 39; or
wherein the first immunoglobulin single variable domain comprises CDR1 shown in SEQ ID NO: 37, CDR2 shown in SEQ ID NO: 38 and CDR3 shown in SEQ ID NO: 39, and the second immunoglobulin single variable domain comprises CDR1 shown in SEQ ID NO: 4, CDR2 shown in SEQ ID NO: 5 and CDR3 shown in SEQ ID NO: 6; or
wherein the first immunoglobulin single variable domain comprises CDR1 shown in SEQ ID NO: 7, CDR2 shown in SEQ ID NO: 8 and CDR3 shown in SEQ ID NO: 9, and the second immunoglobulin single variable domain comprises CDR1 shown in SEQ ID NO: 31, CDR2 shown in SEQ ID NO: 32 and CDR3 shown in SEQ ID NO: 33; or
wherein the first immunoglobulin single variable domain comprises CDR1 shown in SEQ ID NO: 31, CDR2 shown in SEQ ID NO: 32 and CDR3 shown in SEQ ID NO: 33, and the second immunoglobulin single variable domain comprises CDR1 shown in SEQ ID NO: 7, CDR2 shown in SEQ ID NO: 8 and CDR3 shown in SEQ ID NO: 9; or
wherein the first immunoglobulin single variable domain comprises CDR1 shown in SEQ ID NO: 13, CDR2 shown in SEQ ID NO: 14 and CDR3 shown in SEQ ID NO: 15, and the second immunoglobulin single variable domain comprises CDR1 shown in SEQ ID NO: 31, CDR2 shown in SEQ ID NO: 32 and CDR3 shown in SEQ ID NO: 33; or
wherein the first immunoglobulin single variable domain comprises CDR1 shown in SEQ ID NO: 13, CDR2 shown in SEQ ID NO: 14 and CDR3 shown in SEQ ID NO: 15, and the second immunoglobulin single variable domain comprises CDR1 shown in SEQ ID NO: 37, CDR2 shown in SEQ ID NO: 38 and CDR3 shown in SEQ ID NO: 39; or
wherein the first immunoglobulin single variable domain comprises CDR1 shown in SEQ ID NO: 31, CDR2 shown in SEQ ID NO: 32 and CDR3 shown in SEQ ID NO: 33, and the second immunoglobulin single variable domain comprises CDR1 shown in SEQ ID NO: 37, CDR2 shown in SEQ ID NO: 38 and CDR3 shown in SEQ ID NO: 39; or
wherein the first immunoglobulin single variable domain comprises CDR1 shown in SEQ ID NO: 7, CDR2 shown in SEQ ID NO: 8 and CDR3 shown in SEQ ID NO: 9, and the second immunoglobulin single variable domain comprises CDR1 shown in SEQ ID NO: 13, CDR2 shown in SEQ ID NO: 14 and CDR3 shown in SEQ ID NO: 15; or
wherein the first immunoglobulin single variable domain comprises CDR1 shown in SEQ ID NO: 13, CDR2 shown in SEQ ID NO: 14 and CDR3 shown in SEQ ID NO: 15, and the second immunoglobulin single variable domain comprises CDR1 shown in SEQ ID NO: 7, CDR2 shown in SEQ ID NO: 8 and CDR3 shown in SEQ ID NO: 9; or
wherein the first immunoglobulin single variable domain comprises CDR1 shown in SEQ ID NO: 7, CDR2 shown in SEQ ID NO: 8 and CDR3 shown in SEQ ID NO: 9, and the second immunoglobulin single variable domain comprises CDR1 shown in SEQ ID NO: 37, CDR2 shown in SEQ ID NO: 38 and CDR3 shown in SEQ ID NO: 39; or
wherein the first immunoglobulin single variable domain comprises CDR1 shown in SEQ ID NO: 37, CDR2 shown in SEQ ID NO: 38 and CDR3 shown in SEQ ID NO: 39, and the second immunoglobulin single variable domain comprises CDR1 shown in SEQ ID NO: 7, CDR2 shown in SEQ ID NO: 8 and CDR3 shown in SEQ ID NO: 9.

In some embodiments, the pertussis toxin binding protein comprises a first immunoglobulin single variable domain and a second immunoglobulin single variable domain, wherein the first immunoglobulin single variable domain is located at the N-terminal of the second immunoglobulin single variable domain, and
the first immunoglobulin single variable domain comprises the amino acid sequence shown in one of SEQ ID NOs: 41, 53-63 and the second immunoglobulin single variable domain comprises the amino acid sequence shown in one of SEQ ID NOs: 44, 64-74; or
the first immunoglobulin single variable domain comprises the amino acid sequence shown in one of SEQ ID NOs: 44, 64-74 and the second immunoglobulin single variable domain comprises the amino acid sequence shown in one of SEQ ID NOs: 41, 53-63; or
the first immunoglobulin single variable domain comprises the amino acid sequence shown in one of SEQ ID NOs: 41, 53-63 and the second immunoglobulin single variable domain comprises the amino acid sequence shown in SEQ ID NO: 47; or
the first immunoglobulin single variable domain comprises the amino acid sequence shown in SEQ ID NO: 47 and the second immunoglobulin single variable domain comprises the amino acid sequence shown in one of SEQ ID NOs: 41, 53-63; or
the first immunoglobulin single variable domain comprises the amino acid sequence shown in one of SEQ ID NOs: 41, 53-63 and the second immunoglobulin single variable domain comprises the amino acid sequence shown in one of SEQ ID NOs: 52, 75-85; or
the first immunoglobulin single variable domain comprises the amino acid sequence shown in one of SEQ ID NOs: 52, 75-85 and the second immunoglobulin single variable domain comprises the amino acid sequence shown in one of SEQ ID NOs: 41, 53-63; or
the first immunoglobulin single variable domain comprises the amino acid sequence shown in SEQ ID NO: 42 and the second immunoglobulin single variable domain comprises the amino acid sequence shown in SEQ ID NO: 50; or
the first immunoglobulin single variable domain comprises the amino acid sequence shown in SEQ ID NO: 50 and the second immunoglobulin single variable domain comprises the amino acid sequence shown in SEQ ID NO: 42; or
the first immunoglobulin single variable domain comprises the amino acid sequence shown in one of SEQ ID NOs: 44, 64-74 and the second immunoglobulin single variable domain comprises the amino acid sequence shown in SEQ ID NO: 50; or
the first immunoglobulin single variable domain comprises the amino acid sequence shown in one of SEQ ID NOs: 44, 64-74 and the second immunoglobulin single variable domain comprises the amino acid sequence shown in one of SEQ ID NOs: 52, 75-85; or
the first immunoglobulin single variable domain comprises the amino acid sequence shown in SEQ ID NO: 50 and the second immunoglobulin single variable domain comprises the amino acid sequence shown in one of SEQ ID NOs: 52, 75-85; or
the first immunoglobulin single variable domain comprises the amino acid sequence shown in SEQ ID NO: 42 and the second immunoglobulin single variable domain comprises the amino acid sequence shown in one of SEQ ID NOs: 44, 64-74; or
the first immunoglobulin single variable domain comprises the amino acid sequence shown in one of SEQ ID NOs: 44, 64-74 and the second immunoglobulin single variable domain comprises the amino acid sequence shown in SEQ ID NO: 42; or
the first immunoglobulin single variable domain comprises the amino acid sequence shown in SEQ ID NO: 42 and the second immunoglobulin single variable domain comprises the amino acid sequence shown in one of SEQ ID NOs: 52, 75-85; or
the first immunoglobulin single variable domain comprises the amino acid sequence shown in one of SEQ ID NOs: 52, 75-85 and the second immunoglobulin single variable domain comprises the amino acid sequence shown in SEQ ID NO: 42.

In some embodiments, the pertussis toxin binding protein comprises a first immunoglobulin single variable domain and a second immunoglobulin single variable domain, wherein the first immunoglobulin single variable domain is located at the N-terminal of the second immunoglobulin single variable domain, and
the first immunoglobulin single variable domain comprises the amino acid sequence shown in SEQ ID NO: 63 and the second immunoglobulin single variable domain comprises the amino acid sequence shown in SEQ ID NO: 70; or
the first immunoglobulin single variable domain comprises the amino acid sequence shown in SEQ ID NO: 63 and the second immunoglobulin single variable domain comprises the amino acid sequence shown in SEQ ID NO: 74; or
the first immunoglobulin single variable domain comprises the amino acid sequence shown in SEQ ID NO: 80 and the second immunoglobulin single variable domain comprises the amino acid sequence shown in SEQ ID NO: 74; or
the first immunoglobulin single variable domain comprises the amino acid sequence shown in SEQ ID NO: 80 and the second immunoglobulin single variable domain comprises the amino acid sequence shown in SEQ ID NO: 63.

In some embodiments, the pertussis toxin binding protein of the present invention comprises the amino acid sequence selected from SEQ ID NOs: 87-105.

In some embodiments, the pertussis toxin binding protein of the present invention comprises an immunoglobulin Fc region in addition to at least one immunoglobulin single variable domain capable of specifically binding to pertussis toxin. The inclusion of immunoglobulin Fc region in the pertussis toxin binding protein of the present invention allows for the formation of dimer molecules by the binding proteins, while prolonging the half-life of the binding proteins in vivo. The Fc region may be from different subtypes of immunoglobulin, such as IgG (e.g., an IgG1, IgG2, IgG3, or IgG4 subtype), IgA1, IgA2, IgD, IgE, or IgM. The immunoglobulin Fc region generally includes the hinge region or part of the hinge region, CH2 region and CH3 region of the immunoglobulin constant region.

In some embodiments, mutations can be introduced into the wild-type Fc sequence to change the associated activity mediated by Fc. The mutations include, but are not limited to: a) mutations that change Fc-mediated CDC activity; b) mutations that change Fc-mediated ADCC activity; or c) mutations that change the half-life in vivo mediated by FcRn. Such mutations are described in the following documents: Leonard G Presta, Current Opinion in Immunology 2008, 20:460-470; Esohe E. Idusogie et al., J Immunol 2000, 164: 4178-4184; RAPHAEL A. CLYNES et al., Nature Medicine, 2000, Volume 6, Number 4: 443-446; Paul R. Hinton et al., J Immunol, 2006, 176:346-356. For example, a mutation of 1, 2, 3, 4, 5, 6, 7, 8, 9 or 10 amino acids in CH2 region can be used to increase or remove the activity of ADCC or CDC mediated by Fc or to increase or decrease the affinity of FcRn. In addition, the stability of the protein can be increased by a mutation of 1, 2, 3, 4, 5, 6, 7, 8, 9 or 10 amino acids in the hinge region.

In some embodiments, mutations can be introduced into the Fc sequence to make the mutated Fc more easier to form a homodimer or heterodimer. For example, the knob-hole model mentioned in Ridgway, Presta et al. 1996 and Carter 2001, makes it easier to form heterodimers between different Fc mutations, which makes use of spatial effects of amino acid side chain groups at Fc contact interfaces; for another example, in CN 102558355A or CN 103388013A, by changing the charge of amino acids at Fc contact interfaces to change the ionic interaction between Fc contact interfaces, it is easier to form heterodimers between different Fc mutation pairs (CN 102558355A), or form homodimers between Fcs with the same mutation (CN 103388013A).

The immunoglobulin Fc region is preferably the human immunoglobulin Fc region, more preferably the Fc region of human IgG1. In some embodiments, the amino acid sequence of the immunoglobulin Fc region is shown in SEQ ID NO: 86.

In some embodiments, in the pertussis toxin binding protein of the present invention, the at least one immunoglobulin single variable domain capable of specifically binding to pertussis toxin is directly linked to the immunoglobulin Fc region or linked via a linker. The linker may be a nonfunctional amino acid sequence having 1-20 or more amino acids in length, without no secondary and above structure. For example, the linker is a flexible linker, such as GGGGS, GS, GAP, and so on. In some embodiments, the immunoglobulin Fc region is located at the C-terminal of the at least one immunoglobulin single variable domain that specifically binds to pertussis toxin.

In some embodiments, the pertussis toxin binding protein of the present invention comprises a first amino acid sequence selected from one of SEQ ID NOs: 40-85 and 87-105, and a second amino acid sequence shown in SEQ ID NO: 86 directly linked to the first amino acid sequence or linked via a linker, wherein the second amino acid sequence is located at the C-terminal of the first amino acid sequence.

In some embodiments, the pertussis toxin binding protein of the present invention comprises the amino acid sequence selected from SEQ ID NOs: 106-109.

In some embodiments, the pertussis toxin binding protein of the present invention comprises an immunoglobulin single variable domain that specifically binds to pertussis toxin, which is directly connected or connected via a linker to an immunoglobulin Fc region which allows the pertussis toxin binding protein to form a dimer molecule comprising two pertussis toxin binding domains. Such pertussis toxin binding protein is also called a bivalent pertussis toxin binding protein. In some embodiments, the dimer is a homodimer.

In some embodiments, the pertussis toxin binding protein of the present invention comprises two immunoglobulin single variable domains that specifically bind to pertussis toxin, which are directly interconnected or interconnected via a linker, and an immunoglobulin Fc region which allows the pertussis toxin binding protein to form a dimer molecule comprising four pertussis toxin binding domains. Such pertussis toxin binding protein is also called a tetravalent pertussis toxin binding protein. In some embodiments, the dimer is a homodimer.

### Nucleic acid, vector and host cell

In another aspect, the invention relates to a nucleic acid molecule that encodes the pertussis toxin-binding proteins of the invention. The nucleic acid of the invention may be RNA, DNA or cDNA. According to one embodiment of the invention, the nucleic acid of the invention is in essentially isolated form.

The nucleic acid of the invention may also be in the form of, may be present in and/or may be part of a vector, such as for example a plasmid, cosmid or YAC. The vector may especially be an expression vector, i.e. a vector that can provide for expression of the pertussis toxin-binding protein in vitro and/or in vivo (i.e. in a suitable host cell, host organism and/or expression system). Such expression vector generally comprises at least one nucleic acid of the invention that is operably linked to one or more suitable regulatory elements, such as promoter(s), enhancer(s), terminator(s), and the like. Such elements and their selection in view of expression of a specific sequence in a specific host are common knowledge of the skilled person. Specific examples of regulatory elements and other elements useful or necessary for expressing pertussis toxin-binding protein of the invention include such as promoters, enhancers, terminators, integration factors, selection markers, leader sequences, reporter genes, and the like.

The nucleic acids of the invention may be prepared or obtained in a manner known per se (e.g. by automated DNA synthesis and/or recombinant DNA technology), based on the information on the amino acid sequences for the polypeptides of the invention given herein, and/or can be isolated from a suitable natural source.

In another aspect, the invention relates to recombination host cells that express or are capable of expressing one or more pertussis toxin-binding protein of the invention; and/or that contain a nucleic acid of the invention. According to a particularly preferred embodiment, said host cells are bacterial cells; other useful cells are yeast cells, fungal cells or mammalian cells.

Suitable bacterial cells include cells from gram-negative bacterial strains such as strains of Escherichia coli, Proteus, and Pseudomonas, and gram-positive bacterial strains such as strains of Bacillus, Streptomyces, Staphylococcus, and Lactococcus. Suitable fungal cell include cells from species of Trichoderma, Neurospora, and Aspergillus.

Suitable fungal cell include cells from species of Trichoderma, Neurospora, and Aspergillus. Suitable yeast cells include cells from species of Saccharomyces (for example, Saccharomyces cerevisiae), Schizosaccharomyces (for example, Schizosaccharomyces pombe), Pichia (for example, Pichia pastoris and Pichia methanolica), and Hansenula.

Suitable mammalian cells include for example HEK293 cells, CHO cells, BHK cells, HeLa cells, COS cells, and the like.

However, amphibian cells, insect cells, plant cells, and any other cells used in the art for the expression of heterologous proteins can be used as well.

The invention further provides methods of manufacturing a pertussis toxin-binding protein of the invention, such methods generally comprise the steps of:
- culturing host cells of the invention under conditions that allow expression of the pertussis toxin-binding protein of the invention; and
- recovering the pertussis toxin-binding protein expressed by the host cells from the culture; and
- optionally further purifying and/or modifying the pertussis toxin-binding protein of the invention.

Pertussis toxin-binding proteins of the invention may be produced in a cell as set out above either intracellullarly (e.g. in the cytosol, in the periplasma or in inclusion bodies) and then isolated from the host cells and optionally further purified; or they can be produced extracellularly (e.g. in the medium in which the host cells are cultured) and then isolated from the culture medium and optionally further purified.

Methods and reagents used for the recombinant production of polypeptides, such as specific suitable expression vectors, transformation or transfection methods, selection markers, methods of induction of protein expression, culture conditions, and the like, are known in the art. Similarly, protein isolation and purification techniques useful in a method of manufacture of a pertussis toxin-binding protein of the invention are well known to the skilled person.

However, the pertussis toxin-binding proteins of the invention can also be obtained by other methods for production of proteins known in the art, such as, chemical synthesis, including solid phase or liquid phase synthesis.

### Pharmaceutical Compositions

In another aspect, the present invention provides a composition, e.g., a pharmaceutical composition, containing one or a combination of pertussis toxin-binding protein of the present invention, formulated together with a pharmaceutically acceptable carrier. Such compositions may include one or a combination of (e.g., two or more different) pertussis toxin-binding proteins of the invention. For example, a pharmaceutical composition of the invention can comprise a combination of antibody molecules that bind to different epitopes on the target antigen (pertussis toxin).

As used herein, "pharmaceutically acceptable carrier" includes any and all solvents, dispersion media, coatings, antibacterial and antifungal agents, isotonic and absorption delaying agents, and the like that are physiologically compatible. Preferably, the carrier is suitable for intravenous, intramuscular, subcutaneous, parenteral, spinal or epidermal administration (e.g., by injection or infusion). Depending on the route of administration, the active compound, i.e., antibody, or immunoconjugate, may be coated in a material to protect the compound from the action of acids and other natural conditions that may inactivate the compound.

The pharmaceutical compounds of the invention may include one or more pharmaceutically acceptable salts. A "pharmaceutically acceptable salt" refers to a salt that retains the desired biological activity of the parent compound and does not impart any undesired toxicological effects (see e.g., Berge, S.M., et al. (1977) J. Pharm. Sci. 66:1-19). Examples of such salts include acid addition salts and base addition salts. Acid addition salts include those derived from nontoxic inorganic acids, such as hydrochloric, nitric, phosphoric, sulfuric, hydrobromic, hydroiodic, phosphorous and the like, as well as from nontoxic organic acids such as aliphatic mono- and dicarboxylic acids, phenyl-substituted alkanoic acids, hydroxy alkanoic acids, aromatic acids, aliphatic and aromatic sulfonic acids and the like. Base addition salts include those derived from alkaline earth metals, such as sodium, potassium, magnesium, calcium and the like, as well as from nontoxic organic amines, such as N,N'-dibenzylethylenediamine, N-methylglucamine, chloroprocaine, choline, diethanolamine, ethylenediamine, procaine and the like.

A pharmaceutical composition of the invention also may include a pharmaceutically acceptable anti-oxidant. Examples of pharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

These compositions may also contain adjuvants such as preservatives, wetting agents, emulsifying agents and dispersing agents.

Prevention of presence of microorganisms may be ensured both by sterilization procedures, supra, and by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

Pharmaceutically acceptable carriers include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions of the invention is contemplated. Supplementary active compounds can also be incorporated into the compositions.

Therapeutic compositions typically must be sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants.

Sterile injectable solutions can be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

The amount of active ingredient which can be combined with a carrier material to produce a single dosage form will vary depending upon the subject being treated, and the particular mode of administration. The amount of active ingredient which can be combined with a carrier material to produce a single dosage form will generally be that amount of the composition which produces a therapeutic effect. Generally, out of one hundred percent, this amount will range from about 0.01 percent to about ninety-nine percent of active ingredient, preferably from about 0.1 per cent to about 70 per cent, most preferably from about 1 percent to about 30 percent of active ingredient in combination with a pharmaceutically acceptable carrier.

Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic effect to be achieved, and (b) the limitations inherent in the art of compounding such an active compound for the treatment of sensitivity in individuals.

For administration of the antibody molecule, the dosage ranges from about 0.0001 to 100 mg/kg, and more usually 0.01 to 20 mg/kg, of the subject body weight. For example dosages can be 0.3 mg/kg body weight, 1 mg/kg body weight, 3 mg/kg body weight, 5 mg/kg body weight, 10 mg/kg body weight, 20 mg/kg body weight or 30 mg/kg body weight or within the range of 1-30 mg/kg. An exemplary treatment regime entails administration once per week, once every two weeks, once every three weeks, once every four weeks, once a month, once every 3 months or once every three to 6 months, or with a short administration interval at the beginning (such as once per week to once every three weeks), and then an extended interval later (such as once a month to once every three to 6 months).

Alternatively, antibody can be administered as a sustained release formulation, in which case less frequent administration is required. Dosage and frequency vary depending on the half-life of the antibody in the patient. In general, human antibodies show the longest half-life, followed by humanized antibodies, chimeric antibodies, and nonhuman antibodies. The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated, and preferably until the patient shows partial or complete amelioration of symptoms of disease. Thereafter, the patient can be administered a prophylactic regime.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

A composition of the present invention can be administered via one or more routes of administration using one or more of a variety of methods known in the art. As will be appreciated by the skilled artisan, the route and/or mode of administration will vary depending upon the desired results. Preferred routes of administration for pertussis toxin-binding proteins of the invention include intravenous, intramuscular, intradermal, intraperitoneal, subcutaneous, spinal or other parenteral routes of administration, for example by injection or infusion. The phrase "parenteral administration" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion.

Alternatively, a pertussis toxin-binding protein of the invention can be administered via a non-parenteral route, such as a topical, epidermal or mucosal route of administration, for example, intranasally, orally, vaginally, rectally, sublingually or topically.

### Disease prevention and treatment

The present invention relates to a method for treating Bordetella pertussis infection in a subject, the method comprising administering to the subject the pertussis toxin binding protein of the present invention or pharmaceutical composition of the present invention (in an effective amount, for example).

In another aspect, the present invention relates to a method for preventing Bordetella pertussis infection in a subject, the method comprising administering to the subject the pertussis toxin binding protein of the present invention or pharmaceutical composition of the present invention (in an effective amount, for example). In some embodiments, the subject is at risk of Bordetella pertussis infection (for example, the patient is a pre-vaccinated baby and/or the patient has been exposed to pertussis toxin).

In aother aspect, the present invention relates to a method for neutralizing pertussis toxin in a subject, the method comprising administering to the subject pertussis toxin binding protein of the present invention or pharmaceutical composition of the present invention (in an effective amount, for example). In some embodiments, the subject is infected with Bordetella pertussis.

In another aspect, the present invention relates to a method for treating Bordetella pertussis infection-related diseases or disorders in a subject, the method comprising administering to the subject pertussis toxin binding protein of the present invention or pharmaceutical composition of the present invention (in an effective amount, for example).

Bordetella pertussis infection is characterized by leukocytosis or elevated white blood cell count. In one embodiment, the method of the present invention includes reducing the white blood cell count in a patient. In one embodiment, the method of the present invention produces accelerated reduction of leukocytosis. In another embodiment, the method of the present invention produces a decrease in the maximum white blood cell count during infection.

In one embodiment, the method of the present invention produces neutralization (inhibition or antagonism) of pertussis toxin protein. For example, the pertussis toxin binding protein of the present invention can bind to the pertussis toxin protein to partially or completely inhibit one or more biological activities of pertussis toxin protein. One of the biological activities of the pertussis toxin protein that can be inhibited or blocked by the neutralizing antibody is the ability of pertussis toxin protein to bind to cell receptors. The receptor-binding region of pertussis toxin protein consists of four polypeptide subunits, called subunit S2, subunit S3, subunit S4 and subunit S5, respectively. Examples of cellular receptors bound by subunits S2, S3, S4 and S5 of pertussis toxin protein are members of the N-linked sialoglycoprotein family, such as fetoprotein, haptoblobin and transferrin. In an exemplary embodiment, the pertussis toxin binding protein of the present invention prevents the pertussis toxin protein from binding to its cellular receptors. In another embodiment, the pertussis toxin binding protein of the present invention changes the intracellular transport steps of pertussis toxin so that the toxin will not reach the cell cytoplasm. Another important activity of pertussis toxin protein that can be inhibited by the pertussis toxin binding protein of the present invention is the enzymatic activity of pertussis toxin protein as ADP ribose enzyme against G protein. The subunit of pertussis toxin protein which is endowed with enzyme activity as ADP ribose enzyme is subunit S1. In some embodiments, the pertussis toxin protein is pertussis holothurin. The pertussis holothurin called pertussis toxin protein herein comprises all five pertussis toxin protein subunits. In other embodiments, the pertussis toxin protein is a truncated pertussis toxin protein. The truncated pertussis protein mentioned herein comprises at least one of pertussis toxin protein subunits (i.e., S1, S2, S3, S4 and S5). Various forms of pertussis toxin protein have been described in the prior art.

In various embodiments, the compositions and methods of the present invention are suitable for any stage of treating or preventing pertussis infection. For example, the incubation period of pertussis is usually 7-10 days, ranging from 4-21 days, and rarely as long as 42 days. In various embodiments, the compositions and methods of the present invention increase the length of the incubation period by making infection more difficult to occur. The clinical course of the disease is divided into three stages. The first stage, also known as catarrhal stage, begins with cold-like symptoms, such as insidious attack of rhinitis, sneezing, low fever and mild, occasional cough. Cough gradually worsens, and after 1-2 weeks, the second or paroxysmal stage begins. In various embodiments, the compositions and methods of the present invention reduce the length of the catarrhal phase and optionally prevent it from progressing to the paroxysmal phase. In various embodiments, the compositions and methods of the present invention are used to treat one or more of rhinitis, sneezing, low fever and cough. This is during the paroxysmal stage of pertussis which is usually suspected to be diagnosed. Typically, patients manifest many rapid paroxysmal cough, apparently due to the difficulty of expelling thick mucus from the tracheobronchial tree. At the end of paroxysmal cough, long inspiratory efforts are usually accompanied by a specific high-pitched "whoop" sound. During this coughing fit, the patient may become cyanotic. Especially children and infants feel very uncomfortable and painful. After coughing fits, vomiting and fatigue may usually occur. In various embodiments, the compositions and methods of the present invention reduce the amount and/or frequency of paroxysm. In various embodiments, the compositions and methods of the present invention prevent patients from becoming cyanotic. Paroxysmal coughing fits may occur more frequently at night, with an average of 15 times every 24 hours. In the first 1 or 2 weeks of this stage, the seizure frequency increases, stays at the same level for 2 to 3 weeks, and then gradually decreases. Paroxysmal stage usually lasts 1 to 6 weeks, but it may also last as long as 10 weeks. In various embodiments, the compositions and methods of the present invention reduce the length of this stage. During the recovery period, recovery is gradual. Paroxysmal cough may decrease and disappear in 2 to 3 weeks. In various embodiments, the compositions and methods of the present invention accelerate the onset of this stage and/or reduce its duration. Furthermore, in various embodiments, the compositions and methods of the present invention prevent or reduce paroxysmal recurrence, which may occur with subsequent respiratory infections. In various embodiments, the compositions and methods of the present invention prevent or reduce one or more of the following paroxysms: secondary bacterial pneumonia, neurological complications such as epilepsy and encephalopathy, hypoxia, otitis media, dehydration, pneumothorax, epistaxis, subdural hematoma, hernia, rectal prolapse, difficulty sleeping, urinary incontinence, pneumonia and rib fracture. Furthermore, in some embodiments, the compositions and methods of the present invention reduce or prevent necrotizing bronchiolitis, pneumonia (for example, derived from Bordetella pertussis), pulmonary edema, pulmonary hypertension and death.

In another embodiment, the method comprises administering to a subject a combination of the pertussis toxin binding protein of the present invention or the pharmaceutical composition of the present invention and an antimicrobial agent. Co-administration of the combination of the pertussis toxin binding protein of the present invention or the pharmaceutical composition of the present invention and the antimicrobial agent is expected to produce synergistic effect. Illustrative antimicrobial agents suitable for the present invention include, but are not limited to, azithromycin, clarithromycin, erythromycin, trimethoprim-sulfamethoxazole, roxithromycin, ketolide (e.g., telithromycin), ampicillin, amoxicillin, tetracycline, chloramphenicol, fluoroquinolones (e.g., ciprofloxacin, levofloxacin, ofloxacin, moxifloxacin), and cephalosporin.

In various embodiments, the subject treated by the method of the present invention is a human. In one embodiment, the subject is an infant. In one embodiment, the subject is a newborn. In another embodiment, the subject is a newborn who is less than four weeks, less than three weeks, less than two weeks, less than one week, less than six days, less than five days, less than four days, less than three days, less than two days or less than one day. In some embodiments, the human is one month old, two months old, three months old, four months old, five months old or six months old. In some embodiments, the human is at the age of about 6 to about 18 months, about 18 to about 36 months, about 1 to about 5 years, about 5 to about 10 years, about 10 to about 15 years, about 15 to about 20 years, about 20 to about 25 years, about 25 to about 30 years, about 30 to about 35 years, about 35 to about 40 years, about 40 to about 45 years, about 45 to about 50 years, about 50 to about 55 years, about 55 to about 60 years, about 60 to about 65 years, about 65 to about 70 years, about 70 to about 75 years, about 75 to about 80 years, about 80 to about 85 years, about 85 to about 90 years, about 90 to about 95 years, or about 95 to about 100 years.

On the other hand, the method of the present invention prevents Bordetella pertussis infection in a subject previously exposed to Bordetella pertussis bacteria, which comprises administering to the subject the pertussis toxin binding protein of the present invention or the pharmaceutical composition of the present invention. In various embodiments, the method provides effective preventive treatment in preventing Bordetella pertussis infection in a subject exposed to Bordetella pertussis bacteria.

In some embodiments, the pertussis toxin binding protein of the present invention or the pharmaceutical composition of the present invention is used for prophylactic application in a subject that has not been previously vaccinated against the bacteria.

It is expected that the pertussis toxin binding protein of the present invention or the pharmaceutical composition of the present invention can also serve as an adjuvant for vaccination, such as DtaP or Tdap. Furthermore, in various embodiments, the methods of the present invention treat or prevent Bordetella pertussis infection in a subject who has not been previously vaccinated against Bordetella pertussis bacteria.

### Detection

On the other hand, the invention also provides a method for detecting the presence and/or amount of pertussis toxin in a biological sample, the method comprising contacting the biological sample and the control sample with the pertussis toxin binding protein of the invention under conditions allowing the formation of complex between the pertussis toxin binding protein and pertussis toxin. Then detection of the complex is performed, wherein the difference in the complex formation between the biological sample and the control sample indicates the presence and/or amount of pertussis toxin in the sample.

In some embodiments, the pertussis toxin binding protein of the present invention is also conjugated with fluorescent dyes, chemicals, polypeptides, enzymes, isotopes, labels and the like that can be used for detection or can be detected by other reagents.

In some embodiments, the biological sample is a vaccine for preventing Bordetella pertussis infection, for example, the vaccine comprises pertussis toxin.

### Kits

The scope of the present invention also includes a kit for use in the method of the present invention, which includes the pertussis toxin binding protein of the present invention and instructions. The kit may further comprise at least one reagent for detection. The kit generally includes labels indicating the intended use of the contents of the kit. The term label includes any written or recorded material provided on the kit or coming with the kit or otherwise provided with the kit.

### Examples

The following examples are used to further illustrate the present invention, but the scope of the present invention is not limited to these examples.

### Example 1: Screening of heavy chain single domain antibody against Pertussis Toxin (PT)

### 1.1 Construction of library:

Before immunization, 5 mL of arterial blood from bactrian camel was collected into a vacuum blood collection tube, and the supernatant was collected as the pre-immunization serum. For the first immunization, a healthy 2-year-old Xinjiang bactrian camel was selected, 300 µg recombinant pertussis toxin protein was taken as antigen and mixed with complete Freund's adjuvant in equal volume for emulsifying the proteins completely, which was then injected into the bactrian camel's neck muscles at multiple points; for the later stage of immunization, the same amount of antigen and incomplete Freund's adjuvant were uniformly mixed in equal volume each time; immunization was performed once a week, with a total of six times of immunization on bactrian camels in the later period. At the end of the last immunization, 5 mL of bactrian camel arterial blood was collected into the vacuum blood collection tube, and the supernatant was collected as the post-immunization serum, the variation of antibody in animal serum before and after immunization were detected by ELISA, wherein, the antibody in serum increased obviously after immunization, so it was determined that the immune effect caused by antigen met the

### experimental requirements.

Lymphocytes were separated by density gradient centrifugation, total RNA was extracted by a RNA extraction kit provided by QIAGEN Company, all extracted RNA was reverse transcribed into cDNA by SuperScript III FIRST STRANDSUPERMIX kit according to the instructions, and nucleic acid fragments encoding the variable region of the heavy chain antibody were amplified by nested PCR.

The nucleic acid fragments of the target heavy chain single domain antibody was recovered and cloned into phage display vector pMECS by using restriction enzymes (purchased from NEB) PstI and NotI. Subsequently, the product was electrotransformed into the electroporation-competent cells TG1 of Escherichia coli, and the phage display library of anti-PT toxin immune single domain antibody was constructed and verified. Through planking with gradient dilution, the capacity of the library was calculated as 1.2×10⁸. In order to detect the insertion rate of the library, 50 clones were randomly selected for sequencing, and 50 clones with correct foreign fragment insertion were found, showing an accuracy of 100%. Through the analysis and alignment of DNAs and amino acid sequences of sequenced clones, respectively, it was confirmed that all sequences were expected camel VHH sequences, and the diversity was estimated to be more than 95%.

### 1.2 Panning of antibodies against pertussis toxin (PT)

Recombinant pertussis toxin protein PT was coated on a plate at 10 µg/ well, and left overnight at 4°C. The next day, after being blocked with 2% skim milk at room temperature for 2 hours, 100 µL of phage (about 10 ¹⁰ PFU) from the phage library constructed in Example 1.1 was added and functioned at room temperature for 2 hours. Afterwards, the resultant was washed 25 times with PBST (0.05% Tween 20 in PBS) to wash off unbound phages. At last, the phages specifically binding to PT protein were dissociated with Glycine (100 mM, pH 2.0) and infected with E.coli TG1 growing in logarithmic phase, the bacteria solution was collected by centrifugation and coated on a 2TYAG plate, and cultured at 37 °C overnight. The next day, E. coli on the plate was eluted and collected to obtain about 50 mL of bacteria solution. By calculating according to the OD value, about 10¹¹ Escherichia coli bacteria were obtained.

### 1.3 Screening sequence of anti-PT heavy chain single domain antibody by high-throughput sequencing:

2 mL of E.coli obtained in Example 1.2 was commissioned to Suzhou Hongxun Technology Co., Ltd. for high-throughput sequencing.

A total of 286,521 sequences of anti-PT single domain antibody were obtained in this high-throughput sequencing, among which the top 50 sequences with higher abundance were selected, and then 32 sequences were further selected as candidate sequences according to the individual abundance of CDR1, CDR2 and CDR3 regions in the anti-PT single domain antibody as well as the abundance of pairwise combinations.

### Example 2: Preparation of FC fusion protein of PT single domain antibody with mammalian cells

### 2.1 Preparation of Fc fusion plasmid of PT single domain antibody

32 candidate sequences obtained in Example 1.3 were commissioned to Suzhou Hongxun Biotechnology Co., Ltd. for gene synthesis, with enzyme digestion sites added at both ends thereof.

The VHH fragment of PT single domain antibody was performed with double enzyme digestion, and fused with the DNA fragment encoding human IgG1FC, and cloned into the conventional mammalian expression vector to obtain the recombinant plasmid for expressing the Fc fusion protein of PT single domain antibody in mammals.

### 2.2 Preparation of Fc fusion protein of PT single domain antibody

The vector constructed in 2.1 was transfected into HEK293 cells for transient expression of the antibody. The recombinant expression plasmid was diluted with Freestyle293 medium and added with PEI (Polyethylenimine) solution needed for transformation, and each group of plasmid /PEI mixture was added into HEK293 cell suspension and placed at 37°C, 5% CO₂ for culture at 130 rpm. Four hours later, EXCELL293 medium, 2 mM glutamine were added for culture at 130 rpm. After 24 hours, 3.8 mM VPA was added, and with 4 g/L glucose after 72 hours. After culture for 5-6 days, the supernatant of the transient expression culture was collected, and the Fc fusion protein of target PT single domain antibody was obtained by purification using Protein A affinity chromatography. The purity of protein was preliminarily investigated by SDSPAGE and SECHPLC. The expression and purity analysis of a fraction of proteins were shown in Table 1 below:

**Table 1**

| Antibody | Expression amount (mg/L) | SDSPAGE purity% | Main peak ratio% |
|---|---|---|---|
| iPT3Fc | 380 | >95% | 99.3 |
| iPT7Fc | 511 | >95% | 98.5 |
| iPT12Fc | 553 | >95% | 98.8 |
| iPT13Fc | 568 | >95% | 97.7 |
| iPT15Fc | 426 | >95% | 99.3 |
| iPT20Fc | 473 | >95% | 98.9 |
| iPT21Fc | 445 | >95% | 98.3 |
| iPT22Fc | 529 | >95% | 98.9 |
| iPT26Fc | 439 | >95% | 98.9 |
| iPT28Fc | 226 | >95% | 99 |
| iPT35Fc | 355 | >95% | 99.6 |
| iPT36Fc | 417 | >95% | 98.4 |
| iPT42Fc | 334 | >95% | 97.3 |

As can be seen, the expression levels of Fc fusion proteins of PT monoclonal antibody were all above 200 mg/L, and after one-step purification by Protein A affinity chromatography column, the concentrations of FC fusion protein were mostly above 1.0 mg/mL, and the purity was also very high.

Sequence information of the variable region of the selected PT single domain antibody was as follows (the boxes from left to right of each sequence showed CDR1, CDR2 and CDR3 respectively):
>SEQ ID NO:40 iPT3 
QVQLQESGGGSVQAGESLRLSCVVSGYTVSSICMGWFRQAPGKEREGVAAVASDGVTSYTDSVK
GRFTISKDNAKNTLYLQMNSLTPEDTSMYYCAARKRGYCSLDRTFFTWWGQGTQVTVSS
>SEQ ID NO:41 iPT7 
QVQLQESGGGSVQPGGSLRLSCAASGYTYSSYCMGWFRQAPGKEREGVAAIDSDGRTSYADFVK
GRFTISKDNAKNTLYLQMNSLKPEDTAKYYCATEQRIGGSWYDLCGGADFGYWGQGTQVTVSS
>SEQ ID NO:42 iPT12 
QVQLQESGGGSVQAGGSLRLSCAASGYTYSSYCMGWFRQAPGKEREGVAGIESDGSTSYADSVK
GRFSISKDNVKNTLYLQMNTLKPEDTGIYYCAVQSPLPGGSWYSLCGGADFGYWGQGTQVTVSS
>SEQ ID NO:43 iPT13 
QVQLQESGGGSVQAGGSLRLTCAASGYRSCMGWFRQAPGKEREGVAAIWPDGITYYADSVKGRF
TISRDKAVNTMYLQMNSLKPEDTAMYYCATADLICPTDWAQRLQYNYWGQGTQVTVSS
>SEQ ID NO:44 iPT15 
QVQLQESGGGSVQAGGSLRLSCAASGYTYGSYCMAWFRQAPGKEREGVAGVDSDGTTRYADSV
KGRFTISKDNAENTLYLQMNSLKPEDTSMYYCAAGVCGTGGSWYEGDAGLFMYEFAYWGQGTQ
VTVSS
>SEQ ID NO:45 iPT20 
QVQLQESGGGSVQAGGSLRLSCAASEYRYCMAWFRQAPGKEREGVAGIYIGDRTVYSDSVKGRF
TISQDNADNTVYLQMNSLKPEDTAMYYCATVDLICPSDWAQRLQYQYWGQGTQVTVSS
>SEQ ID NO:46 iPT21 
QVQLQESGGGSVQAGGSLRLSCAASGYPYSRNCMAWFRQAPEKQREGVATIYNGDGTPYYADSV
KGRFTISQDNAKNTVYLQMNSLKPDDTAIYYCAAARGCSLRTWSDQNLYAYWGQGTQVTVSS
>SEQ ID NO:47 iPT22 
QVQLQESGGGSVQAGGSLQLSCAATDITYSRCMAWFRQAPGKSREGVAALSTNGGSTWYADSVK
GRFTISRDGAKNTIYLQMRSLKPEDTAMYYCADSSFFSCRDCSGGDCPRSRDYKYWGQGTQVTV
SS
>SEQ ID NO:48 iPT26 
QVQLQESGGGSVQAGGSLRLSCEASGFTTSSRCMAWFRQAPGKEREGVGLIVTGTGATRYADSM
KGRFTISQDNAKNTMWLQINSLKPEDTAMYFCAAIDITWRGCSFEKRDVNYWGQGTQVTVSS
>SEQ ID NO:49 iPT28 
QVQLQESGGGSVQAGGSLRLSCAASGYTYTSYCMGWFRQAPGKEREGVAGIDRDGIAVYADSVK
GRFTVSKDNVEDTLSLQMNSLKPEDTAMYYCAADYRRWCRDEYEYWGQGTQVTVSS
>SEQ ID NO:50 iPT35 
QVQLQESGGGSVQAGGSLRLSCAASTYTTTSRCMAWFRQAPGKEREGVGLIVTGTGATRYADSM
KGRFTISQDTAKNTMWLQMNSLKPEDTGMYFCAAIDITWRGCSFETRDVNYWGQGTQVTVSS
>SEQ ID NO:51 iPT36 
QVQLQESGGGSVQVGGSLRLTCAASEYRSCMGWFRQAPGKEREGVAAIWPDGITYYADSVKGRF
TISRDKDVNTMYLAMNSLKPEDTAMYYCATAELICPTDWAQRQQYNYWGQGTQVTVSS
>SEQ ID NO:52 iPT42

QVQLQESGGGSVQAGGSLRLSCAASGYTYSDVCMGWFRQAPGKEREGVAAIDSVGNTNYADSV
KGRFTISKDNAKNTLYLQMNSLKPEDTAMYYCAAQRCLGWVPLVPHEYGFWGQGTQVTVSS

### Example 3: Verification of functions of FC fusion protein of PT single domain antibody

### 3.1 Detection of the affinity of Fc fusion protein of PT single domain antibody (Bio-Layer Interferometry, BLI)

The binding kinetics of the Fc fusion protein of PT single domain antibody obtained in the above example against the recombinant human PT protein was measured by Biolayerinterferometry (BLI) using a molecular interaction instrument. The Fc fusion protein of PT single domain antibody obtained in Example 2.2 was diluted to a final concentration of 2.5 µg/mL and directly immobilized on an AHC biosensor, for kinetic measurement, PT was diluted with 0.02% PBST20 to five concentrations of 100 nM, 50 nM, 25 nM, 12.5 nM and 6.25 nM, respectively, with the sample injection time of 120s, the dissociation time of 600s and 10 mM glycine-HCl (pH1.7) regeneration for 5s. The binding rate (kon) and the dissociation rate (kdis) were calculated Using simple one-to-one Languir binding model (Octet K2 data analysis software version 9.0 (Data analysis 9.0)). The equilibrium dissociation constant (kD) was calculated as the ratio of kdis/kon.

The affinity results of a fraction of Fc fusion proteins of PT single domain antibody against PT measured were shown in Table 2. Some candidate antibodies were excluded due to poor affinity (data not shown).

**Table 2**

| Antibody | KD (M) | kon (1/Ms) | kdis (1/s) |
|---|---|---|---|
| iPT3Fc | 1.32E09 | 3.88E+05 | 6.02E04 |
| iPT7Fc | 1.46E10 | 5.55E+05 | 1.03E04 |
| iPT12Fc | 8.91E11 | 6.18E+05 | 5.51E05 |
| iPT13Fc | 1.33E09 | 7.04E+05 | 6.19E04 |
| iPT15Fc | 1.38E10 | 3.93E+05 | 5.40E05 |
| iPT20Fc | 2.21E10 | 4.67E+05 | 9.46E05 |
| iPT21Fc | 5.37E10 | 5.17E+05 | 2.78E04 |
| iPT22Fc | 7.39E10 | 5.13E+05 | 3.79E04 |
| iPT26Fc | 3.40E10 | 4.27E+05 | 1.29E04 |
| iPT28Fc | 2.78E10 | 3.99E+05 | 1.11E04 |
| iPT35Fc | 2.74E10 | 3.78E+05 | 1.04E04 |
| iPT36Fc | 8.07E10 | 4.42E+05 | 3.57E04 |
| iPT42Fc | 3.75E10 | 3.86E+05 | 1.45E04 |

### 3.2 Identification of the neutralizing activity of FC fusion protein of PT monoclonal antibody against pertussis toxin

PT toxin at a concentration of 434.3 µg/mL was formulated to 12 ng/mL with 10% FBS+ F12K+10% FBS, and added with 50 µL to per well, according to the results of Example 3.1, the FC fusion proteins of PT monoclonal antibody with good affinity were selected to be 2x diluted from the original concentration with 10%FBS+ F12K, to obtain 10 concentrations, 50 µL of which was added to each well, after stay at 37°C for 1 hour, CHOK1 cells were collected and counted, and the number of cells was adjusted to 3×10⁵ cells/mL, 50 µL of solution was added to each well of the 96-well plate, after 24 hours, 100% alcohol fixing and 0.1% crystal violet staining were carried out successively, after washing with PBS, observation and photos were taken under microscope.

The results were shown in Table 3 below: iPT13Fc, iPT21Fc, iPT28Fc and iPT36Fc showed partial neutralizing activity at the concentration of 100 µg/mL; iPT3Fc, iPT7Fc, iPT12Fc, iPT15Fc, iPT20Fc, iPT22Fc, iPT26Fc, iPT35Fc and iPT42Fc showed relatively high neutralizing activity. Some candidate antibodies were excluded due to poor neutralizing activity (data not shown).

**Table 3**

| Sample | Completely neutralizing concentration (µg/mL) |
|---|---|
| iPT3Fc | 50 |
| iPT7Fc | 1.56 |
| iPT12Fc | 1.125 |
| iPT13Fc | 100 µg/mL partial neutralizing activity |
| iPT15Fc | 1.56 |
| iPT20Fc | 50 |
| iPT21Fc | 100 µg/mL partial neutralizing activity |
| iPT22Fc | 3.13 |
| iPT26Fc | 1.56 |
| iPT28Fc | 100 |
| iPT35Fc | 2.25 |
| iPT36Fc | 100 |
| iPT42Fc | 12.5 |

### 3.3 Detection of different PT-binding epitopes of Fc fusion protein of PT single domain antibody (Bio-Layer Interferometry (BLI): epitope binning)

By using the intandem method, PT-biotin was diluted with 0.02% PBST20 to 10 ug/mL and immobilized on SAbiosensor for 120s. The Fc fusion protein of PT single domain antibody was diluted to 100 nM with 0.02% PBST20, and divided into two groups with the antibody-binding time of 300s for each group and the regeneration solution of 10 mM glycine-HCl (pH 1.7), wherein the first antibody (saturated antibody) binded to the sensor until saturated, and then the second antibody (competitive antibody) competed with the first antibody at the same concentration, and the percentage was calculated. The percentage was calculated following the formula: Ab2 with Ab1/Ab2 without Ab1.

The results of these measurements were shown in Table 4 and Table 5. The results showed that there may be competition among iPT13, iPT20 and iPT36, and there may be competition between iPT7 and iPT12, between iPT26 and iPT35, and between iPT22 and iPT15; other antibodies have no obvious competitive relationship and have different antigen-binding epitopes.

**Table 4**

| Ab1\Ab2 | iPT5FC | iPT3FC | iPT7FC | iPT13FC | iPT20FC | iPT26FC | iPT36FC |
|---|---|---|---|---|---|---|---|
| iPT5FC | 8.10% | 85.50% | 66.39% | 48.14% | 105.13% | 19.11% | 104.26% |
| iPT3FC | 104.30% | 29.80% | 42.11% | 43.99% | 96.72% | 18.41% | 93.65% |
| iPT7FC | 71.50% | 63.40% | 11.15% | 55.60% | 60.00% | 70.80% | 58.70% |
| iPT13FC | 79.00% | 79.75% | 74.50% | 22.90% | 0.10% | 76.50% | 0.116 |
| iPT20FC | 87.10% | 92.90% | 93.00% | 49.30% | 6.72% | 95.30% | 0.0359 |
| iPT26FC | 49.60% | 54.20% | 45.60% | 26.70% | 13.80% | 31.40% | 28.00% |
| iPT36FC | 91.60% | 91.15% | 92.40% | 51.80% | 20.30% | 92.10% | 8.12% |

**Table 5**

| Ab1\Ab2 | iPT7 | iPT26 | iPT12 | iPT15 | iPT22 | iPT35 | iPT42 |
|---|---|---|---|---|---|---|---|
| iPT7FC | 11.97% | 79.48% | 17.43% | 79.76% | 41.72% | 26.26% | 79.59% |
| iPT26FC | 117.29% | 6.85% | 110.26% | 102.65% | 51.00% | 12.80% | 93.59% |
| iPT12FC | 16.29% | 16.08% | 11.88% | 86.81% | 53.34% | 97.95% | 79.00% |
| iPT15FC | 115.91% | 16.60% | 108.40% | 15.40% | 6.73% | 88.28% | 79.85% |
| iPT22FC | 82.67% | 13.39% | 78.47% | 32.60% | 7.10% | 71.23% | 84.19% |
| iPT35FC | 118.80% | 3.67% | 119.64% | 71.08% | 30.57% | 9.72% | 98.61% |
| iPT42FC | 110.00% | 10.72% | 105.09% | 77.60% | 45.61% | 85.17% | 10.48% |

### 3.4 Detection of the nonspecific binding of Fc fusion protein of PT single domain antibody to empty cells

CHOK1 empty cells and 293F empty cells were resuspended in 3% BSAPBS and adjusted to 6× 10⁶ cells/mL, and added with the Fc fusion protein of PT monoclonal antibody obtained in Example 2.2 with a final concentration of 100 µg/mL, meanwhile the negative control and blank control were set, each being cooled in an ice bath for 30 min. After washing, Boster secondary antibody FITC rabbit anti-human IgG antibodies were added, with being cooled in an ice bath for 30min. After washing, the cells were resuspended in 300 µL of 1% PBSBSA Buffer, and detected by flow cytometry. The results were shown in Table 6 that there was no significant difference in the MFI between the antibody and negative control and blank control, and no non-specific binding occurred.

**Table 6**

| | Concentration (µg/mL) | | | MFI (CHO) | | MFI (293) |
|---|---|---|---|---|---|---|
| Blank | —— | | | 2.62 | | 2.08 |
| Negative | —— | | | 3.09 | | 2.38 |
| Secondary antibody | —— | | | 3.01 | | 2.67 |
| iPT3Fc | 100 ug/mL | | | 2.77 | | 2.8 |
| iPT7Fc | 100 ug/mL | | | 2.68 | | 2.6 |
| iPT13Fc | 100 ug/mL | | | 2.81 | | 2.6 |
| iPT21Fc | 100 ug/mL | | | 2.76 | | 2.64 |
| iPT20Fc | 100 ug/mL | | | 2.89 | | 2.77 |
| iPT26Fc | 100 ug/mL | | | 2.89 | | 2.77 |
| iPT36Fc | 100 ug/mL | | | 2.68 | | 2.67 |
| | | | | | | |

| | | Concentration (µg/mL) | MFI (CHO) | | MFI (293) | |
|---|---|---|---|---|---|---|
| Blank | | —— | 2.50 | | 2.31 | |
| Negative | | —— | 2.87 | | 2.40 | |
| Secondary antibody | | —— | 3.42 | | 5.41 | |
| iPT12Fc | | 100 ug/mL | 3.54 | | 5.69 | |
| iPT15Fc | | 100 ug/mL | 3.6 | | 5.78 | |
| iPT22Fc | | 100 ug/mL | 3.61 | | 5.59 | |
| iPT26Fc | | 100 ug/mL | 3.79 | | 5.84 | |
| iPT35Fc | | 100 ug/mL | 3.76 | | 5.95 | |
| iPT42Fc | | 100 ug/mL | 3.66 | | 5.56 | |

### 3.6 Detection of the thermal stability of Fc fusion protein of PT single domain antibodies

The trace volume of protein samples was determined by using the protein thermostability detector from UNCHAINED LABS. Heating program: initial temperature: 15°C, heating rate: 0.3 °C /min, final temperature: 95°C. The fluorescence absorbance values of samples at each temperature and wavelength were recorded, and the denaturation temperature Tm was fitted using a software as the highest point of the next derivative of confidence wavelength BCM; the initial polymerization temperature Tagg was one tenth of the next derivative of static light scattering SLS at 473 nm. The higher the Tm value, the more stable the protein was. The results were shown in Table 7 below, as can be seen, each antibody had a Tm basically above 60°C with good stability.

**Table 7**

| | 10 mg/mL | | | 2 mg/mL | | |
|---|---|---|---|---|---|---|
| | Tm1 (°C) | Tm2 (°C) | Tagg (°C) | Tm1 (°C) | Tm2 (°C) | Tagg (°C) |
| iPT3Fc | 66.3 | 78.9 | 68 | 66.9 | 81.7 | 74.5 |
| iPT7Fc | 64.6 | 81.2 | 66 | 66.9 | 81.6 | 74.5 |
| iPT12Fc | 64 | 78 | 53 | 63 | 87 | 74 |
| iPT13Fc | 80.9 | 80.9 | 66 | 67 | 82.2 | 70.2 |
| iPT15Fc | 60 | 79 | 61 | 64 | 80 | 74 |
| iPT20Fc | 78.6 | —— | 66.2 | 84.2 | 84.2 | 78 |
| iPT21Fc | 64 | —— | —— | 65 | —— | 64 |
| iPT22Fc | 65 | 80 | 61 | 65 | —— | 66 |
| iPT26Fc | 75.5 | —— | 60.1 | 67.2 | —— | 72.1 |
| iPT36Fc | 67.2 | 81.9 | 69 | 68.4 | 83 | 76 |
| iPT28Fc | 49 | —— | —— | 50 | —— | 77 |
| iPT35Fc | 58 | —— | 58 | 58 | —— | 70 |
| iPT42Fc | 67 | 85 | 70 | 68 | 83 | 70 |

### Example 4: preparation of FC fusion protein of PT tetravalent antibody with mammalian cells

### 4.1 Preparation of Fc fusion plasmid of PT tetravalent antibody

Seven antibodies iPT7, iPT12, iPT15, iPT22, iPT26, iPT35 and iPT42 were selected to be divided into four groups according to the overlapping of antigen-recognizing epitopes of antibodies (epitope binning results), sequences with small overlapping of antigen-recognizing epitopes were combined in tandem in pairs, and fused with the DNA fragment encoding human IgG1 FC, and cloned into the conventional mammalian expression vector to obtain the recombinant plasmid for expressing the Fc fusion protein of PT single domain antibody in mammals, which was expected to increase its activity by this combination; in addition, iPT12diFc and iPT15diFc were formed by combination in tandem of two identical iPT12 or iPT15 sequences, respectively, and fusion with human IgG1 Fc fragment, respectively, which were used as controls.

### 4.2 Preparation of Fc fusion protein of PT tetravalent antibody

The vector constructed in 4.1 was transfected into HEK293 cells for transient expression of the antibody. The recombinant expression plasmid was diluted with Freestyle293 medium and added with PEI (Polyethylenimine) solution needed for transformation, and each group of plasmid /PEI mixture was added into HEK293 cell suspension and placed at 37°C, 5% CO₂ for culture at 130 rpm. Four hours later, EXCELL293 medium, 2 mM glutamine were added for culture at 130 rpm. After 24 hours, 3.8 mM VPA was added, and with 4 g/L glucose after 72 hours. After culture for 5-6 days, the supernatant of transient expression culture was collected, and the Fc fusion protein of the target PT tetravalent antibody was obtained by purification using Protein A affinity chromatography. The purity of protein was preliminarily investigated by SDSPAGE and SECHPLC. The expression and purity analysis of each protein were shown in Table 8 below, as can be seen, the expression levels of Fc fusion proteins of PT tetravalent antibody were all above 200 mg/L, and after one-step purification by Protein A affinity chromatography column, most of them had a concentration greater than 1.0 mg/mL, and the purity was also very high as shown by the SEC results.

**Table 8**

| Antibody | Expression amount (mg/L) | SDS PAGE purity% | Main peak ratio% |
|---|---|---|---|
| iPT7n15Fc | 281 | >95% | 98.9% |
| iPT15n7Fc | 271 | >95% | 97.9% |
| iPT7n22Fc | 374 | >95% | 98.0% |
| iPT22n7Fc | 320 | >95% | 97.1% |
| iPT7n42Fc | 308 | >95% | 97.6% |
| iPT42n7Fc | 310 | >95% | 98.7% |
| iPT12n35Fc | 316 | >95% | 98.0% |
| iPT35n12Fc | 306 | >95% | 99.0% |
| iPT15n35Fc | 328 | >95% | 99.3% |
| iPT15n42Fc | 320 | >95% | 93.0% |
| iPT35n42Fc | 290 | >95% | 98.4% |
| iPT12n15Fc | 367 | >95% | 97.9% |
| iPT15n12Fc | 359 | >95% | 98.8% |
| iPT12n42Fc | 399 | >95% | 95.2% |
| iPT42n12Fc | 254 | >95% | 97.4% |
| iPT15diFc | 344 | >95% | 99.1% |
| iPT12diFc | 257 | >95% | 95.8% |

### Example 5: Verification of functions of FC fusion protein of PT tetravalent antibody

### 5.1 Detection of the affinity of Fc fusion protein of PT tetravalent antibody (Bio-Layer Interferometry, BLI)

The Fc fusion protein of PT tetravalent antibody prepared in Example 4.2 was diluted to 2.5 µg/mL and immobilized on a biosensor for 60 seconds, with a curing height of about 0.8 nm. PTpuri was diluted to five gradients as 100 nM, 50 nM, 25 nM, 12.5 nM and 6.25 nm, baseline: 60s, binding: 120s, dissociation: 600s. The diluent was 0.02 PBST20%, the regeneration solution was glycine-HCl (pH1.7), the neutralizing solution was the diluent and the biosensor was AHC, as seen from Table 9, the antibodies all showed high affinity, especially the antibody iPT15n7Fc showed the highest affinity.

**Table 9**

| Sample | KD (M) | kon (1/Ms) | kdis (1/s) |
|---|---|---|---|
| iPT7n15Fc | 5.24E10 | 1.05E+05 | 5.47E05 |
| iPT15n7Fc | <1.0E12 | 1.07E+05 | < 1.0E07 |
| iPT7n22Fc | 2.23E10 | 7.01E+05 | 1.56E04 |
| iPT22n7Fc | 2.40E10 | 5.73E+05 | 1.38E04 |
| iPT7n42Fc | 3.01E10 | 6.07E+05 | 1.82E04 |
| iPT42n7Fc | 2.67E10 | 6.41E+05 | 1.71E04 |
| iPT12n35Fc | 1.57E10 | 6.02E+05 | 9.43E05 |
| iPT35n12Fc | 1.97E10 | 5.42E+05 | 1.07E04 |
| iPT15n42Fc | 1.67E10 | 7.22E+05 | 1.21E04 |
| iPT35n42Fc | 1.24E10 | 5.46E+05 | 6.75E05 |
| iPT15n35Fc | 9.12E11 | 5.85E+05 | 5.33E05 |
| iPT12n15Fc | 2.63E10 | 5.67E+05 | 1.49E04 |
| iPT15n12Fc | 1.83E10 | 6.14E+05 | 1.12E04 |
| iPT12n42Fc | 2.42E10 | 5.35E+05 | 1.29E04 |
| iPT42n12Fc | 2.37E10 | 5.64E+05 | 1.34E04 |
| iPT12diFc | 2.67E10 | 3.34E+05 | 8.89E05 |
| iPT15diFc | — | — | — |

### 5.2 Identification of the neutralizing activity of FC fusion protein of PT tetravalent antibody against pertussis toxin

PT toxin at a concentration of 434.3 µg/ml was formulated to 12 ng/mL with 10% FBS+ F12K+10% FBS, and added with 50 µL to per well, the FC fusion protein of PT tetravalent antibody with good affinity prepared in Example 4.2 was 2x diluted from the original concentraiton with 10% FBS+ F12K, to obtain 10 concentrations, 50 µL was added to each well, after stay at 37°C for 1 hour, CHOK1 cells were collected and counted, and the number of cells was adjusted to 3x10⁵ cells/mL, then 50 µL was added to each well of the 96-well plate, after 24 hours, 100% alcohol fixing and 0.1% crystal violet staining were carried out successively, after washing with PBS, observation and photos were taken under microscope. As seen from the results shown in Table 10, tetravalent antibodies iPT7n15Fc, iPT42n7Fc, iPT12n15Fc, iPT12n42Fc, iPT12diFc, iPT15n12Fc and iPT42n12Fc all showed good neutralizing activity, and the neutralizing activity of iPT7n15Fc, iPT42n7Fc, iPT12n15Fc, iPT12n42Fc, iPT15n12Fc and iPT42n12Fc was higher than that of iPT15diFc and iPT12diFc, and still higher than that of bivalent antibody iPT12Fc, as can be seen, the neutralizing activity was improved after the combination of single-domain antibodies that recognize different epitopes.

**Table 10**

| Antibody | Concentration for completely neutralizing PTToxin (µg/mL) |
|---|---|
| iPT15n7FC | 5 µg/mL partial neutralizing |
| iPT7n22FC | 5 |
| iPT22n7FC | 100 |
| iPT7n42FC | 2.5 µg/mL partial neutralizing |
| iPT12n35Fc | 100 |
| iPT35n12Fc | 100 |
| iPT15n35Fc | 20 µg/mL partial neutralizing |
| iPT15n42Fc | 100 |
| iPT35n42Fc | 100 µg/mL partial neutralizing |
| iPT12Fc | 0.42 |
| iPT7n15Fc | 0.1 |
| iPT42n7Fc | 0.05 |
| iPT12n15Fc | 0.111 |
| iPT12n42Fc | 0.333 |
| iPT 12diF c | 0.333 µg/mL partial neutralizing |
| iPT15n12Fc | 0.111 |
| iPT15diFc | 1 µg/mL partial neutralizing |
| iPT42n12Fc | 0.333 |

### 5.3 Detection of the nonspecific binding of Fc fusion protein of PT tetravalent antibody to empty cells

CHOK1 empty cells and 293F empty cells were resuspended in 3% BSAPBS and adjusted to 6× 10⁶ cells/mL, and added with the Fc fusion protein of PT monoclonal antibody obtained in Example 4.2 with a final concentration of 100 µg/mL, meanwhile the negative control and blank control were set, each being cooled in an ice bath for 30 min. After washing, Boster secondary antibody FITC rabbit anti-human IgG antibody was added, with being cooled in an ice bath for 30min. After washing, the cells were resuspended in 300 µL of 1% PBSBSA Buffer, and detected by flow cytometry. The results were shown in Table 11, which showed that no nonspecific binding had been found.

**Table 11**

| Sample name | Concentration (µg/mL) | MFI (293) | MFI(CHO) |
|---|---|---|---|
| Blank | —— | 2.62 | 2.08 |
| Negative | —— | 3.09 | 2.38 |
| iPT12n15Fc | 100 | 3.40 | 2.40 |
| iPT12n42Fc | 100 | 3.46 | 2.39 |
| iPT12diFc | 100 | 3.57 | 2.40 |
| iPT15n12Fc | 100 | 3.57 | 2.33 |
| iPT15diFc | 100 | 3.50 | 2.40 |
| iPT42n12Fc | 100 | 3.45 | 2.51 |
| Blank | —— | 4.64 | 4.29 |
| Negative | —— | 6.04 | 5.43 |
| iPT7n15Fc | 100 | 7.27 | 5.46 |
| iPT42n7Fc | 100 | 6.44 | 5.35 |

### Example 6 Efficacy in vivo study of Fc fusion protein of PT tetravalent antibody

### 6.1 Protection experiment against PT toxin

The NIH mice used were divided into groups with 10 mice in each group, and were injected intraperitoneally with PTtoxin at a dose of 5 µg/mouse to establish a challenged mouse model for evaluating PT toxin antibody.

In this experiment, FC fusion proteins of PT tetravalent antibody iPT7n15Fc, iPT42n7Fc, iPT15n12Fc, iPT42n12Fc and iPT12diFc were injected intraperitoneally into the challenged mouse model, and mouse mortality was recorded every day. Two hours after challenge, the FC fusion protein of PT tetravalent antibody was injected, for single dose administration ,the dose was 20 µg/dose and 40 µg/dose. respectively; for multiple dose administration, it was carried out in five times, with doses being 20 µg/dose and 40 µg/dose, the day of grouping was recorded as Day 0, meanwhile the negative control was set, then weighing of mice, measuring the numbers of white blood cells and lymphocytes were carried out at D0, D5, D10 and D15, respectively, and the survival rate of mice was recorded every day.

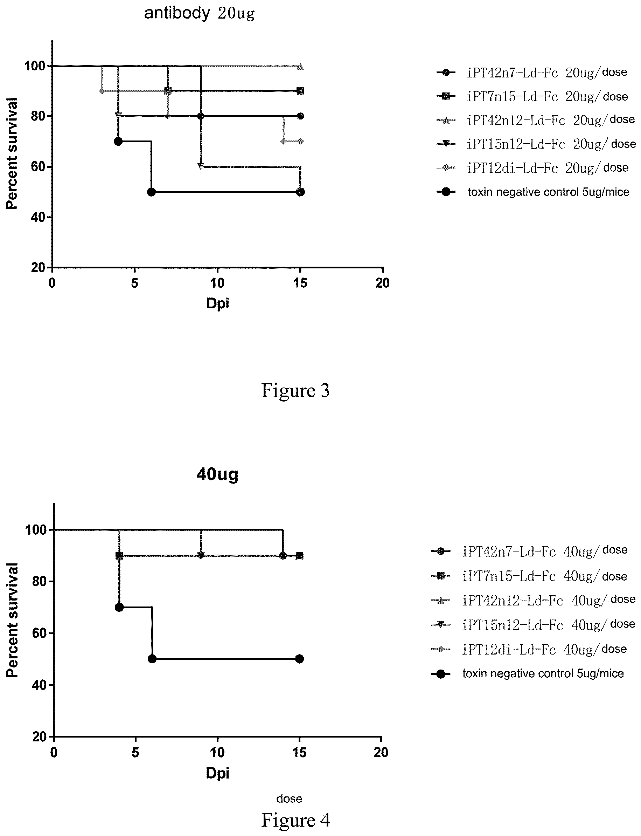

The results were shown in Table 12 below, compared with the negative control, all antibodies had protective effect on the challenged mice; for single dose and multiple dose administration, the survival rate of mice was significantly increased when iPT15n12FC and iPT12diFC were administrated at 40 µg/dose as compared with at 20 µg/dose, and there was no significant difference among other antibodies. Survival curves for single dose administration of 20 µg/dose and 40 µg/dose were shown in Fig.1 and Fig. 2, respectively, and survival curves for multiple dose administration of 20 µg/dose and 40 µg/dose were shown in Fig. 3 and Fig. 4, respectively.

**Table 12**

| Antibody name | Survival rate for single dose administration % | | | | Survival rate for multiple dose administration % | | | |
|---|---|---|---|---|---|---|---|---|
| | 20 ug/dose | | 40 ug/dose | | 20 ug/dose | | 40 ug/dose | |
| | D10 | D15 | D10 | D15 | D10 | D15 | D10 | D15 |
| Negative control group | 0 | 0 | 0 | 0 | 50 | 50 | 50 | 50 |
| iPT7n15Fc | 40 | 30 | 70 | 40 | 90 | 90 | 90 | 90 |
| iPT42n7Fc | 40 | 40 | 60 | 20 | 80 | 80 | 100 | 90 |
| iPT15n12F c | 20 | 20 | 60 | 50 | 60 | 50 | 90 | 90 |
| iPT42n12F c | 50 | 40 | 80 | 30 | 100 | 100 | 90 | 90 |
| iPT12diFc | 40 | 20 | 100 | 50 | 80 | 70 | 90 | 90 |

### 6.2 Protection experiment against bacteria

All NIH mice to be used were females, weighed in 1622 g/mouse, with 10 mice in each group, the day of grouping was recorded as Day 0, the mice were weighed, the numbers of white blood cells and lymphocytes were measured, and pertussis Bacillus was injected intraperitoneally into the NIH mice, thus establishing a challenged mouse model for evaluating PT toxin antibody by intraperitoneal injection of pertussis Bacillus.

In this experiment, FC fusion proteins of PT tetravalent antibody iPT7n15Fc, iPT42n7Fc, iPT15n12Fc, iPT42n12Fc and iPT12diFc were injected intraperitoneally into the challenged mouse model, and mouse mortality was recorded every day. Two hours after challenge, the FC fusion protein of PT tetravalent antibody was injected with multiple dose administration in five times, with doses being 20 µg/dose and 40 (.ig/dose. respectively, the day of grouping was recorded as Day 0, meanwhile the negative control was set, then weighing of mice, measuring the numbers of white blood cells and lymphocytes were carried out at D0, D5, D10 and D15, respectively, and the survival rate of mice was recorded every day.

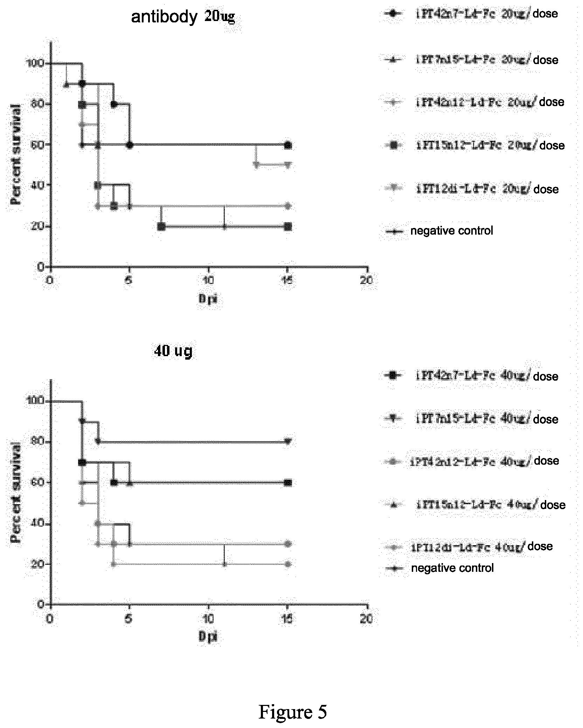

The results were shown in Table 13, compared with the negative control, all antibodies had protective effect on the challenge mice, among which the antibodies iPT7n15FC and iPT42n7Fc had good protective effect, the survival rate of mice was significantly higher when administrated with iPT7n15FC and iPT15n12Fc at 40 µg/dose than at 20 µg/dose. The survival curves were shown in Figure 5.

**Table 13**

| Antibody name | Survival rate for multiple dose administration% | | | |
|---|---|---|---|---|
| | 20 ug/dose | | 40 ug/dose | |
| | D10 | D15 | D10 | D15 |
| Negative control | 20 | 20 | 20 | 20 |
| iPT7n15Fc | 60 | 60 | 80 | 80 |
| iPT42n7Fc | 60 | 60 | 60 | 60 |
| iPT15n12Fc | 20 | 20 | 60 | 60 |
| iPT42n12Fc | 30 | 30 | 30 | 30 |
| iPT12diFc | 60 | 50 | 20 | 20 |

### Example 7: Humanization of PT single domain antibody

The humanization method was achieved by humanization of amino acids on the protein surface (resurfacing) and the VHH humanized universal framework transplantation method (CDR grafting to a universal framework).

The humanization steps were as follows: subjecting the antibody strains iPT7, iPT15 and iPT42 to homologous modeling by the software Modeller 9. By using NbBcII10 antibody (PDB number: 3DWT) as the reference sequence, the relative solvent accessibility of amino acids was calculated based on the three-dimensional structure of protein. If an amino acid from antibody strains iPT7, iPT15 and iPT42 was exposed to solvent, which was replaced with the amino acid at the same position in the reference humanized antibody 10HQ sequence, and finally all the substitutions were done.

The specific steps of the VHH humanized universal framework transplantation method were as follows: firstly, the universal humanized VHH framework hNbBcII10FGLA (PDB number: 3EAK) designed and made according to sequence homology by Cécile Vincke et al. was obtained, which was designed based on the nanoantibody NbBcII10 antibody (PDB number: 3DWT), and the reference humanized antibody 10HQ was humanized on the amino acids on the protein surface, and the modifications of some amino acids VLP from the VHH sequence frame 1(framework1), some amino acids GL from the VHH sequence frame 2 (framework2), some amino acids RSKRAAV of the VHH sequence frame 3 (framework3) and amino acid L of the VHH sequence frame 4(framework4) were done. By using hNbBcII10FGLA as the framework, the CDR was replaced by the CDR region of antibody strains iPT7, iPT15 and iPT42 to accomplish the humanization of the antibody.

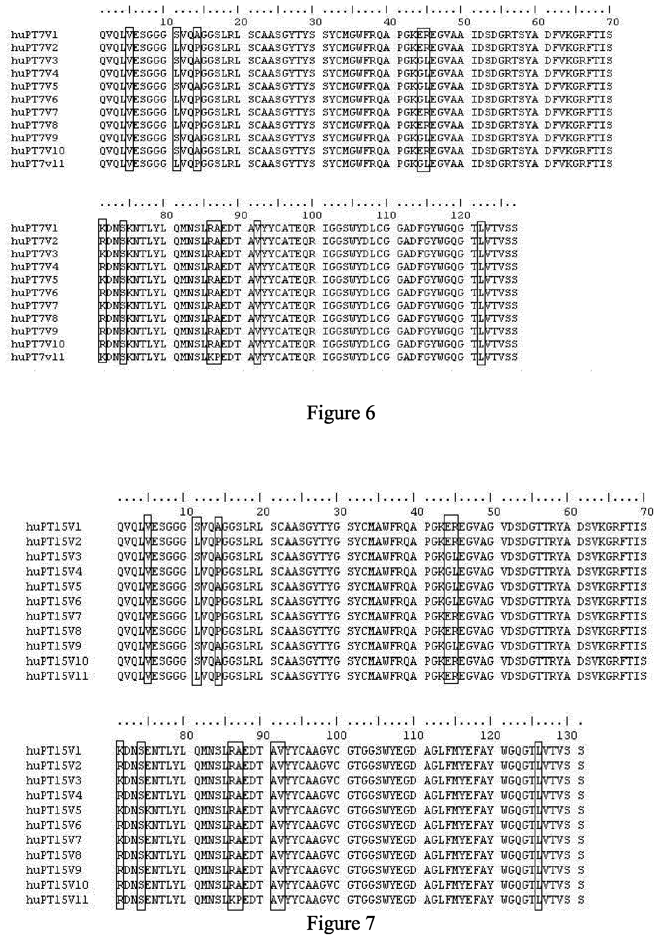

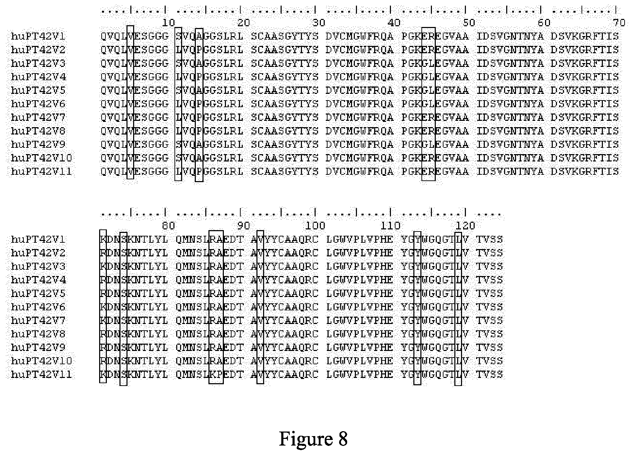

The antibody strains iPT7, iPT15 and iPT42 were humanized to obtain 11 humanized variants of antibody strain huPT. The sequence numbering of these humanized variants and the numbering of the amino acid residues therein were done according to Kabat, ant the comparison results of humanized sequences were shown in Figs. 6-8.

### Example 8: Preparation of FC fusion protein of huPT single domain antibody with mammalian cells

### 8.1 Preparation of Fc fusion plasmid of huPT single domain antibody

The coding sequences of huPT single domain antibody in Example 7 were commissioned to Suzhou Hongxun Biotechnology Co., Ltd. for gene synthesis, with enzyme digestion sites added at both ends.

The VHH fragment of huPT single domain antibody was performed with double enzyme digestion, and fused with the DNA fragment encoding human IgG1FC, and cloned into the conventional mammalian expression vector to obtain the recombinant plasmid for expressing the Fc fusion protein of PT single domain antibody in mammals.

### 8.2 Preparation of Fc fusion plasmid of huPT single domain antibody

The vector constructed in 8.1 was transfected into HEK293 cells for transient expression of the antibody. The recombinant expression plasmid was diluted with Freestyle293 medium and added with PEI (Polyethylenimine) solution needed for transformation, and each group of plasmid /PEI mixture was added into HEK293 cell suspension and placed at 37°C, 5% CO₂ for culture at 130 rpm. Four hours later, EXCELL293 medium, 2 mM glutamine were added for culture at 130 rpm. After 24 hours, 3.8 mM VPA was added, and with 4 g/L glucose after 72 hours. After culture for 5-6 days, the supernatant of transient expression culture was collected, and the Fc fusion protein of target huPT single domain antibody was obtained by purification using Protein A affinity chromatography. The purity of protein was preliminarily investigated by SDSPAGE and SECHPLC. The expression and purity analysis of each protein were shown in Table 14 below. The results showed that all humanized variants of iPT7 and iPT15 were not significantly different in expression level and purity, which were acceptable. However, the humanized variant of iPT42 showed certain difference in stability. Wherein, the humanized variants v1, v2, v7, v8, v10 and v11 were not desirable in either expression level or product purity.

**Table 14**

| Antibody | Expression amount (mg/L) | Main peak ratio% |
|---|---|---|
| huPT7v1Fc | 325 | 98.5 |
| huPT7v2 Fc | 336 | 98.3 |
| huPT7v3 Fc | 288 | 98.2 |
| huPT7v4Fc | 278 | 97.5 |
| huPT7v5Fc | 314 | 98.3 |
| huPT7v6Fc | 309 | 97.6 |
| huPT7v7Fc | 294 | 98.1 |
| huPT7v8Fc | 321 | 97.6 |
| huPT7v9Fc | 286 | 98.2 |
| huPT7v10Fc | 296 | 97.3 |
| huPT7v11Fc | 301 | 97.7 |
| huPT15vlcFc | 462 | 97.3 |
| huPT15v2c Fc | 179 | 94.0 |
| huPT15v3c Fc | 306 | 95.3 |
| huPT15v4cFc | 251 | 66.0 |
| huPT15v5cFc | 398 | 92.3 |
| huPT15v6cFc | 356 | 95.3 |
| huPT15v7cFc | 451 | 97.4 |
| huPT15v8cFc | 432 | 94.6 |
| huPT15v9cFc | 386 | 96.8 |
| huPT15v10cFc | 453 | 97.5 |
| huPT15v11cFc | 492 | 96.6 |
| huPT42v1Fc | 146 | 32.0 |
| huPT42v2Fc | 145 | 18.7 |
| huPT42v3Fc | 286 | 90.5 |
| huPT42v4Fc | 297 | 91.2 |
| huPT42v5Fc | 300 | 92.0 |
| huPT42v6Fc | 472 | 95.4 |
| huPT42v7Fc | 156 | 56.6 |
| huPT42v8Fc | 120 | 42.5 |
| huPT42v9Fc | 356 | 95.6 |
| huPT42v10Fc | 189 | 38.8 |
| huPT42v11Fc | 133 | 53.3 |

### Example 9: Verification of functions of FC fusion protein of humanized huPT single domain antibody

### 9.1 Identification of PT binding capability of Fc fusion protein of huPT single domain antibody (ELISA)

The recombinant human PT protein were coated on a plate with 0.3 µg/well for staying at 4°C overnight, and reacted with the gradient dilution series of Fc fusion protein of huPT single domain antibody obtained in Example 8.2 for 2 hours at room temperature. After washing, the secondary antibody goat anti-human IgGFC horseradish peroxidase labeled antibody (Sigma) was added and reacted at room temperature for 2 hours. After washing, the developing solution was added, and the absorbance value was read at 450 nm and 650 nm, and the final absorbance value was given by subtracting the absorbance value at 650 nm from that at 450 nm. The software SotfMax Pro v5.4 was applied for data processing and mapping analysis, by using four-parameter fitting, the binding curve and EC₅₀ of huPT antibody for PT protein were obtained, which reflected the affinity of the antibody for FIX. The results were shown in Table 15 below. As can be seen, all humanized variants had similar binding capacity to PT toxin protein.

**Table 15**

| Antibody | EC₅₀ (ng/mL) |
|---|---|
| huPT7v1Fc | 34.9 |
| huPT7v2 Fc | 33.7 |
| huPT7v3 Fc | 34.2 |
| huPT7v4Fc | 36.8 |
| huPT7v5Fc | 35.7 |
| huPT7v6Fc | 35.0 |
| huPT7v7Fc | 36.0 |
| huPT7v8Fc | 33.8 |
| huPT7v9Fc | 34.6 |
| huPT7v10Fc | 35.6 |
| huPT7v11Fc | 33.3 |
| huPT15vlcFc | 20.2 |
| huPT15v2c Fc | 19.8 |
| huPT15v3c Fc | 21.8 |
| huPT15v4cFc | 22.0 |
| huPT15v5cFc | 21.8 |
| huPT15v6cFc | 20.9 |
| huPT15v7cFc | 22.1 |
| huPT15v8cFc | 20.7 |
| huPT15v9cFc | 21.3 |
| huPT15v10cFc | 22.1 |
| huPT15v11cFc | 20.5 |
| huPT42v1Fc | 25.8 |
| huPT42v2Fc | 27.6 |
| huPT42v3Fc | 26.2 |
| huPT42v4Fc | 26.4 |
| huPT42v5Fc | 25.7 |
| huPT42v6Fc | 25.6 |
| huPT42v7Fc | 22.9 |
| huPT42v8Fc | 24.9 |
| huPT42v9Fc | 27.2 |
| huPT42v10Fc | 25.5 |
| huPT42v11Fc | 25 |

### 9.2 Identification of the neutralizing activity of FC fusion protein of humanized PT monoclonal antibody against pertussis toxin

PT toxin at a concentration of 434.3 µg/ml was formulated to 12 ng/mL with 10% FBS+ F12K+10% FBS, and added with 50 µL to per well, the FC fusion protein of PT antibody was 2x diluted from the original concentraiton with 10% FBS+ F12K, to obtain 10 concentrations, 50 µL was added to each well, after stay at 37°C for 1 hour, CHOK1 cells were collected and counted, and the number of cells was adjusted to 3x10⁵ cells/mL, and 50 µL was added to each well of the 96-well plate, after 24 hours, 100% alcohol fixing and 0.1% crystal violet staining were carried out successively, after washing with PBS, observation and photos were taken under microscope. Three humanized variants of each of iPT7, iPT15 and iPT42 were randomly selected for investigating the neutralizing activity thereof. Among the variants of iPT42, v1, v2, v7, v8, v10 and v11 with poor stability were discarded.

The results were shown in Table 16 below, the cytotoxic neutralizing activity of the selected humanized variants were equal to or slightly better than that of the maternal antibody.

**Table 16**

| Sample | Minimum neutralizing concentration (ug/ml) |
|---|---|
| iPT15-Fc | <1 |
| huPT15V3c-ld-Fc | <1 |
| huPT15V7c-ld-Fc | <1 |
| huPT15V11c-1d-Fc | <1 |
| iPT7-Fc | <1 |
| huPT7V1-1d-Fc | < 0.5 |
| huPT7V4-ld-Fc | <0.5 |
| huPT7V11-ld-Fc | < 0.5 |
| iPT42-Fc | -32 |
| huPT42V4-ld-Fc | -32 |
| huPT42V6-ld-Fc | -16 |
| huPT42V9-ld-Fc | -32 |

### 9.3 Detection of the nonspecific binding of Fc fusion protein of huPT single domain antibody to empty cells

CHOK1 empty cells and 293F empty cells were resuspended in 3% BSAPBS and adjusted to 6× 10⁶ cells/mL, and added with the Fc fusion protein of huPT monoclonal antibody obtained in Example 8.2 with a final concentration of 100 µg/mL and 10 µg/mL, respectively, meanwhile, the negative control and blank control were set, each being cooled in an ice bath for 30 min. After washing, Boster secondary antibody FITC rabbit anti-human IgG antibody was added, with being cooled in an ice bath for 30min. After washing, the cells were resuspended in 300 µL of 1% PBSBSA Buffer, and detected by flow cytometry. The results were shown in Table 17, which showed that no nonspecific binding had been found.

**Table 17**

| | Concentration (µg/mL) | MFI (CHO) | MFI (293) |
|---|---|---|---|
| Blank | —— | 3.31 | 3.28 |
| Negative | —— | 4.86 | 5.44 |
| huPT7v1Fc | 100 ug/mL | 5.35 | 6.17 |
| | 10 ug/mL | 6.05 | 6.21 |
| huPT7v2Fc | 100 ug/mL | 5.42 | 6.70 |
| | 10 ug/mL | 5.56 | 6.63 |
| huPT7v4 Fc | 100 ug/mL | 5.83 | 7.69 |
| | 10 ug/mL | 5.74 | 8.35 |
| huPT7v7Fc | 100 ug/mL | 5.63 | 6.99 |
| | 10 ug/mL | 6.13 | 6.42 |
| huPT7v11Fc | 100 ug/mL | 5.93 | 7.01 |
| | 10 ug/mL | 6.29 | 6.41 |
| huPT42v4Fc | 100 ug/mL | 5.76 | 5.85 |
| | 10 ug/mL | 6.05 | 6.51 |
| huPT42v6Fc | 100 ug/mL | 6.23 | 6.15 |
| | 10 ug/mL | 6.00 | 6.81 |
| huPT42v9Fc | 100 ug/mL | 7.56 | 5.12 |
| | 10 ug/mL | 7.00 | 6.20 |
| huPT15V1c-ld-Fc | 100 ug/mL | 7.04 | 6.93 |
| | 10 ug/mL | 7.20 | 6.68 |
| huPT15V3c-ld-Fc | 100 ug/mL | 7.13 | 6.30 |
| | 10 ug/mL | 6.38 | 7.40 |
| huPT15V7c-ld-Fc | 100 ug/mL | 6.54 | 6.00 |
| | 10 ug/mL | 7.37 | 7.06 |
| huPT15V11c-ld-Fc | 100 ug/mL | 6.50 | 5.58 |
| | 10 ug/mL | 5.55 | 6.68 |

### 9.4 Detection of the thermal stability of Fc fusion protein of huPT single domain antibody

The trace volume of protein samples was determined by using the protein thermostability detector from UNCHAINED LABS. Heating program: inital temperature: 15°C, heating rate: 0.3 °C /min, final temperature: 95°C. The fluorescence absorbance values of samples at each temperature and wavelength were recorded, and the denaturation temperature Tm was fitted using a software as the highest point of the next derivative of confidence wavelength BCM; the initial polymerization temperature Tagg was one tenth of the next derivative of static light scattering SLS at 473 nm. The higher the Tm value, the more stable the protein was. The results were shown in Table 18 below, as can be seen, each antibody had a Tm basically above 60°C with good stability.

**Table 18**

| | Tm1 | Tagg |
|---|---|---|
| huPT7v4-Fc | 60.8 | 58.5 |
| huPT7v7 -Fc | 60.4 | 58.0 |
| huPT7v2-Fc | 60.6 | 58.2 |
| huPT7v1-Fc | 60.3 | 58.1 |
| huPT7v11-Fc | 62.1 | 60.0 |
| huPT42v4-Fc | 64.5 | 62.0 |
| huPT42v6-Fc | 67.7 | 72.8 |
| huPT42v9-Fc | 64.2 | 66.6 |
| huPT15V1c-ld-Fc | 66.8 | 69.6 |
| huPT15V3c-ld-Fc | 66.5 | 69.2 |
| huPT15V7c-ld-Fc | 66.8 | 69.5 |
| huPT15V11c-ld-Fc | 67.9 | 72.0 |

### Example 10: Preparation of Fc fusion protein of tetravalent humanized PT antibody

According to the method of Example 4, the humanized antibody sequences, huPT7v11, huPT15v7c and huPT42v6, which were obtained and investigated in Examples 7-9 were selected to be combined in tandem in pairs according to the overlapping of antigen-recognizing epitopes of antibodies (epitope binning results) and fused with the DNA fragment encoding human IgG1FC, to obtain tetravalent bispecific antibodies huPT7v11n15v7c-Fc, huPT7v11n15v11c-Fc, huPT42v6n15v11c-Fc and huPT42v6n7v11-Fc.

The HEK293 cells were instantly expressed and purified to obtain the Fc fusion protein of target PT tetravalent antibody.

### Example 11 Further investigation on the activity of Fc fusion protein of tetravalent humanized PT antibody

### 11.1 Investigation on the affinity for PT toxin

The Fc fusion protein of PT tetravalent antibody prepared in Example 10 was diluted to 2.5 µg/mL and immobilized on biosensor for 60 seconds, with a curing height of about 0.8 nm. PTpuri was diluted to five gradients as 100 nM, 50 nM, 25 nM, 12.5 nM and 6.25 nm, baseline: 60s, binding: 120s, dissociation: 600s. The diluent was 0.02PBST20%, the regeneration solution was glycine-HCl (pH1.7), the neutralizing solution was the diluent, the biosensor was AHC, and the results were shown in Table 19 below. Among them, 1B7 and 11E6 were used as control antibodies, which binded to PT toxin and showed the neutralizing effect. Self-cloning and preparation were carried out according to the sequences in patent US10035846.

**Table 19**

| Sample | KD (M) | kon (1/Ms) | kdis (1/s) |
|---|---|---|---|
| huPT7n15-Fc | 8.84E-11 | 3.75E+05 | 3.32E-05 |
| huPT42n7-Fc | 2.00E-10 | 4.59E+05 | 9.18E-05 |
| huPT42n15-Fc | <1.0E-12 | 3.37E+05 | < 1.0E-07 |
| 1B7 | <1.0E-12 | 3.87E+05 | < 1.0E-07 |
| 11E6 | <1.0E-12 | 3.11E+05 | < 1.0E-07 |

### 11.2 Invesgation on the neutralizing activity of Fc fusion protein of tetravalent humanized PT antibody and comparison with control antibody

PT toxin at a concentration of 434.3 µg/ml was formulated to 12 ng/mL with 10% FBS+ F-12K+10% FBS, and added with 50 µL to per well, the FC fusion protein of PT tetravalent antibody with good affinity prepared in Example 4.2 was 2x diluted from the original concentraiton with 10% FBS+ F-12K, to obtain 10 concentrations, 50 µL was added to each well, after stay at 37°C for 1 hour, CHO-K1 cells were collected and counted, and the number of cells was adjusted to 3x10⁵ cells/mL, 50 µL was added to each well of the 96-well plate, after 24 hours, 100% alcohol fixing and 0.1% crystal violet staining were carried out successively, after washing with PBS, observation and photos were taken under microscope. 1B7 and 11E6 were control antibodies, which binded to PT toxin and showed the neutralizing effect. Self-cloning and preparation were carried out according to the sequences in patent US10035846.

The results were shown in Table 20, wherein, tetravalent antibodies huPT7n15-Fc, huPT42n7-Fc, huPT42n15-Fc, iPT12n42-Fc, iPT12di-Fc, iPT15n12-Fc and iPT42n12-Fc showed better neutralizing activity, and the neutralizing activitity of each of iPT7n15-Fc, iPT42n7-Fc, iPT12n15-Fc, iPT12n42-Fc, iPT15n12-Fc and iPT42n12-Fc was higher than that of iPT15di-Fc and iPT12di-Fc, and also higher than that of bivalent antibody iPT12-Fc, as can be seen, the neutralizing activity was improved after the combination of single-domain antibodies that recognize different epitopes.

**Table 20**

| Antibody | Minimum neutralizing concentration |
|---|---|
| huPT7n15-Fc | 0.025 µg/mL |
| huPT42n7-Fc | 0.05 µg/mL |
| huPT42n15-Fc | 0.1 µg/mL |
| 1B7 | 1.6 µg/mL |
| 11E6 | 0.8 µg/mL |
| 1B7-11E6 | (0.4+0.4) µg/mL |
| huPT7n15-Fc÷ huPT42n7-Fc | (0.00625 + 0.00625)µg/mL |
| huPT7n15-Fc+ huPT42n15-Fc | (0.0125 + 0.0125) µg/mL |

### Example 12 Comparison of epitopes between Fc fusion protein of Pt single domain antibody and control antibody

In this example, the binding epitopes of the Fc fusion protein of PT single domain antibody of the present invention and that of the control antibody were compared by epitope binning using Bio-Layer Interferometry (BLI).

Specifically, by using the intandem method, PT-biotin was diluted with 0.02% PBST20 to 10 ug/mL and immobilized on SAbiosensor for 120s. The Fc fusion protein of PT single domain antibody was diluted to 100 nM with 0.02% PBST20, and divided into two groups with the antibody-binding time of 300s for each group and the regeneration solution of 10 mM glycine-HC1 (pH 1.7), wherein the first antibody (saturated antibody) binded to the sensor until saturated, and then the second antibody (competitive antibody) competed with the first antibody at the same concentration, and the percentage was calculated. The percentage was calculated following the formula: Ab2 with Ab1/Ab2 without Ab1. Wherein, the control antibody (11E6, 1B7, derived from patent US10035846) was Ab1, and the subsequent antibody was Ab2. At the same time, the control antibody was also added to Ab2 group as a reference for the complete overlapping of antigenic epitopes.

The results were shown in Table 21 below. The results showed that, except that the epitopes of iPT12 overlapped with that of 11E6 control antibody, and the epitopes of iPT7 partially overlapped with that of 11E6 control antibody, all the other antibodies had no obvious competitive relationship with the two control antibodies and had completely non-overlapping antigen-binding epitopes.

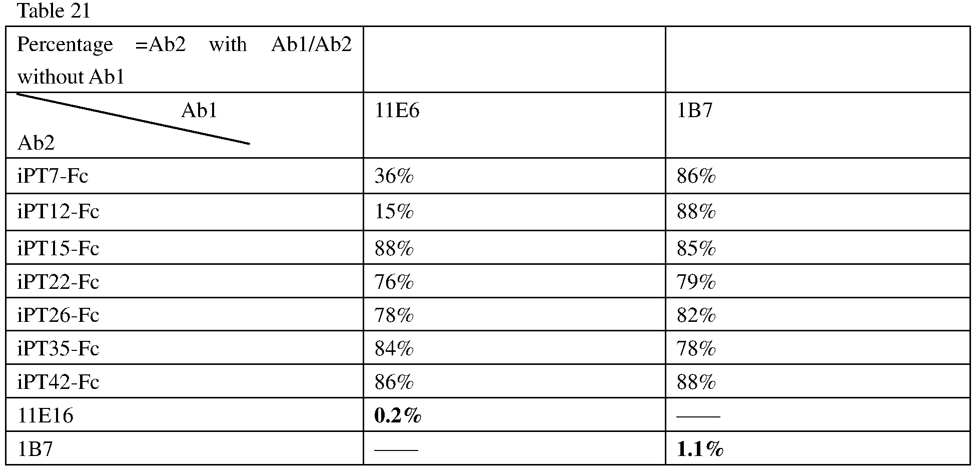

## Claims

1. A pertussis toxin-binding protein capable of specifically binding to pertussis toxin and comprising at least one immunoglobulin single variable domain comprising CDR1, CDR2 and CDR3 selected from the group consisting of:
(1) CDR1 shown in SEQ ID NO: 1, CDR2 shown in SEQ ID NO: 2, and CDR3 shown in SEQ ID NO: 3;
(2) CDR1 shown in SEQ ID NO: 4, CDR2 shown in SEQ ID NO: 5, and CDR3 shown in SEQ ID NO: 6;
(3) CDR1 shown in SEQ ID NO: 7, CDR2 shown in SEQ ID NO: 8, and CDR3 shown in SEQ ID NO: 9;
(4) CDR1 shown in SEQ ID NO: 10, CDR2 shown in SEQ ID NO: 11, and CDR3 shown in SEQ ID NO: 12;
(5) CDR1 shown in SEQ ID NO: 13, CDR2 shown in SEQ ID NO: 14, and CDR3 shown in SEQ ID NO: 15;
(6) CDR1 shown in SEQ ID NO: 16, CDR2 shown in SEQ ID NO: 17, and CDR3 shown in SEQ ID NO: 18;
(7) CDR1 shown in SEQ ID NO: 19, CDR2 shown in SEQ ID NO: 20, and CDR3 shown in SEQ ID NO: 21;
(8) CDR1 shown in SEQ ID NO: 22, CDR2 shown in SEQ ID NO: 23, and CDR3 shown in SEQ ID NO: 24;
(9) CDR1 shown in SEQ ID NO: 25, CDR2 shown in SEQ ID NO: 26, and CDR3 shown in SEQ ID NO: 27;
(10) CDR1 shown in SEQ ID NO: 28, CDR2 shown in SEQ ID NO: 29, and CDR3 shown in SEQ ID NO: 30;
(11) CDR1 shown in SEQ ID NO: 31, CDR2 shown in SEQ ID NO: 32, and CDR3 shown in SEQ ID NO: 33;
(12) CDR1 shown in SEQ ID NO: 34, CDR2 shown in SEQ ID NO: 35, and CDR3 shown in SEQ ID NO: 36;
(13) CDR1 shown in SEQ ID NO: 37, CDR2 shown in SEQ ID NO: 38, and CDR3 shown in SEQ ID NO: 39.

2. The pertussis toxin-binding protein according to claim 1, wherein the immunoglobulin single variable domain is a VHH.

3. The pertussis toxin-binding protein according to claim 2, wherein the VHH is a humanized VHH.

4. The pertussis toxin-binding protein according to claim 2, wherein the VHH comprises an amino acid sequence of any one of SEQ ID NOs: 40-52.

5. The pertussis toxin-binding protein according to claim 3, wherein the VHH comprises an amino acid sequence having at least 80%, preferably at least 90%, more preferably at least 95%, even more preferably at least 99% sequence identity to the sequence of any one of SEQ ID NOs: 40-52.

6. The pertussis toxin-binding protein according to claim 3, wherein the humanized VHH comprises an amino acid sequence of any one of SEQ ID NOs: 53-85.

7. The pertussis toxin-binding protein according to any one of claims 1-6, which comprises at least two immunoglobulin single variable domains.

8. The pertussis toxin-binding protein according to claim 7, wherein the at least two immunoglobulin single variable domains bind to the same epitope or compete for binding to the same epitope, for example, the at least two immunoglobulin single variable domains are the same.

9. The pertussis toxin-binding protein according to claim 7, wherein the at least two immunoglobulin single variable domains bind to different epitopes or do not compete for binding to the same epitope.

10. The pertussis toxin-binding protein according to claim 9, which comprises a first immunoglobulin single variable domain and a second immunoglobulin single variable domain, wherein the first immunoglobulin single variable domain is located at the N-terminal of the second immunoglobulin single variable domain, and
wherein the first immunoglobulin single variable domain comprises CDR1 shown in SEQ ID NO: 4, CDR2 shown in SEQ ID NO: 5 and CDR3 shown in SEQ ID NO: 6, and the second immunoglobulin single variable domain comprises CDR1 shown in SEQ ID NO: 13, CDR2 shown in SEQ ID NO: 14 and CDR3 shown in SEQ ID NO: 15; or
wherein the first immunoglobulin single variable domain comprises CDR1 shown in SEQ ID NO: 13, CDR2 shown in SEQ ID NO: 14 and CDR3 shown in SEQ ID NO: 15, and the second immunoglobulin single variable domain comprises CDR1 shown in SEQ ID NO: 4, CDR2 shown in SEQ ID NO: 5 and CDR3 shown in SEQ ID NO: 6; or
wherein the first immunoglobulin single variable domain comprises CDR1 shown in SEQ ID NO: 4, CDR2 shown in SEQ ID NO: 5 and CDR3 shown in SEQ ID NO: 6, and the second immunoglobulin single variable domain comprises CDR1 shown in SEQ ID NO: 22, CDR2 shown in SEQ ID NO: 23 and CDR3 shown in SEQ ID NO: 24; or
wherein the first immunoglobulin single variable domain comprises CDR1 shown in SEQ ID NO: 22, CDR2 shown in SEQ ID NO: 23 and CDR3 shown in SEQ ID NO: 24, and the second immunoglobulin single variable domain comprises CDR1 shown in SEQ ID NO: 4, CDR2 shown in SEQ ID NO: 5 and CDR3 shown in SEQ ID NO: 6; or
wherein the first immunoglobulin single variable domain comprises CDR1 shown in SEQ ID NO: 4, CDR2 shown in SEQ ID NO: 5 and CDR3 shown in SEQ ID NO: 6, and the second immunoglobulin single variable domain comprises CDR1 shown in SEQ ID NO: 37, CDR2 shown in SEQ ID NO: 38 and CDR3 shown in SEQ ID NO: 39; or
wherein the first immunoglobulin single variable domain comprises CDR1 shown in SEQ ID NO: 37, CDR2 shown in SEQ ID NO: 38 and CDR3 shown in SEQ ID NO: 39, and the second immunoglobulin single variable domain comprises CDR1 shown in SEQ ID NO: 4, CDR2 shown in SEQ ID NO: 5 and CDR3 shown in SEQ ID NO: 6; or
wherein the first immunoglobulin single variable domain comprises CDR1 shown in SEQ ID NO: 7, CDR2 shown in SEQ ID NO: 8 and CDR3 shown in SEQ ID NO: 9, and the second immunoglobulin single variable domain comprises CDR1 shown in SEQ ID NO: 31, CDR2 shown in SEQ ID NO: 32 and CDR3 shown in SEQ ID NO: 33; or
wherein the first immunoglobulin single variable domain comprises CDR1 shown in SEQ ID NO: 31, CDR2 shown in SEQ ID NO: 32 and CDR3 shown in SEQ ID NO: 33, and the second immunoglobulin single variable domain comprises CDR1 shown in SEQ ID NO: 7, CDR2 shown in SEQ ID NO: 8 and CDR3 shown in SEQ ID NO: 9; or
wherein the first immunoglobulin single variable domain comprises CDR1 shown in SEQ ID NO: 13, CDR2 shown in SEQ ID NO: 14 and CDR3 shown in SEQ ID NO: 15, and the second immunoglobulin single variable domain comprises CDR1 shown in SEQ ID NO: 31, CDR2 shown in SEQ ID NO: 32 and CDR3 shown in SEQ ID NO: 33; or
wherein the first immunoglobulin single variable domain comprises CDR1 shown in SEQ ID NO: 13, CDR2 shown in SEQ ID NO: 14 and CDR3 shown in SEQ ID NO: 15, and the second immunoglobulin single variable domain comprises CDR1 shown in SEQ ID NO: 37, CDR2 shown in SEQ ID NO: 38 and CDR3 shown in SEQ ID NO: 39; or
wherein the first immunoglobulin single variable domain comprises CDR1 shown in SEQ ID NO: 31, CDR2 shown in SEQ ID NO: 32 and CDR3 shown in SEQ ID NO: 33, and the second immunoglobulin single variable domain comprises CDR1 shown in SEQ ID NO: 37, CDR2 shown in SEQ ID NO: 38 and CDR3 shown in SEQ ID NO: 39; or
wherein the first immunoglobulin single variable domain comprises CDR1 shown in SEQ ID NO: 7, CDR2 shown in SEQ ID NO: 8 and CDR3 shown in SEQ ID NO: 9, and the second immunoglobulin single variable domain comprises CDR1 shown in SEQ ID NO: 13, CDR2 shown in SEQ ID NO: 14 and CDR3 shown in SEQ ID NO: 15; or
wherein the first immunoglobulin single variable domain comprises CDR1 shown in SEQ ID NO: 13, CDR2 shown in SEQ ID NO: 14 and CDR3 shown in SEQ ID NO: 15, and the second immunoglobulin single variable domain comprises CDR1 shown in SEQ ID NO: 7, CDR2 shown in SEQ ID NO: 8 and CDR3 shown in SEQ ID NO: 9; or
wherein the first immunoglobulin single variable domain comprises CDR1 shown in SEQ ID NO: 7, CDR2 shown in SEQ ID NO: 8 and CDR3 shown in SEQ ID NO: 9, and the second immunoglobulin single variable domain comprises CDR1 shown in SEQ ID NO: 37, CDR2 shown in SEQ ID NO: 38 and CDR3 shown in SEQ ID NO: 39; or
Wherein the first immunoglobulin single variable domain comprises CDR1 shown in SEQ ID NO: 37, CDR2 shown in SEQ ID NO: 38 and CDR3 shown in SEQ ID NO: 39, and the second immunoglobulin single variable domain comprises CDR1 shown in SEQ ID NO: 7, CDR2 shown in SEQ ID NO: 8 and CDR3 shown in SEQ ID NO: 9.

11. The pertussis toxin-binding protein according to claim 10, wherein
the first immunoglobulin single variable domain comprises the amino acid sequence shown in one of SEQ ID NOs: 41, 53-63 and the second immunoglobulin single variable domain comprises the amino acid sequence shown in one of SEQ ID NOs: 44, 64-74; or
the first immunoglobulin single variable domain comprises the amino acid sequence shown in one of SEQ ID NOs: 44, 64-74 and the second immunoglobulin single variable domain comprises the amino acid sequence shown in one of SEQ ID NOs: 41, 53-63; or
the first immunoglobulin single variable domain comprises the amino acid sequence shown in one of SEQ ID NOs: 41, 53-63 and the second immunoglobulin single variable domain comprises the amino acid sequence shown in SEQ ID NO: 47; or
the first immunoglobulin single variable domain comprises the amino acid sequence shown in SEQ ID NO: 47 and the second immunoglobulin single variable domain comprises the amino acid sequence shown in one of SEQ ID NOs: 41, 53-63; or
the first immunoglobulin single variable domain comprises the amino acid sequence shown in one of SEQ ID NOs: 41, 53-63 and the second immunoglobulin single variable domain comprises the amino acid sequence shown in one of SEQ ID NOs: 52, 75-85; or
the first immunoglobulin single variable domain comprises the amino acid sequence shown in one of SEQ ID NOs: 52, 75-85 and the second immunoglobulin single variable domain comprises the amino acid sequence shown in one of SEQ ID NOs: 41, 53-63; or
the first immunoglobulin single variable domain comprises the amino acid sequence shown in SEQ ID NO: 42 and the second immunoglobulin single variable domain comprises the amino acid sequence shown in SEQ ID NO: 50; or
the first immunoglobulin single variable domain comprises the amino acid sequence shown in SEQ ID NO: 50 and the second immunoglobulin single variable domain comprises the amino acid sequence shown in SEQ ID NO: 42; or
the first immunoglobulin single variable domain comprises the amino acid sequence shown in one of SEQ ID NOs: 44, 64-74 and the second immunoglobulin single variable domain comprises the amino acid sequence shown in SEQ ID NO: 50; or
the first immunoglobulin single variable domain comprises the amino acid sequence shown in one of SEQ ID NOs: 44, 64-74 and the second immunoglobulin single variable domain comprises the amino acid sequence shown in one of SEQ ID NOs: 52, 75-85; or
the first immunoglobulin single variable domain comprises the amino acid sequence shown in SEQ ID NO: 50 and the second immunoglobulin single variable domain comprises the amino acid sequence shown in one of SEQ ID NOs: 52, 75-85; or
the first immunoglobulin single variable domain comprises the amino acid sequence shown in SEQ ID NO: 42 and the second immunoglobulin single variable domain comprises the amino acid sequence shown in one of SEQ ID NOs: 44, 64-74; or
the first immunoglobulin single variable domain comprises the amino acid sequence shown in one of SEQ ID NOs: 44, 64-74 and the second immunoglobulin single variable domain comprises the amino acid sequence shown in SEQ ID NO: 42; or
the first immunoglobulin single variable domain comprises the amino acid sequence shown in SEQ ID NO: 42 and the second immunoglobulin single variable domain comprises the amino acid sequence shown in one of SEQ ID NOs: 52, 75-85; or
the first immunoglobulin single variable domain comprises the amino acid sequence shown in one of SEQ ID NOs: 52, 75-85 and the second immunoglobulin single variable domain comprises the amino acid sequence shown in SEQ ID NO: 42.

12. The pertussis toxin-binding protein according to any one of claims 1-11, further comprising an immunoglobulin Fc region.

13. The pertussis toxin-binding protein according to claim 12, wherein the immunoglobulin Fc region is the Fc region of human immunoglobulin, preferably the Fc region of human IgG1.

14. The pertussis toxin-binding protein according to claim 13, wherein the amino acid sequence of the immunoglobulin Fc region is shown in SEQ ID NO: 86.

15. The pertussis toxin-binding protein according to any one of claims 12-14, which comprises the amino acid sequence selected from SEQ ID NOs: 106-109.

16. A nucleic acid molecule encoding the pertussis toxin-binding protein according to any one of claims 1-15.

17. An expression vector comprising the nucleic acid molecule of claim 16 operably linked to an expression regulatory element.

18. A recombinant cell comprising the nucleic acid molecule of claim 16 or transformed with the expression vector of claim 17, and capable of expressing the pertussis toxin binding protein.

19. A method for producing the pertussis toxin-binding protein according to any one of claims 1-15, comprising:
a) culturing the recombinant cell of claim 18 under conditions allowing the expression of the pertussis toxin-binding protein;
b) recovering the pertussis toxin-binding protein expressed by the recombinant cells from the culture obtained from step a); and
c) optionally further purifying and/or modifying the pertussis toxin-binding protein obtained from step b).

20. A pharmaceutical composition, comprising the pertussis toxin-binding protein according to any one of claims 1-15 and a pharmaceutically acceptable carrier.

21. A method for treating Bordetella pertussis infection in a subject, the method comprising administering to the subject an effective amount of the pertussis toxin-binding protein according to any one of claims 1-15 or the pharmaceutical composition according to claim 20.

22. A method for preventing Bordetella pertussis infection in a subject, the method comprising administering to the subject an effective amount of the pertussis toxin-binding protein according to any one of claims 1-15 or pharmaceutical composition according to claim 20.

23. A method for detecting the presence and/or amount of pertussis toxin in a biological sample, comprising:
(a) contacting the biological sample and the control sample with the pertussis toxin-binding protein according to any one of claims 1 to 15 under the condition that a complex can be formed between the pertussis toxin-binding protein and pertussis toxin;
(b) detecting the formation of the complex,
wherein the difference in the complex formation between the biological sample and the control sample indicates the presence and/or amount of pertussis toxin in the sample.

24. A kit comprising the pertussis toxin-binding protein according to any one of claims 1 to 15.
